# EUROPEAN PATENT APPLICATION

(11) **EP 0 838 471 A1**
(43) Date of publication of application: **29.04.1998**
(21) Application number: 96922208.2
(22) Date of filing: 03.07.1996
(51) Int. Cl.: C07K 5/062, C07K 5/083, C07K 5/087, C07K 5/09, C07K 5/097, C07K 5/023, A61K 38/05, A61K 38/06

(54) **PEPTIDE DERIVATIVES AND ANGIOTENSIN IV RECEPTOR AGONIST**

(30) Priority: 07.07.1995 JP 171251/95; 05.10.1995 JP 258635/95
(71) Applicant: SAGAMI CHEMICAL RESEARCH CENTER, Sagamihara-shi, Kanagawa 229 (JP); TAISHO PHARMACEUTICAL CO. LTD, Tokyo 171 (JP)
(72) Inventor: KOBORI, Takeo, Atsugi-shi, Kanagawa 243 (JP); GODA, Kenichi, Sagamihara-shi, Kanagawa 229 (JP); SUGIMOTO, Kikuo, Tsukui-gun, Kanagawa 199-02 (JP); OTA, Tomomi, Taisho Pharmaceutical Co., Ltd., Tokyo 171 (JP); TOMISAWA, Kazuyuki, Taisho Pharmaceutical Co. Ltd., Tokyo 171 (JP)
(74) Representative: Kraus, Walter, Dr.
(86) International application number: JP9601836
(87) International publication number: WO9703093

(57) **Abstract**

Short-chain peptide derivatives acting on angiotensin IV receptor at low concentrations. Because of agonistically acting on angiotensin IV receptor, the novel peptide derivatives of the present invention represented by the following formula (1) are useful as remedies for various diseases in which angiotensin IV participates:

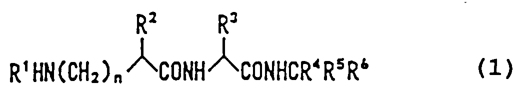

## Description

### TECHNICAL FIELD

This invention relates to peptide derivatives. More particularly, it relates to peptide derivatives useful as remedies for various diseases in which angiotensin IV participates.

### BACKGROUND ART

In the renin-angiotensin system, peptides relating to coronary vessels and electrolyte homeostasis are produced by the peptide degradation cascade. In this cascade, angiotensinogen is first converted by renin into physiologically inactive angiotensin I. Next, angiotensin I is converted successively into angiotensin II, angiotensin III and finally angiotensin IV which is a hexapeptide. It is known that angiotensin IV receptor occurs at high concentrations in various organs such as brain (in particular, hippocampus), adrenal gland, heart, kidney, smooth muscle cells and endothelial cells. It is reported that angiotensin IV relates to various physiological functions, for example, acceleration of renal blood flow (Swanson *et al*., *Regulatory Peptides, 40*:409 (1992)), cerebral vasodilation (Haber *et al*., *Circ. Res*., *68*:1621 (1991)), inhibition of cell proliferation (Barker and Aceto, *Am. J. Physio*., *259*:H610 (1990)) and hypermnesia (Miller-Wing *et al.*, *J. Pharmacol. Exp. Thr.*, *266*:1718 (1993)).

Although there have been reported several peptide compounds agonistically acting on the angiotensin IV receptor (Sardinia *et al.*, *Peptides, 14*:949 (1993); *ibid*., *8*:1399 (1994)), these peptide derivatives should be composed of at least 5 amino acids to express high activities and have some problems in stability, etc. There has been known no peptide derivative as those of the present invention so far.

### DISCLOSURE OF THE INVENTION

The present inventors have conducted extensive studies to develop short-chain peptide derivatives capable of agonistically acting on the angiotensin IV receptor at low concentrations. As a result, they have found that peptide derivatives having specific sequences and specific substituents can agonistically act on the angiotensin IV receptor strongly, even though they are short-chain peptide derivatives fundamentally comprising 2 or 3 amino acids, thus completing the present invention.

That is, the present invention relates to peptide derivatives represented by the following formula (1): 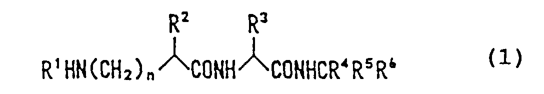 wherein R¹ represents a hydrogen atom or a lower alkyl group; R² represents a hydrogen atom or a substituted or unsubstituted lower alkyl, cycloalkyl, cycloalkyl-substituted alkyl, aralkyl or aryl group, or R¹ and R² may together form a substituted or unsubstituted alkylene group having 2 to 4 carbon atoms; R³ represents a substituted or unsubstituted lower alkyl or aralkyl group; R⁴ represents a hydrogen atom or a substituted or unsubstituted lower alkyl or aralkyl group; R⁵ represents a hydrogen atom or a substituted or unsubstituted lower alkyl, alkenyl or aralkyl group; R⁶ represents a hydrogen atom or a substituted or unsubstituted alkyl, alkenyl, cycloalkyl, aralkyl, aryl, N-alkoxyiminomethyl, N-aralkyloxyiminomethyl, aralkyloxycarbonyl or CONR⁷R⁸ group, in which R⁷ represents a hydrogen atom or a substituted or unsubstituted lower alkyl group; and R⁸ represents a substituted or unsubstituted alkyl, cycloalkyl, aralkyl or aryl group; and n is an integer of from 0 to 3, or a pharmaceutically acceptable salt thereof and an angiotensin IV receptor agonist comprising the same as an active ingredient.

Unless otherwise indicated, the terms as employed in the formulae herein have the following meanings. The lower alkyl group means a linear or branched C₁-C₆ alkyl group. Specific examples of the unsubstituted lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl groups.

The alkyl group is preferably a linear or branched C₁-C₁₂ alkyl group. Specific examples of the unsubstituted alkyl group include the lower alkyl groups as cited above and heptyl, octyl, nonyl, decyl and dodecyl groups and iso-, sec- and tert-compounds thereof.

The alkenyl group is preferably a linear or branched C₂-C₆ alkenyl group. Specific examples of the unsubstituted alkenyl group include vinyl, allyl, 1-propenyl, 1-butenyl, 2-butenyl, 3-butenyl, 2-methyl-1-propenyl, 2-methyl-2-propenyl, 1-pentenyl, 2-pentenyl, 3-pentenyl, 4-pentenyl, 3-methyl-1-butenyl, 3-methyl-2-butenyl, 3-methyl-3-butenyl, 2-methyl-1-butenyl, 2-methyl-2-butenyl, 2-methyl-3-butenyl, 2-ethyl-2-propenyl, 1-methyl-1-butenyl, 1-methyl-2-butenyl, 1-methyl-3-butenyl, 1-hexenyl, 2-hexenyl, 3-hexenyl, 4-hexenyl, 5-hexenyl, 4-methyl-3-pentenyl and 2,3-dimethyl-2-butenyl groups.

The cycloalkyl group is preferably a C₃-C₈ cycloalkyl group. Specific examples of the unsubstituted cycloalkyl group include cyclopropyl, cyclobutyl, cyclopentyl, cyclohexyl, cycloheptyl and cyclooctyl groups.

The cycloalkyl-substituted alkyl group is preferably a C₄-C₁₀ group. Specific examples of the unsubstituted cycloalkyl-substituted alkyl group include cyclopropylmethyl, cyclopropylbutyl, cyclobutylpropyl, cyclopentylethyl, cyclohexylmethyl, cycloheptylmethyl and cyclooctylethyl groups.

The aralkyl group means an alkyl group substituted with an aryl group including a heteroaryl group. Specific examples of the unsubstituted aralkyl group include benzyl, phenethyl, phenylpropyl, phenylbutyl, naphthylmethyl, naphthylethyl, naphthylpropyl, naphthylbutyl, diphenylmethyl, diphenylpropyl, diphenylbutyl, biphenylmethyl, biphenylethyl, biphenylpropyl, biphenylbutyl, pyrrolylmethyl, furylmethyl, thienylethyl, imidazolylpropyl, pyrazolylmethyl, oxazolylethyl, thiazolylbutyl, triazolylpentyl, thiadiazolylhexyl, pyridylmethyl, pyrazinylpropyl, pyrimidylheptyl, pyridazylethyl, indolylbutyl, benzofurylmethyl, benzothienylethyl, benzimidazolyloctyl, benzoxazolylethyl, benzothiazolylmethyl, benzotriazolylbutyl, quinolylmethyl, isoquinolylethyl, phthalazinylpropyl, pyrrolidinylmethyl, piperidinylethyl and piperazinylbutyl groups.

The aryl group includes a heteroaryl group. Specific examples of the unsubstituted aryl group include phenyl, naphthyl, biphenyl, anthranyl, pyrrolyl, furyl, thienyl, imidazolyl, pyrazolyl, oxazolyl, thiazolyl, triazolyl, thiadiazolyl, pyridyl, pyrazinyl, pyrimidyl, pyridazyl, indolyl, benzofuryl, benzothienyl, benzimidazolyl, benzoxazolyl, benzothiazolyl, benzotriazolyl, quinolyl, isoquinolyl, phthalazinyl, pyrrolidinyl, piperidinyl and piperazinyl groups.

The N-alkoxyiminomethyl group is preferablly an N-alkoxyiminomethyl group having a linear or branched, substituted or unsubstituted C₁-C₁₀ alkoxy group. Specific examples of the unsubstituted N-alkoxyiminomethyl group include N-methoxyiminomethyl, N-ethoxyiminomethyl, N-propoxyiminomethyl, N-isopropoxyiminomethyl, N-butoxyiminomethyl, N-isobutoxyiminomethyl, N-pentyloxyiminomethyl, N-hexyloxyiminomethyl, N-heptyloxyiminomethyl, N-octyloxyiminomethyl, N-nonyloxyiminomethyl and N-decyloxyiminomethyl groups.

The N-aralkyloxyiminomethyl group is preferably an N-aralkyloxyiminomethyl group having a benzyl, phenethyl or naphthyl group. Specific examples of the unsubstituted N-aralkyloxyiminomethyl group include N-benzyloxyiminomethyl, N-phenethyloxyiminomethyl and N-(naphthylmethoxy)iminomethyl groups.

The aralkyloxycarbonyl group is preferably an aralkyloxycarbonyl group having a benzyl, phenethyl or naphthyl group. Specific examples of the unsubstituted aralkyloxycarbonyl group include benzyloxycarbonyl, phenethyloxycarbonyl and naphthylmethoxycarbonyl groups.

The above-mentioned alkyl, alkenyl, cycloalkyl, cycloalkyl-substituted alkyl, aralkyl, aryl, N-alkoxyiminomethyl, N-aralkyloxyiminomethyl or aralkyloxycarbonyl groups may have a substituent, for example, a halogen atom (e.g., chlorine, bromine, iodine and fluorine atoms); a nitro group; a hydroxyl group; a formyl group; a carboxyl group; a cyano group; a carbamoyl group; an alkyl group (e.g., methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl groups); an aryl group (e.g., phenyl, p-tolyl, m-fluorophenyl, o-chlorophenylnaphthyl, biphenyl, anthranyl, pyrrolyl, furyl, thienyl, imidazolyl, pyrazolyl, oxazolyl, thiazolyl, triazolyl, thiadiazolyl, pyridyl, pyrazinyl, pyrimidyl, pyridazyl, indolyl, benzofuryl, benzothienyl, benzimidazolyl, benzoxazolyl, benzothiazolyl, benzotriazolyl, quinolyl, isoquinolyl, phthalazinyl, pyrrolidinyl, piperidinyl and piperazinyl groups); a perfluoroalkyl group (e.g., trifluoromethyl, pentafluoroethyl and 3,3,3-trifluoropropyl groups); an alkenyl group (e.g., vinyl and propenyl groups); an alkynyl group (e.g., ethenyl and propargyl groups); an alkoxycarbonyl group (e.g., methoxycarbonyl, ethoxycarbonyl, propyloxycarbonyl and benzyloxycarbonyl groups); an acyl group (e.g., acetyl, propionyl, butyryl, isobutyryl, valeryl, isovaleryl, pivaloyl, benzoyl, 4-methylbenzoyl, 3-methylbenzoyl, 2-methylbenzoyl, naphthoyl, nicotinoyl and isonicotinoyl groups); an amino group (e.g., amino, methylamino, ethylamino, propylamino, isopropylamino, butylamino, isobutylamino, sec-butylamino, tert-butylamino, pentylamino, isopentylamino, hexylamino, dimethylamino, diethylamino, dipropylamino, diisopropylamino, dibutylamino, ethylmethylamino, methylpropylamino and ethylpropylamino groups); an oxy group (e.g., methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutyloxy, 2-methylbutyloxy, 1,2-dimethylpropyloxy, hexyloxy, benzyloxy, p-methylbenzyloxy, o-fluorobenzyloxy, phenethyloxy, biphenylmethyloxy, pyridylmethyloxy, naphthylmethyloxy and phenoxy groups); a thio group (e.g., thiol, methylthio, ethylthio, propylthio, isopropylthio, butylthio, isobutylthio, sec-butylthio, tert-butylthio, pentylthio, hexylthio, benzylthio, phenethylthio, biphenylthio, pyridylthio, naphthylmethylthio and phenylthio groups); and a sulfonyl group (e.g., methanesulfonyl, benzylsulfonyl and benzenesulfonyl groups).

Among the peptide derivatives represented by the above formula (1) of the present invention, the compounds wherein R¹ is a hydrogen atom, i.e., those represented by the following formula (2) are preferred because they have a high activity: 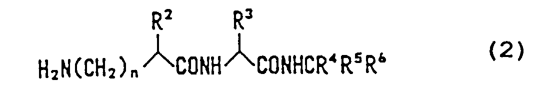 wherein R², R³, R⁴, R⁵, R⁶ and n each has the same meanings as defined above.

Among the peptide derivatives represented by the above formula (2) of the present invention, the compounds wherein n is 0, i.e., those represented by the following formula (3) are preferred because they have a high activity: 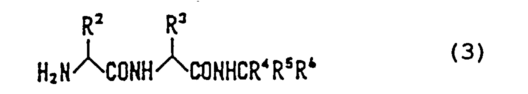 wherein R², R³, R⁴, R⁵ and R⁶ each has the same meanings as defined above.

Among the peptide derivatives represented by the above formula (3) of the present invention, the compounds wherein R³ is a 4-hydroxybenzyl group, i.e., those represented by the following formula (4) are preferred because they have a high activity: 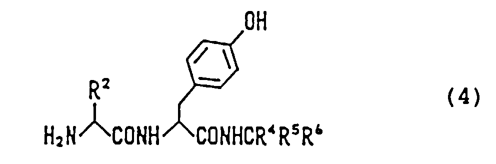 wherein R², R⁴, R⁵ and R⁶ each has the same meanings as defined above.

Among the peptide derivatives represented by the above formula (4) of the present invention, the compounds wherein R² is a substituted or unsubstituted lower alkyl or aralkyl group, i.e., those represented by the following formula (5) are preferred because they have a high activity: 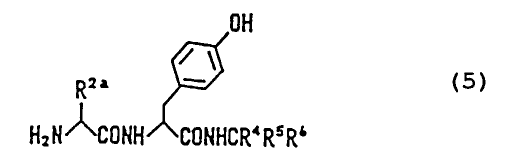 wherein R^{2a} represents a substituted or unsubstituted lower alkyl or aralkyl group; and R⁴, R⁵ and R⁶ each has the same meanings as defined above.

Among the peptide derivatives represented by the above formula (5) of the present invention, the compounds wherein R⁴ represents a substituted or unsubstituted lower alkyl group; R⁵ represents a hydrogen atom; R⁶ represents a CONR⁷R⁸ group; R⁷ represents a hydrogen atom or a substituted or unsubstituted lower alkyl group; and R⁸ represents a substituted or unsubstituted lower alkyl, cycloalkyl, aralkyl or aryl group, i.e., those represented by the following formula (6) are preferred because they have a high activity: 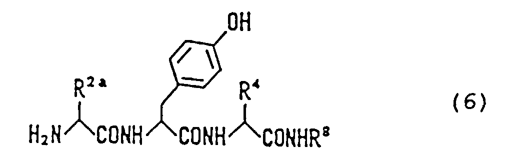 wherein R^{2a} represents a substituted or unsubstituted lower alkyl or aralkyl; R⁴ represents a substituted or unsubstituted lower alkyl group; and R⁸ represents a substituted or unsubstituted lower alkyl, cycloalkyl, aralkyl or aryl group.

Among the peptide derivatives represented by the above formula (6) of the present invention, those wherein R^{2a} represents a group selected from the group consisting of methyl, ethyl, propyl, isopropyl, butyl, isobutyl, sec-butyl and 2-methylthioethyl groups are preferred because they have a high activity.

Among the peptide derivatives represented by the above formula (5) of the present invention, the compounds wherein R⁴ represents a hydrogen atom; R⁵ represents a substituted or unsubstituted lower alkyl, alkenyl or aralkyl group; and R⁶ represents a substituted or unsubstituted lower alkyl, alkenyl, cycloalkyl, aralkyl, aryl, N-alkoxyiminomethyl, N-aralkyloxyiminomethyl or aralkyloxycarbonyl group, i.e., those represented by the following formula (7) are preferred because they have a high activity: 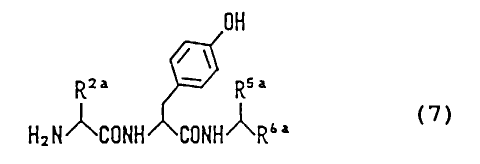 wherein R^{2a} represents a substituted or unsubstituted lower alkyl or aralkyl; R^{5a} represents a substituted or unsubstituted lower alkyl, alkenyl or aralkyl group; and R^{6a} represents a substituted or unsubstituted lower alkyl, alkenyl, cycloalkyl, aralkyl, aryl, N-alkoxyiminomethyl, N-aralkyloxyiminomethyl or aralkyloxycarbonyl group.

Among the peptide derivatives represented by the above formula (7) of the present invention, the dipeptide derivatives wherein R^{5a} represents a substituted or unsubstituted lower alkyl group; and R^{6a} represents a substituted or unsubstituted hydroxyalkyl, alkenyl, aralkyl, N-alkoxyiminomethyl or N-aralkyloxyiminomethyl group, i.e., those represented by the following formula (8) are preferred because they have a high activity: 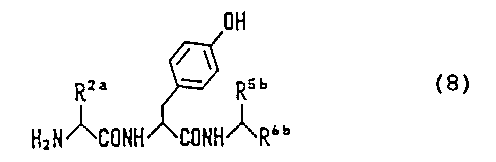 wherein R^{2a} is as defined above; R^{5a} represents a substituted or unsubstituted lower alkyl group; and R^{6b} represents a substituted or unsubstituted lower hydroxyalkyl, alkenyl, aralkyl, N-alkoxyiminomethyl or N-aralkyloxyiminomethyl group.

Among the dipeptide derivatives represented by the above formula (8) of the present invention, those wherein R^{2a} represents a group selected from the group consisting of propyl, isopropyl, butyl, isobutyl, sec-butyl and 2-methylthioethyl groups are preferred because they have a high activity.

The peptide derivatives represented by the above formula (1) of the present invention can form pharmaceutically acceptable salts thereof. Specific examples of these salts include, when acidic groups are contained therein, metal salts (e.g., lithium, sodium, potassium, magnesium and calcium salt), and ammonium salts (e.g., ammonium, methylammonium, dimethylammonium, trimethylammonium, tetramethylammonium and dicyclohexylammonium salts); and, when basic groups are contained therein, mineral acid salts (e.g., hydrochlorides, hydrobromides, sulfates, nitrates and phosphates), and organic acid salts (e.g., methanesulfonates, benzenesulfonates, p-toluenesulfonates, acetates, propionates, tartarates, fumarates, maleates, malates, oxalates, succinates, citrates, benzoates, mandelates and lactates). The stereochemistry of asymmetric carbon atoms in the peptide derivatives represented by the formula (1) may be (D)-, (L)- or (DL)-configuration independently.

Now, methods for producing the compounds of the present invention will be described. The above-mentioned peptide derivatives can be produced by, for example, the following methods.

### Method A:

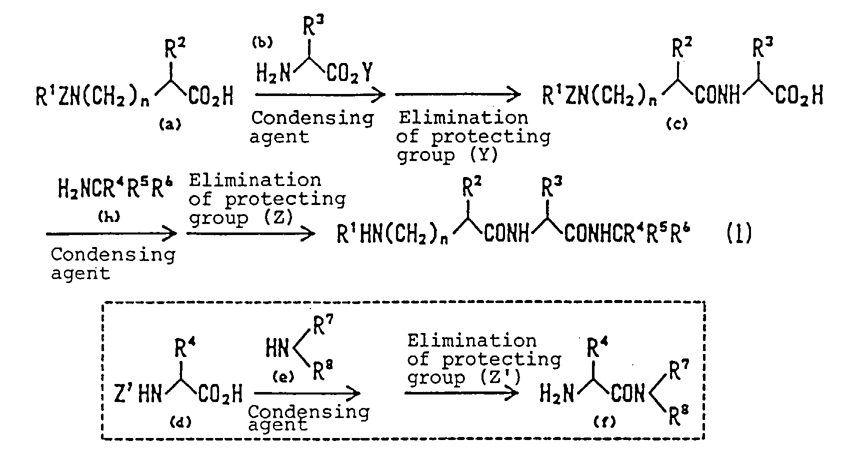

wherein R¹ to R⁸ each has the same meanings as defined above; Z and Z' each represents an amino-protective group; and Y represents a carboxyl-protective group.

That is, a carboxyl compound (a) is allowed to react with an amino compound (b) in the presence of a condensing agent and then the protecting group (Y) is eliminated to thereby give a compound (c). Next, this compound (c) is allowed to react with a compound (h) using a condensing agent and then the protecting group (z) is eliminated to thereby give a peptide derivative (1).

The compound (h) wherein R⁵ is a hydrogen atom and R⁶ is a CONR⁷R⁸ group can be obtained by reacting a carboxyl compound (d) with an amino compound (e) by using a condensing agent and then eliminating the protecting group (Z').

The amino-protective group for use in the above-mentioned method for producing the compounds of the present invention includes those which can be easily eliminated by acid hydrolysis or hydrogenolysis, for example, benzyloxycarbonyl, tert-butoxycarbonyl, formyl, chloroacetyl, trityl and trialkylsilyl groups. The carboxyl-protective group includes methyl, ethyl, benzyl and phenacyl groups.

### Method B:

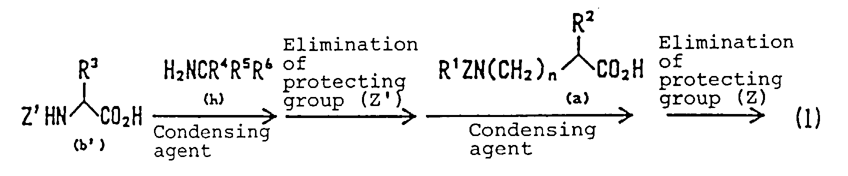

wherein R¹ to R⁶ each has the same meanings as defined above; Z and Z' each represents an amino-protective group; and Y represents a carboxyl-protective group.

That is, an amino compound (b') is allowed to react with another amino compound (h) in the presence of a condensing agent and then the protecting group (Z') is eliminated. Next, the obtained product is allowed to react with a carboxyl compound (a) using a condensing agent and then the protecting group (Z) is eliminated to thereby give a peptide derivative (1).

The peptide derivatives of the formula (1) wherein R⁵ is a hydrogen atom and R⁶ is a CONR⁷R⁸ group may be produced by the following method, too.

### Method C:

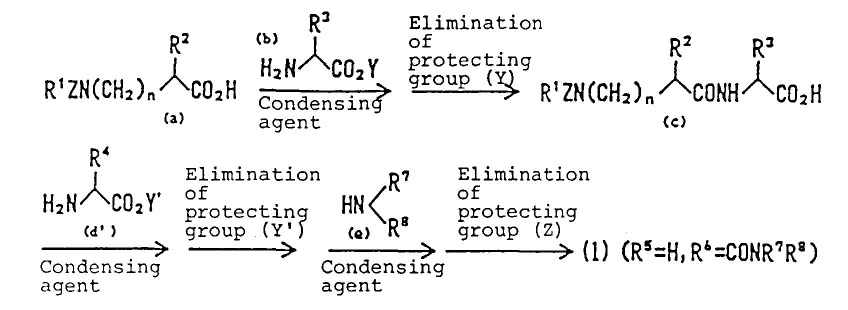

wherein R¹ to R⁸ each has the same meanings as defined above; Z represents an amino-protective group; and Y and Y' each represents a carboxyl-protective group.

That is, a carboxyl compound (a) is allowed to react with an amino compound (b) in the presence of a condensing agent and then the protecting group (Y) is eliminated. Next, the obtained product is allowed to react with another amino compound (d') using a condensing agent and then the protecting group (Y') is eliminated. Furthermore, the obtained product is allowed to react with another amino compound (e) by using a condensing agent and then the protecting group (Z) is eliminated to thereby give a peptide derivative (1).

In the above-mentioned methods A to C, the condensation reactions may be appropriately carried out, for example, by the method using N,N'-dicyclohexylcarbodiimide optionally together with 1-hydroxybenzotriazole, the method using 1-ethyl-3-(3-dimethylaminopropyl)carbodiimide with 1-hydroxybenzotriazole, the method using 1-ethyl-3-(3-dimethylaminopropyl)carbodiimide with 3-hydroxy-3,4-dihydro-4-oxo-1,2,3-bensotriazine, or the method using isobutyl chloroformate or diphenylphosphoryl azide in the presence of 1,1'-carbonyldiimidazole or triethylamine.

The protecting groups can be eliminated according to methods described in literature, i.e., hydrolysis using mineral acids (hydrofluoric acid, hydrochloric acid, hydrobromic acid, etc.), organic acids (trifluoroacetic acid, etc.) or alkali hydroxides (sodium hydroxide, barium hydroxide, etc.) or hydrogen reduction in the presence of palladium metal compounds [T.W. Greene *et al*., *Protective Groups in Organic Synthesis*, John Wiley & Sons, Inc., New York, pp 14-142, pp 397-405 (1991)].

To produce the compounds of the present invention, it is preferred to use solvents. Examples of the solvents include water, alcohols (e.g., methanol, ethanol, isopropanol), organic acids (e.g., acetic acid, propionic acid), esters (e.g., methyl acetate, ethyl acetate), ethers (e.g., diisopropyl ether, tetrahydrofuran, dioxane, anisole), aromatic hydrocarbons (e.g., benzene, toluene), halogenated aromatic hydrocarbons (e.g., dichloromethane, chloroform, dichloroethane), ketones (e.g., acetone, ethyl methyl ketone), nitriles (e.g., acetonitrile, propiononitrile, benzonitrile), aprotic polar solvents (e.g., dimethyl sulfoxide, N,N-dimethylformamide), and mixed solvents thereof. Any solvent may be used therefor, so long as it remains inert during the reaction. The reaction temperature ranges from -78 to 200°C. In particular, it is preferred that the condensation reactions are effected at -30 to 50°C and the protecting groups are eliminated at -30 to 100°C.

### BEST MODE FOR CARRYING OUT THE INVENTION

To further illustrate the present invention in greater detail, and not by way of limitation, the following Examples will be given.

### EXAMPLE 1

### Production of L-valyl-L-tyrosyl-N-(diphenylmethyl)-L-isoleucinamide

(1) N-(Benzyloxycarbonyl)-L-isoleucine (530 mg, 2.0 mmol), 1-hydroxybenzotriazole monohydrate (306 mg, 2.0 mmol) and N,N'-dicyclohexylcarbodiimide (412 mg, 2.0 mmol) were stirred in acetonitrile (8.0 ml) at room temperature for 30 minutes. To the reaction mixture were added a solution of diphenylmethylamine (366 mg, 2.0 mmol) in acetonitrile (3.0 ml) and tetrahydrofuran (20 ml) and the mixture was stirred at room temperature for 20 hours. After the reaction, the solvent was distilled off under reduced pressure and the residue was recrystallized from ethyl acetate to thereby give N'-(benzyloxycarbonyl)-N-(diphenylmethyl)-L-isoleucinamide (680 mg).
   ¹H-NMR (CDCl₃, 200 MHz) δ (ppm);
      0.87 (t, J = 6.7 Hz, 3H), 0.91 (d, J = 6.7 Hz, 3H), 1.11 (m, 1H), 1.50 (m, 1H), 1.90 (m, 1H), 4.06 (dd, J = 7.2, 8.7 Hz, 1H), 5.08 (s, 2H), 5.33 (m, 1H), 6.22 (d, J = 8.0 Hz, 1H), 6.60 (m, 1H), 7.12-7.42 (m, 15H)
(2) Under a hydrogen gas stream, N'-(benzyloxycarbonyl)-N-diphenylmethyl-L-isoleucinamide (430 mg, 0.35 mmol) and 10% palladium-carbon (120 mg) were stirred in a solution of methanol (12 ml) and tetrahydrofuran (4.0 ml) at room temperature for 5 hours. After filtering off the catalyst, the solvent was distilled off under reduced pressure to thereby give N-(diphenylmethyl)-L-isoleucinamide (280 mg).
   ¹H-NMR (CDCl₃, 200 MHz) δ (ppm);
      0.87 (t, J = 7.2 Hz, 3H), 0.95 (d, J = 7.0 Hz, 3H), 1.12 (m, 1H), 1.37 (m, 1H), 2.03 (m, 1H), 3.35 (d, J = 3.8 Hz, 1H), 6.25 (d, J = 8.5 Hz, 1H), 7.20-7.38 (m, 10H), 8.10 (d, J = 8.5 Hz, 1H)
(3) Under an argon gas stream, N-[N-(benzyloxycarbonyl)-L-valyl]-L-tyrosine (248 mg, 0.60 mmol) and 1-hydroxybenzotriazole monohydrate (136 mg, 0.66 mmol) were dissolved in tetrahydrofuran (10 ml). Then N,N'-dicyclohexylcarbodiimide (136 mg, 0.66 mmol) was added thereto and the resulting mixture was stirred at room temperature for 1 hour. To the reaction mixture were added a suspension of N-diphenylmethyl-L-isoleucinamide hydrochloride (200 mg, 0.60 mmol) and triethylamine (120 mg, 1.2 mmol) in tetrahydrofuran (10 ml) and the obtained mixture was stirred at room temperature for 8 hours. After the reaction, the organic matters were extracted with chloroform/methanol and washed with 1 N hydrochloric acid, a saturated aqueous solution of sodium hydrogencarbonate, water and a saturated aqueous solution of sodium chloride. The organic layer was dried over anhydrous sodium sulfate and the solvent was distilled off under reduced pressure. The solid thus obtained was purified by recrystallization to thereby give N'-[N-[N-(benzyloxycarbonyl)-L-valyl]-L-tyrosyl]-N-(diphenylmethyl)-L-isoleucinamide (220 mg).
   ¹H-NMR (DMSO-d₆, 400 MHz) δ (ppm);
      0.68-0.82 (m, 12H), 1.05 (m, 1H), 1.39 (m, 1H), 1.72 (m, 1H), 1.87 (m, 1H), 2.66 (dd, J = 9.4, 14.0 Hz, 1H), 2.84 (dd, J = 4.3, 14.0 Hz, 1H), 3.82 (dd, J = 7.2, 9.0 Hz, 1H), 4.33 (dd, J = 8.3, 8.3 Hz, 1H), 4.52 (m, 1H), 4.98 (d, J = 12.6 Hz, 1H), 5.05 (d, J = 12.6 Hz, 1H), 6.12 (d, J = 8.5 Hz, 1H), 6.59 (d, J = 8.4 Hz, 2H), 6.99 (d, J = 8.4 Hz, 2H), 7.17-7.40 (m, 16H), 7.90 (d, J = 9.0 Hz, 1H), 8.80 (d, J = 8.5 Hz, 1H), 9.13 (s, 1H)
(4) Using N'-[N-[N-(benzyloxycarbonyl)-L-valyl]-L-tyrosyl]-N-(diphenylmethyl)-L-isoleucinamide (145 mg, 0.28 mmol) and 10% palladium-carbon (35 mg), a reaction was performed in methanol (8.0 ml) and tetrahydrofuran (8.0 ml) in the same manner as in the above (2) to thereby give the title compound (90 mg).
   ¹H-NMR (DMSO-d₆, 400 MHz) δ (ppm);
      0.72-0.82 (m, 9H), 0.85 (d, J = 6.9 Hz, 3H), 1.06 (m, 1H), 1.39 (m, 1H), 1.73 (m, 1H), 1.98 (m, 1H), 2.68 (dd, J = 9.3, 13.9 Hz, 1H), 2.83 (dd, J = 4.8, 13.9 Hz, 1H), 3.27 (m, 1H), 4.34 (dd, J = 8.5, 8.5 Hz, 1H), 4.58 (m, 1H), 6.14 (d, J = 8.5 Hz, 1H), 6.60 (d, J = 8.5 Hz, 2H), 7.01 (d, J = 8.5 Hz, 2H), 7.18-7.38 (m, 10H), 8.13 (d, J = 9.0 Hz, 1H), 8.25 (d, J = 7.9 Hz, 1H), 8.94 (d, J = 8.5 Hz, 1H), 9.18 (s, 1H)
   MASS (m/e):
      558 (M⁺)

### EXAMPLE 2

### Production of L-valyl-L-tyrosyl-N-[(1,1'-biphenyl-4-yl)methyl]-L-isoleucinamide hydrochloride

(1) Using N-(benzyloxycarbonyl)-L-isoleucine (265 mg, 1.0 mmol), 1-hydroxybenzotriazole monohydrate (183 mg, 1.0 mmol), N,N'-dicyclohexylcarbodiimide (1.1 g, 5.5 mmol) and 4-biphenylmethylamine (265 mg, 1.0 mmol), a reaction was performed in acetonitrile (8.0 ml), tetrahydrofuran (1.0 ml) and dichloromethane (4.0 ml) in the same manner as in Example 1 (1) to thereby give N'-(benzyloxycarbonyl)-N-[(1,1'-biphenyl-4-yl)methyl]-L-isoleucinamide (182 mg).
   ¹H-NMR (CDCl₃, 200 MHz) δ (ppm);
      0.91 (t, J = 6.8 Hz, 3H), 0.96 (d, J = 6.7 Hz, 3H), 1.05-2.05 (m, 3H), 4.03 (dd, J = 6.6, 8.6 Hz, 1H), 4.45 (dd, 1H), 4.55 (dd, 1H), 5.09 (s, 2H), 5.35 (d, J = 8.3 Hz, 1H), 6.31 (m, 1H), 7.27-7.60 (m, 14H)
(2) Using N'-(benzyloxycarbonyl)-N-[(1,1'-biphenyl-4-yl)methyl]-L-isoleucinamide (150 mg, 0.35 mmol) and 10% palladium-carbon (40 mg), a reaction was performed in methanol (5.0 ml) and dichloroethane (5.0 ml) in the same manner as in Example 1 (2). After treating with 4 N hydrochloric acid/ethyl acetate solution, N-[(1,1'-biphenyl-4-yl)methyl]-L-isoleucinamide hydrochloride (93 mg) was obtained.
   ¹H-NMR (DMSO-d₆, 400 MHz) δ (ppm);
      0.86 (t, J = 7.3 Hz, 3H), 0.88 (d, J = 7.1 Hz, 3H), 1.00-2.00 (m, 3H), 3.68 (m, 1H), 4.27-4.56 (m, 2H), 7.23-7.80 (m, 9H), 9.04 (t, J = 5.4 Hz, 1H)
(3) N-[N-(tert-Butoxycarbonyl)-L-valyl]-L-tyrosine (76 mg, 0.20 mmol), 1-hydroxybenzotriazole monohydrate (34 mg, 0.22 mmol), N,N'-dicyclohexylcarbodiimide (45 mg, 0.22 mmol), N-[(1,1'-biphenyl-4-yl)methyl]-L-isoleucinamide hydrochloride (83 mg, 0.25 mmol) and triethylanine (70 mg, 0.70 mmol) were allowed to react in tetrahydrofuran (6.0 ml) in the same manner as in Example 1 (3) to thereby give N'-[N-[N-(tert-butoxycarbonyl)-L-valyl]-L-tyrosyl]-N-[(1,1'-biphenyl-4-yl)methyl]-L-isoleucinamide (93 mg).
   ¹H-NMR (DMSO-d₆, 400 MHz) δ (ppm);
      0.70 (d, J = 6.8 Hz, 3H), 0.73 (d, J = 6.8 Hz, 3H), 0.80 (d, J = 7.2 Hz, 3H), 0.81 (t, J = 6.7 Hz, 3H), 1.00 (m, 1H), 1.29 (m, 1H), 1.37 (s, 9H), 1.73 (m, 1H), 1.82 (m, 1H), 2.68 (dd, J = 9.0, 13.9 Hz, 1H), 2.85 (dd, J = 4.9, 13.9 Hz, 1H), 3.73 (dd, J = 7.4, 8.9 Hz, 1H), 4.19 (dd, J = 8.1, 8.4 Hz, 1H), 4.27 (dd, J = 5.9, 15.3 Hz, 1H), 4.34 (dd, J = 5.9, 15.3 Hz, 1H), 4.57 (m, 1H), 6.59 (d, J = 8.5 Hz, 2H), 6.67 (d, J = 8.9 Hz, 1H), 7.00 (d, J = 8.5 Hz, 2H), 7.29-7.70 (m, 9H), 7.83 (d, J = 8.1 Hz, 1H), 7.95 (d, J = 8.9 Hz, 1H), 8.36 (t, J = 5.9 Hz, 1H), 9.14 (s, 1H)
(4) Under an argon gas stream, N'-[N-[N-(tert-butoxycarbonyl)-L-valyl]-L-tyrosyl]-N-[(1,1'-biphenyl-4-yl)methyl]-L-isoleucinamide (55 mg, 0.083 mmol) was dissolved in ethyl acetate (1.0 ml), methanol (0.50 ml) and chloroform (2.0 ml). Under ice-cooling, a 4 N solution of hydrochloric acid/ethyl acetate (2.0 ml) was added thereto. The resulting mixture was stirred under ice-cooling for 1 hour and then at room temperature for 3 hours. After distilling off the solvent under reduced pressure, the obtained solid was washed with ether and dried to thereby give the title compound (47 mg).
   ¹H-NMR (DMSO-d₆, 400 MHz) δ (ppm);
      0.79 (t, J = 7.4 Hz, 3H), 0.81 (d, J = 6.8 Hz, 3H), 0.88 (d, J = 6.9 Hz, 3H), 0.93 (d, J = 6.9 Hz, 3H), 1.09 (m, 1H), 1.42 (m, 1H), 1.76 (m, 1H), 2.10 (m, 1H), 2.70 (dd, J = 9.0, 14.0 Hz, 1H), 2.88 (dd, J = 5.0, 14.0 Hz, 1H), 3.59 (d, J = 4.7 Hz, 1H), 4.21 (dd, J = 8.4, 8.7 Hz, 1H), 4.27 (dd, J = 5.8, 15.3 Hz, 1H), 4.34 (dd, J = 6.0, 15.3 Hz, 1H), 4.63 (m, 1H), 6.65 (d, J= 8.5 Hz, 2H), 7.08 (d, J= 8.5 Hz, 2H), 7.30-7.67 (m, 9H), 8.01 (br, 3H), 8.22 (d, J = 8.7 Hz, 1H), 8.39 (dd, J = 5.8, 6.0 Hz, 1H), 8.52 (d, J = 8.1 Hz, 1H), 9.24 (s, 1H)
   MASS (m/e):
      558 (M⁺)

### EXAMPLE 3

### Production of L-valyl-L-tyrosyl-N-benzyl-L-isoleucinamide

(1) Under an argon gas stream, N-(tert-butoxycarbonyl)-L-isoleucine (2.3 g, 10 mmol) and 1-hydroxybenzotriazole monohydrate (1.7 g, 11 mmol) were dissolved in tetrahydrofuran (60 ml) and then N,N'-dicyclohexylcarbodiimide (2.3mg, 11 mmol) was added thereto. After stirring the mixture at room temperature for 1 hour, benzylamine (1.2 g, 11 mmol) was added thereto and the obtained mixture was stirred for 17 hours. After the reaction, the insoluble matters were filtered off and the solvent was distilled off under reduced pressure. The residue was extracted with chloroform and dried with 1 N hydrochloric acid, a saturated aqueous solution of sodium hydrogencarbonate, water and a saturated aqueous solution of sodium chloride. Then it was dried over anhydrous sodium sulfate and the solvent was distilled off under reduced pressure to thereby give N'-(tert-butoxycarbonyl)-N-(benzyl)-L-isoleucinamide (2.83 g).
   ¹H-NMR (CDCl₃, 200 MHz) δ (ppm);
      0.92 (t, J = 7.4 Hz, 3H), 0.93 (d, J = 6.8 Hz, 3H), 1.10 (m, 1H), 1.41 (s, 9H), 1.51 (m, 1H), 1.92 (m, 1H), 3.94 (dd, J = 6.5, 8.7 Hz, 1H), 4.34 (m, 2H), 5.03 (m, 1H), 6.34 (m, 1H), 7.18-7.38 (m, 5H)
(2) Under an argon gas stream, N'-(tert-butoxycarbonyl)-N-benzyl-L-isoleucinamide (961 mg, 3.0 mmol) was dissolved in ethyl acetate (20 ml). Then a 4 N solution of hydrochloric acid/ethyl acetate (7.52 ml) was added thereto under ice-cooling and the obtained mixture was stirred for 1 hour. After stirring at room temperature for additional 4 hours and confirming the disappearance of the starting compounds, the solvent was distilled off under reduced pressure to thereby give N-benzyl-L-isoleucinamide hydrochloride (770 mg).
   ¹H-NMR (CDCl₃, 200 MHz) δ (ppm);
      0.83 (t, J = 6.8 Hz, 3H), 0.94 (d, J = 6.3 Hz, 3H), 1.17 (m, 1H), 1.55 (m, 1H), 2.06 (m, 1H), 4.08-4.57 (m, 3H), 7.08-7.38 (m, 5H), 8.23 (br, 3H), 8.44 (br, 1H)
(3) Under an argon gas stream, N-[N-(benzyloxycabronyl)-L-valyl]-L-tyrosine (231 mg, 0.60 mmol) and 1-hydroxybenzotriazole monohydrate (101 mg, 0.66 mmol) were dissolved in tetrahydrofuran (6.0 ml) and N,N'-dicyclohexylcarbodiimide (136 mg, 0.66 mmol) was added thereto. After stirring the reaction mixture at room temperature for 1 hour, a suspension of N-benzyl-L-isoleucinamide hydrochloride (153 mg, 0.60 mmol) in tetrahydrofuran (2.0 ml) neutralized with triethylamine (180 mg, 1.8 mmol) was added thereto. The resulting mixture was stirred for 19 hours. Then the solid matters were filtered and recrystallized from methanol/chloroform/diethyl ether to thereby give N'-[N-[N-(benzyloxycarbonyl)-L-valyl]-L-tyrosyl]-N-benzyl-L-isoleucinamide (220 mg).
   ¹H-NMR (DMSO-d₆, 400 MHz) δ (ppm);
      0.75 (d, J = 6.7 Hz, 3H), 0.75 (d, J = 6.7 Hz, 3H), 0.77 (d, J = 6.6 Hz, 3H), 0.78 (t, 3H), 1.06 (m, 1H), 1.40 (m, 1H), 1.72 (m, 1H), 1.88 (m, 1H), 2.68 (dd, J = 8.9, 13.9 Hz, 1H), 2.86 (dd, J = 4.9, 13.9 Hz, 1H), 3.83 (dd, J = 7.1, 8.9 Hz, 1H), 4.18 (dd, J = 8.3, 8.3 Hz, 1H), 4.23 (dd, J = 5.8, 15.4 Hz, 1H), 4.30 (dd, J = 5.8, 15.4 Hz, 1H), 4.99 (d, J = 12.6 Hz, 1H), 5.06 (d, J = 12.6 Hz, 1H), 6.60 (d, J = 8.3 Hz, 2H), 7.01 (d, J = 8.3 Hz, 2H), 7.18-7.42 (m, 11H), 7.87 (d, J = 8.8 Hz, 1H), 7.96 (d, J = 8.3 Hz, 1H), 8.31 (t, J = 5.8 Hz, 1H), 9.14 (s, 1H)
(4) Using N'-[N-[N-(benzyloxycarbonyl)-L-valyl]-L-tyrosyl]-N-benzyl-L-isoleucinamide (123 mg, 0.20 mmol) and 10% palladium-carbon (100 mg), a reaction was carried in methanol (10 ml) and tetrahydrofuran (10 ml) in the same manner as in Example 1 (4) to thereby give L-valyl-L-tyrosyl-N-benzyl-L-isoleucinamide (86 mg).
   ¹H-NMR (DMSO-d₆, 400 MHz) δ (ppm);
      0.67 (d, J = 6.8 Hz, 3H), 0.79 (t, J = 7.3 Hz, 3H), 0.79 (d, J = 7.3 Hz, 3H), 0.82 (d, J = 8.3 Hz, 3H), 1.06 (m, 1H), 1.41 (m, 1H), 1.73 (m, 1H), 1.90 (m, 1H), 2.70 (dd, J = 8.8, 13.8 Hz, 1H), 2.86 (dd, J = 4.9, 13.8 Hz, 1H), 3.06 (m, 2H), 4.19 (dd, J = 8.6, 8.6 Hz, 1H), 4.22-4.33 (m, 2H), 4.57 (m, 1H), 6.61 (d, J = 8.5 Hz, 2H), 6.99 (d, J = 8.5 Hz, 2H), 7.20-7.33 (m, 5H), 8.00 (d, J = 8.6 Hz, 1H), 8.16 (d, J = 8.6 Hz, 1H), 8.38 (t, J = 6.0 Hz, 1H), 9.15 (s, 1H)
   MASS (m/e):
      482 (M⁺+1)

### EXAMPLE 4

### Production of L-valyl-L-tyrosyl-N-(4-methylbenzyl)-L-isoleucinamide

(1) N-(tert-Butoxycarbonyl)-L-isoleucine (1.2 g, 5.0 mmol), 1-hydroxybenzotriazole monohydrate (0.84 g, 5.5 mmol), N,N'-dicyclohexylcarbodiimide (1.1 g, 5.5 mmol) and 4-methylbenzylamine (0.61 g, 5.0 mmol) were allowed to react in tetrahydrofuran (25 ml) in the same manner as in Example 3 (3) to thereby give N'-(tert-butoxycarbonyl)-N-(4-methylbenzyl)-L-isoleucinamide (1.7 g).
   ¹H-NMR (CDCl₃, 200 MHz) δ (ppm);
      0.90 (t, J = 7.3 Hz, 3H), 0.92 (d, J = 6.8 Hz, 3H), 1.11 (m, 1H), 1.41 (s, 9H), 1.52 (m, 1H), 1.90 (m, 1H), 2.32 (s, 3H), 3.93 (dd, J = 6.5, 8.7 Hz, 1H), 4.35 (dd, J = 5.6, 14.7 Hz, 1H), 4.43 (dd, J = 5.6, 14.7 Hz, 1H), 5.06 (m, 1H), 6.28 (m, 1H), 7.13 (s, 4H)
(2) N'-(tert-Butoxycarbonyl)-N-(4-methylbenzyl)-L-isoleucinamide (1.0 g, 3.0 mmol) and a 4 N solution of hydrochloric acid/ethyl acetate (7.9 ml) were allowed to react in ethyl acetate (20 ml) in the same manner as in Example 3 (2) to thereby give N-(4-methylbenzyl)-L-isoleucinamide hydrochloride (0.81 g).
   ¹H-NMR (DMSO-d₆, 400 MHz) δ (ppm);
      0.85 (t, J = 7.2 Hz, 3H), 0.89 (d, J = 6.8 Hz, 3H), 1.10 (m, 1H), 1.50 (m, 1H), 1.57 (m, 1H), 2.28 (m, 1H), 2.28 (s, 3H), 3.66 (m, 1H), 4.23 (dd, J = 5.9, 15.0 Hz, 1H), 4.34 (dd, J = 5.9, 15.0 Hz, 1H), 7.13 (d, J = 8.2 Hz, 2H), 7.19 (d, J = 8.2 Hz, 2H), 8.29 (m, 3H), 9.00 (t, J = 5.9 Hz, 1H)
(3) N-[N-(Benzyloxycarbonyl)-L-valyl]-L-tyrosine (384 mg, 1.0 mmol), 1-hydroxybenzotriazole monohydrate (168 mg, 11 mmol), N,N'-dicyclohexylcarbodiimide (226 mg, 1.1 mmol), N-(4-methylbenzyl)-L-isoleucinamide hydrochloride (270 mg, 1.0 mmol) and triethylamine (303 mg, 3.0 mmol) were allowed to react in tetrahydrofuran (20 ml) in the same manner as in Example 1 (3) to thereby give N'-[N-[N-(benzyloxycarbonyl)-L-valyl]-L-tyrosyl]-N-(4-methylbenzyl)-L-isoleucinamide (336 mg).
   ¹H-NMR (DMSO-d₆, 400 MHz) δ (ppm);
      0.75 (d, J = 6.7 Hz, 3H), 0.75 (d, J = 6.8 Hz, 3H), 0.78 (d, J = 6.6 Hz, 3H), 0.79 (t, J = 7.1 Hz, 3H), 1.06 (m, 1H), 1.40 (m, 1H), 1.71 (m, 1H), 1.88 (m, 1H), 2.68 (dd, J = 9.0, 13.8 Hz, 1H), 2.85 (dd, J = 4.9, 13.8 Hz, 1H), 3.83 (dd, J = 7.2, 8.9 Hz, 1H), 4.17 (dd, J = 8.2, 8.2 Hz, 1H), 4.14-4.26 (m, 1H), 4.53 (m, 1H), 4.98 (d, J = 12.4 Hz, 1H), 5.05 (d, J = 12.4 Hz, 1H), 6.60 (d, J = 8.4 Hz, 2H), 7.00 (d, J = 8.4 Hz, 2H), 7.10 (d, J = 8.4 Hz, 2H), 7.13 (d, J = 8.4 Hz, 2H), 7.21 (d, J = 8.9 Hz, 1H), 7.26-7.40 (m, 5H), 7.85 (d, J = 8.9 Hz, 1H), 7.96 (d, J = 8.2 Hz, 1H), 8.24 (t, J = 5.9 Hz, 1H), 9.16 (s, 1H)
(4) Using N'-[N-[N-(benzyloxycarbonyl)-L-valyl]-L-tyrosyl]-N-(4-methylbenzyl)-L-isoleucinamide (126 mg, 0.20 mmol) and 10% palladium-carbon (100 mg), a reaction was performed in methanol (15 ml) and tetrahydrofuran (10 ml) in the same manner as in Example 2 (4) to thereby give the title compound (85 mg).
   ¹H-NMR (DMSO-d₆, 400 MHz) δ (ppm);
      0.68 (d, J = 6.7 Hz, 3H), 0.78 (d, J = 6.7 Hz, 3H), 0.79 (t, 3H), 0.81 (d, J = 6.7 Hz, 3H), 1.04 (m, 1H), 1.42 (m, 1H), 1.72 (m, 1H), 1.91 (m, 1H), 2.69 (dd, J = 8.5, 13.4 Hz, 1H), 2.85 (dd, J = 4.9, 13.4 Hz, 1H), 3.06 (m, 1H), 4.17 (dd, J = 8.6, 8.6 Hz, 1H), 4.23 (d, J = 5.9 Hz, 3H), 4.57 (m, 1H), 6.60 (d, J = 8.5 Hz, 2H), 6.99 (d, J = 8.5 Hz, 2H), 7.10 (d, J = 8.4 Hz, 2H), 7.13 (d, J = 8.4 Hz, 2H), 7.21 (d, J = 8.9 Hz, 1H), 7.97 (d, J = 8.6 Hz, 1H), 8.10 (m, 1H), 8.32 (t, J = 5.9 Hz, 1H), 9.15 (s, 1H)
   MASS (m/e):
      496 (M⁺)

### EXAMPLE 5

### Production of L-valyl-L-tyrosyl-N-hexyl-L-isoleucinamide

(1) N-[N-(Benzyloxycabronyl)-L-valyl]-L-tyrosine (1.5 g, 4.0 mmol), 1-hydroxybenzotriazole monohydrate (673 mg, 4.4 mmol), N,N'-dicyclohexylcarbodiimide (906 mg, 4.4 mmol), L-isoleucine methyl ester hydrochloride (728 mg, 4.0 mmol) and triethylamine (1.2 g, 12 mmol) were allowed to react in tetrahydrofuran (40 ml) in the same manner as in Example 1 (3) to thereby give N-[N-[N-(benzyloxycarbonyl)-L-valyl]-L-tyrosyl]-L-isoleucine methyl (1.5 g).
   ¹H-NMR (DMSO-d₆, 400 MHz) δ (ppm);
      0.74 (d, J = 6.7 Hz, 6H), 0.82 (d, J = 6.8 Hz, 3H), 0.82 (t, J = 7.3 Hz, 3H), 1.18 (m, 1H), 1.39 (m, 1H), 1.76 (m, 1H), 1.87 (m, 1H), 2.66 (dd, J = 9.2, 13.9 Hz, 1H), 2.85 (dd, J = 5.0, 13.9 Hz, 1H), 3.60 (s, 3H), 3.82 (dd, J = 7.1, 9.0 Hz, 1H), 4.20 (dd, J = 7.0, 7.8 Hz, 1H), 4.56 (m, 1H), 4.99 (d, J = 12.6 Hz, 1H), 5.05 (d, J = 12.6 Hz, 1H), 6.61 (d, J = 8.4 Hz, 2H), 7.01 (d, J = 8.4 Hz, 2H), 7.21 (d, J = 9.0 Hz, 1H), 7.26-7.42 (m, 5H), 7.91 (d, J = 8.3 Hz, 1H), 8.13 (d, J = 7.8 Hz, 1H), 9.12 (s, 1H)
(2) N-[N-[N-(Benzyloxycarbonyl)-L-valyl]-L-tyrosyl]-L-isoleucine methyl (300 mg, 0.57 mmol) was dissolved in tetrahydrofuran (8.0 ml) and methanol (8.0 ml). After adding a 1 N aqueous solution of sodium hydroxide, the mixture was stirred at room temperature for 6 hours. After the completion of the reaction, the reaction mixture was neutralized with 1 N hydrochloric acid. The solid matter thus formed was separated by filtration and dried to thereby give N-[N-[N-(benzyloxycarbonyl)-L-valyl]-L-tyrosyl]-L-isoleucine (190 mg).
   ¹H-NMR (DMSO-d₆, 400 MHz) δ (ppm);
      0.71 (d, J = 6.9 Hz, 3H), 0.73 (t, J = 8.1 Hz, 3H), 0.83 (t, J = 7.5 Hz, 3H), 0.84 (d, J = 6.8 Hz, 3H), 1.19 (m, 1H), 1.40 (m, 1H), 1.76 (m, 1H), 1.88 (m, 1H), 2.67 (dd, J = 9.8, 13.9 Hz, 1H), 2.88 (dd, J = 4.3, 13.9 Hz, 1H), 3.81 (dd, J = 7.4, 9.1 Hz, 1H), 4.17 (dd, J = 6.0, 8.3 Hz, 1H), 4.55 (m, 1H), 4.99 (d, J = 12.6 Hz, 1H), 5.05 (d, J = 12.6 Hz, 1H), 6.63 (d, J = 8.4 Hz, 2H), 7.03 (d, J = 8.4 Hz, 2H), 7.24 (d, J = 9.1 Hz, 1H), 7.27-7.39 (m, 5H), 7.97 (d, J = 8.3 Hz, 1H), 8.02 (d, J = 8.3 Hz, 1H), 9.25 (s, 1H), 12.62 (s, 1H)
(3) N-[N-[N-(Benzyloxycarbonyl)-L-valyl]-L-tyrosyl]-L-isoleucine (132 mg, 0.25 mmol), 1-hydroxybenzotriazole monohydrate (42 mg, 0.28 mmol) and N,N'-dicyclohexylcarbodiimide (58 mg, 0.28 mmol) were allowed to react in tetrahydrofuran (8.0 ml) and N,N-dimethylformamide (5.0 ml) in the same manner as in Example 1 (3) to thereby give N'-[N-[N-(benzyloxycarbonyl)-L-valyl]-L-tyrosyl]-N-hexyl-L-isoleucinamide (115 mg).
   ¹H-NMR (DMSO-d₆, 400 MHz) δ (ppm);
      0.70-0.82 (m, 12H), 0.85 (t, J= 6.8 Hz, 3H), 0.97-1.94 (m, 12H), 2.68 (dd, J = 9.1, 13.9 Hz, 1H), 2.84 (dd, J = 5.1, 13.9 Hz, 1H), 2.92-3.10 (m, 2H), 3.83 (dd, J = 7.1, 8.9 Hz, 1H), 4.09 (dd, J = 8.0, 8.3 Hz, 1H), 4.52 (m, 1H), 4.99 (d, J = 12.5 Hz, 1H), 5.05 (d, J = 12.5 Hz, 1H), 6.60 (d, J = 8.4 Hz, 2H), 7.00 (d, J = 8.4 Hz, 2H), 7.22 (d, J = 9.0 Hz, 1H), 7.26-7.42 (m, 5H), 7.71 (t, J = 5.6 Hz, 1H), 7.77 (d, J = 9.0 Hz, 1H), 7.94 (d, J = 8.3 Hz, 1H), 9.12 (s, 1H)
(4) Using N'-[N-[N-(benzyloxycarbonyl)-L-valyl]-L-tyrosyl]-N-hexyl-L-isoleucinamide (100 mg, 0.16 mmol) and 10% palladium-carbon (50 mg), a reaction was performed in methanol (20 ml) and tetrahydrofuran (15 ml) in the same manner as in Example 1 (2) to thereby give the title compound (47 mg).
   ¹H-NMR (DMSO-d₆, 400 MHz) δ (ppm);
      0.63 (d, J = 6.8 Hz, 3H), 0.79 (d, J = 6.8 Hz, 6H), 0.80 (t, J = 7.0 Hz, 3H), 0.85 (t, J = 6.8 Hz, 3H), 1.17-1.76 (m, 12H), 1.86 (m, 1H), 2.70 (dd, J = 8.6, 14.0 Hz, 1H), 2.84 (dd, J = 4.5, 14.0 Hz, 1H), 2.92 (d, J = 4.7 Hz, 1H), 2.93-3.13 (m, 2H), 4.09 (dd, J = 7.8, 8.9 Hz, 1H), 4.54 (m, 1H), 6.59 (d, J = 8.5 Hz, 2H), 6.96 (d, J = 8.5 Hz, 2H), 7.81 (t, J = 5.7 Hz, 1H), 7.86 (d, J = 8.9 Hz, 1H), 7.99 (d, J = 8.3 Hz, 1H), 9.11 (s, 1H)
   MASS (m/e):
      476 (M⁺)

### EXAMPLE 6

### Production of L-valyl-L-tyrosyl-N-(2-phenylethyl)-L-isoleucinamide

(1) N-(tert-Butoxycarbonyl)-L-isoleucine (462 mg, 2.0 mmol), 1-hydroxybenzotriazole monohydrate (337 mg, 2.2 mmol), N,N'-dicyclohexylcarbodiimide (453 mg, 2.2 mmol) and 2-phenylethylamine (266 mg, 2.2 mol) were allowed to react in tetrahydrofuran (20 ml) in the same manner as in Example 3 (1) to thereby give N'-(tert-butoxycarbonyl)-N-(2-phenylethyl)-L-isoleucinamide (668 mg).
   ¹H-NMR (CDCl₃, 200 MHz) δ (ppm);
      0.87 (t, J = 7.1 Hz, 3H), 0.87 (d, J = 6.8 Hz, 3H), 1.05 (m, 1H), 1.42 (s, 9H), 1.67 (m, 1H), 1.84 (m, 1H), 2.81 (t, J = 7.0 Hz, 2H), 3.36-3.68 (m, 2H), 3.84 (dd, J = 6.3, 9.0 Hz, 1H), 5.00 (m, 1H), 5.96 (bs, 1H), 7.14-7.38 (m, 5H)
(2) N'-(tert-Butoxycarbonyl)-N-(2-phenylethyl)-L-isoleucinamide (668 mg, 2.0 mmol) and a 4 N solution of hydrochloric acid/ethyl acetate (8.0 ml) were allowed to react in ethyl acetate (15 ml) and chloroform (8.0 ml) in the same manner as in Example 3 (2) to thereby give N-(2-phenylethyl)-L-isoleucinamide hydrochloride (542 mg).
   ¹H-NMR (DMSO-d₆, 400 MHz) δ (ppm);
      0.79 (t, J = 6.6 Hz, 3H), 0.81 (d, J = 6.6 Hz, 3H), 1.02 (m, 1H), 1.40 (m, 1H), 1.74 (m, 1H), 2.76 (t, J = 7.2 Hz, 2H), 3.15-3.65 (m, 3H), 3.53 (m, 1H), 7.12-7.37 (m, 1H), 8.24 (br, 3H), 8.63 (t, J = 5.5 Hz, 1H)
(3) N-[N-(Benzyloxycarbonyl)-L-valyl]-L-tyrosine (116 mg, 0.30 mmol), 1-hydroxybenzotriazole monohydrate (51 mg, 0.33 mmol), N,N'-dicyclohexylcarbodiimide (0.68 mg, 0.33 mmol), N-(2-phenylethyl)-L-isoleucinamide hydrochloride (81 mg, 0.30 mmol) and triethylamine (90 mg, 0.90 mmol) were allowed to react in tetrahydrofuran (5.0 ml) in the same manner as in Example 1 (3) to thereby give N'-[N-[N-(benzyloxycarbonyl)-L-valyl]-L-tyrosyl]-N-(2-phenylethyl)-L-isoleucinamide (189 mg).
   ¹H-NMR (DMSO-d₆, 400 MHz) δ (ppm);
      0.70-0.85 (m, 12H), 1.00-1.97 (m, 4H), 2.69 (t, J = 7.4 Hz, 2H), 2.69 (dd, 1H), 2.85 (dd, J = 5.0, 14.0 Hz, 1H), 3.19-3.38 (m, 2H), 3.83 (dd, J = 7.2, 8.9 Hz, 1H), 4.08 (dd, J = 7.7, 8.4 Hz, 1H), 4.51 (m, 1H), 4.98 (d, J = 12.6 Hz, 1H), 5.05 (d, J = 12.6 Hz, 1H), 6.62 (d, J = 8.4 Hz, 2H), 7.01 (d, J = 8.4 Hz, 2H), 7.14-7.42 (m, 11H), 7.82 (d, J = 8.9 Hz, 1H), 7.89 (t, J = 5.5 Hz, 1H), 8.01 (d, J = 8.4 Hz, 1H), 9.21 (s, 1H)
(4) Using N'-[N-[N-(benzyloxycarbonyl)-L-valyl]-L-tyrosyl]-N-(2-phenylethyl)-L-isoleucinamide (100 mg, 0.16 mmol) and 10% palladium-carbon (70 mg), a reaction was performed in methanol (10 ml) and tetrahydrofuran (10 ml) in the same manner as in Example 1 (2) to thereby give the title compound (43 mg).
   ¹H-NMR (DMSO-d₆, 400 MHz) δ (ppm);
      0.63 (d, J = 6.7 Hz, 3H), 0.73 (d, J = 8.8 Hz, 3H), 0.78 (t, J = 7.4 Hz, 3H), 0.79 (d, J = 6.9 Hz, 3H), 1.05 (m, 1H), 1.35 (m, 1H), 1.64 (m, 1H), 1.86 (m, 1H), 2.71 (t, J = 7.4 Hz, 2H), 2.71 (dd, J = 8.7, 13.8 Hz, 1H), 2.84 (dd, J = 5.0, 13.8 Hz, 1H), 2.92 (d, J = 4.8 Hz, 1H), 3.19-3.40 (m, 2H), 4.09 (dd, J = 7.8, 8.8 Hz, 1H), 4.54 (m, 1H), 6.60 (d, J = 8.5 Hz, 2H), 6.80 (d, J = 8.5 Hz, 2H), 7.16-7.32 (m, 5H), 7.85 (d, J = 8.8 Hz, 1H), 7.92 (t, J = 5.6 Hz, 1H), 7.99 (d, J = 8.4 Hz, 1H), 9.11 (s, 1H)
   MASS (m/e):
      496 (M⁺)

### EXAMPLE 7

### Production of L-valyl-L-tyrosyl-N-(4-methoxyphenyl)-L-isoleucinamide

(1) N-(tert-Butoxycarbonyl)-L-isoleucine (462 mg, 2.0 mmol), 1-hydroxybenzotriazole monohydrate (337 mg, 2.2 mmol), N,N'-dicyclohexylcarbodiimide (453 mg, 2.2 mmol) and 4-methoxyaniline (246 mg, 2.0 mmol) were allowed to react in tetrahydrofuran (10 ml) in the same manner as in Example 3 (1) to thereby give N'-(tert-butoxycarbonyl)-N-(4-methoxyphenyl)-L-isoleucinamide (670 mg).
   ¹H-NMR (CDCl₃, 200 MHz) δ (ppm);
      0.93 (t, J = 7.3 Hz, 3H), 0.99 (d, J = 6.4 Hz, 3H), 1.17 (m, 1H), 1.45 (s, 9H), 1.62 (m, 1H), 1.98 (m, 1H), 3.76 (s, 3H), 4.04 (dd, J = 6.9, 8.7 Hz, 1H), 5.13 (m, 1H), 6.83 (d, J = 9.0 Hz, 2H), 7.40 (d, J = 9.0 Hz, 2H), 7.90 (m, 1H)
(2) N'-(tert-Butoxycarbonyl)-N-(4-methoxyphenyl)-L-isoleucinamide (336 mg, 1.0 mol) and a 4 N solution of hydrochloric acid/ethyl acetate (4.5 ml) were allowed to react in ethyl acetate (10 ml) and chloroform (5.0 ml) in the same manner as in Example 3 (2) to thereby give N-(4-methoxyphenyl)-L-isoleucinamide hydrochloride (273 mg).
   ¹H-NMR (DMSO-d₆, 400 MHz) δ (ppm);
      0.88 (t, J = 7.3 Hz, 3H), 0.95 (d, J = 6.9 Hz, 3H), 1.18 (m, 1H), 1.59 (m, 1H), 1.92 (m, 1H), 3.73 (s, 3H), 3.82 (m, 1H), 6.92 (d, J = 9.0 Hz, 2H), 7.54 (d, J = 9.0 Hz, 2H), 8.33 (bs, 3H), 10.60 (bs, 1H)
(3) N-[N-(Benzyloxycarbonyl)-L-valyl]-L-tyrosine (154 mg, 0.40 mmol), 1-hydroxybenzotriazole monohydrate (67 mg, 0.44 mmol), N,N'-dicyclohexylcarbodiimide (91 mg, 0.44 mmol), N-(4-methoxyphenyl)-L-isoleucinamide hydrochloride (154 mg, 0.40 mmol) and triethylamine (120 mg, 0.12 mmol) were allowed to react in tetrahydrofuran (6.0 ml) in the same manner as in Example 1 (3) to thereby give N'-[N-[N-(benzyloxycarbonyl)-L-valyl]-L-tyrosyl]-N-(4-methoxyphenyl)-L-isoleucinamide (163 mg).
   ¹H-NMR (DMSO-d₆, 400 MHz) δ (ppm);
      0.76 (d, J = 6.7 Hz, 6H), 0.82 (t, J = 7.4 Hz, 3H), 0.85 (d, J = 6.9 Hz, 3H), 1.12 (m, 1H), 1.49 (m, 1H), 1.77 (m, 1H), 1.89 (m, 1H), 2.70 (dd, J = 9.1, 13.9 Hz, 1H), 2.88 (dd, J = 4.4, 13.9 Hz, 1H), 3.71 (s, 3H), 3.84 (dd, J = 7.4, 8.8 Hz, 1H), 4.28 (dd, J = 8.5, 8.5 Hz, 1H), 4.56 (m, 1H), 4.99 (d, J = 12.6 Hz, 1H), 5.06 (d, J = 12.6 Hz, 1H), 6.57 (d, J = 8.3 Hz, 2H), 6.88 (d, J = 9.0 Hz, 2H), 7.01 (d, J = 8.3 Hz, 2H), 7.23 (d, J = 8.8 Hz, 1H), 7.26-7.42 (m, 5H), 7.50 (d, J = 9.0 Hz, 2H), 7.92 (d, J = 8.2 Hz, 1H), 8.00 (d, J = 8.5 Hz, 1H), 9.10 (s, 1H), 9.89 (s, 1H)
(4) Using N'-[N-[N-(Benzyloxycarbonyl)-L-valyl]-L-tyrosyl]-N-(4-methoxyphenyl)-L-isoleucinamide (100 mg, 0.16 mmol) and 10% palladium-carbon (50 mg), a reaction was performed in methanol (20 ml) and N,N-dimethylformamide (30 ml) in the same manner as in Example 1 (2) to thereby give the title compound (80 mg).
   ¹H-NMR (DMSO-d₆, 400 MHz) δ (ppm);
      0.81 (d, J = 6.8 Hz, 3H), 0.82 (t, J = 7.5 Hz, 3H), 0.85 (d, J = 6.8 Hz, 3H), 0.89 (t, J = 6.9 Hz, 3H), 1.14 (m, 1H), 1.50 (m, 1H), 1.79 (m, 1H), 2.04 (m, 1H), 2.71 (dd, J = 9.0, 14.0 Hz, 1H), 2.88 (dd, J = 4.0, 14.0 Hz, 1H), 3.40 (m, 1H), 3.72 (s, 3H), 4.29 (dd, J = 8.6, 8.6 Hz, 1H), 4.62 (m, 1H), 6.59 (d, J = 8.4 Hz, 2H), 6.88 (d, J = 9.1 Hz, 2H), 7.04 (d, J = 8.4 Hz, 2H), 7.51 (d, J = 9.1 Hz, 2H), 8.26 (d, J = 8.6 Hz, 1H), 8.33 (d, J = 8.1 Hz, 1H), 9.14 (s, 1H), 9.95 (s, 1H)
   MASS (m/e):
      498 (M⁺)

### EXAMPLE 8

### Production of L-valyl-L-tyrosyl-N-(2-phenylethyl)-L-leucinamide

(1) N-(Benzyloxycarbonyl)-L-leucine (1.6 g, 6.0 mmol), 1-hydroxybenzotriazole monohydrate (1.0 g, 6.6 mmol), N,N'-dicyclohexylcarbodiimide (1.4 g, 6.6 mmol) and 2-phenylethylamine (0.73 g, 6.0 mmol) were allowed to react in tetrahydrofuran (60 ml) in the same manner as in Example 1 (3) to thereby give N'-(benzyloxycarbonyl)-N-(2-phenylethyl)-L-leucinamide (2.2 g).
   ¹H-NMR (CDCl₃, 200 MHz) δ (ppm);
      0.89 (t, J = 6.1 Hz, 6H), 1.33-1.70 (m, 3H), 2.79 (t, J = 6.9 Hz, 2H), 3.36-3.63 (m, 2H), 4.08 (m, 1H), 5.08 (s, 2H), 5.15 (m, 1H), 6.04 (m, 1H), 7.12-7.40 (m, 10H)
(2) Using N'-(Benzyloxycarbonyl)-N-(2-phenylethyl)-L-leucinamide (1.5 g, 4.1 mmol) and 10% palladium-carbon, a reaction was performed in methanol (60 ml) in the same manner as in Example 1 (2) to thereby give N-(2-phenylethyl)-L-leucinamide (0.96 g).
   ¹H-NMR (CDCl₃, 200 MHz) δ (ppm);
      0.91 (d, J = 6.0 Hz, 3H), 0.94 (d, J = 6.0 Hz, 3H), 1.27 (m, 1H), 1.51-1.56 (m, 2H), 2.81 (t, J = 6.9 Hz, 2H), 3.33 (dd, J = 3.5, 9.7 Hz, 1H), 3.51 (dt, J = 6.9, 6.9 Hz, 2H), 7.15-7.37 (m, 6H)
(3) N-[N-(Benzyloxycarbonyl)-L-valyl]-L-tyrosine (154 mg, 0.40 mmol), N-(2-phenylethyl)-L-leucinamide (94 mg, 0.40 mmol), 1-hydroxybenzotriazole monohydrate (1.0 g, 6.6 mmol) and N,N'-dicyclohexylcarbodiimide (1.4 g, 6.6 mmol) were allowed to react in tetrahydrofuran (60 ml) in the same manner as in Example 1 (3) to thereby give N'-[N-[N-(benzyloxycarbonyl)-L-valyl]-L-tyrosyl]-N-(2-phenylethyl)-L-leucinamide (180 mg).
   ¹H-NMR (DMSO-d₆, 400 MHz) δ (ppm);
      0.76 (d, J = 6.9 Hz, 6H), 0.78 (d, J = 7.9 Hz, 3H), 0.83 (d, J = 6.5 Hz, 3H), 1.31-1.42 (m, 2H), 1.48 (m, 1H), 1.88 (m, 1H), 2.67 (t, J = 7.3 Hz, 2H), 2.68 (dd, 1H), 2.85 (dd, J = 5.4, 13.9 Hz, 1H), 3.16-3.29 (m, 2H), 3.83 (dd, J = 7.2, 8.5 Hz, 1H), 4.20 (dt, 1H), 4.49 (m, 1H), 4.99 (d, J = 12.6 Hz, 1H), 5.05 (d, J = 12.6 Hz, 1H), 6.63 (d, J = 8.3 Hz, 2H), 7.02 (d, J = 8.3 Hz, 2H), 7.13-7.41 (m, 11H), 7.61 (t, J = 5.5 Hz, 1H), 7.93 (d, J = 8.4 Hz, 1H), 7.97 (d, J = 8.2 Hz, 1H), 9.19 (s, 1H)
(4) Using N'-[N-[N-(benzyloxycarbonyl)-L-valyl]-L-tyrosyl]-N-(2-phenylethyl)-L-leucinamide (140 mg, 0.22 mmol) and 10% palladium-carbon (75 mg), a reaction was performed in methanol (30 ml) and tetrahydrofuran (15 ml) in the same manner as in Example 1 (2) to thereby give the title compound (70 mg).
   ¹H-NMR (DMSO-d₆, 400 MHz) δ (ppm);
      0.65 (d, J = 6.8 Hz, 3H), 0.79 (d, J = 6.4 Hz, 3H), 0.79 (d, J = 6.9 Hz, 3H), 0.83 (d, J = 6.5 Hz, 3H), 1.31-1.41 (m, 2H), 1.47 (m, 1H), 1.86 (m, 1H), 2.68 (t, J = 7.3 Hz, 2H), 2.70 (dd, 1H), 2.84 (dd, J = 5.4, 13.8 Hz, 1H), 2.97 (d, J = 4.7 Hz, 1H), 3.17-3.32 (m, 2H), 4.21 (m, 1H), 4.50 (m, 1H), 6.61 (d, J = 8.5 Hz, 2H), 6.99 (d, J = 8.5 Hz, 2H), 7.16-7.24 (m, 3H), 7.24-7.31 (m, 2H), 7.73 (t, J = 5.6 Hz, 1H), 8.01 (d, J = 8.3 Hz, 1H), 8.01 (m, 1H), 9.20 (s, 1H)
   MASS (m/e):
      496 (M⁺)

### EXAMPLE 9

### Production of L-valyl-L-tyrosyl-N-(4-pyridylmethyl)-L-isoleucinamide

(1) N-[N-[N-(Benzyloxycarbonyl)-L-valyl]-L-tyrosyl]-L-isoleucine (263 mg, 0.50 mmol), 1-hydroxybenzotriazole monohydrate (84 mg, 0.54 mmol) and N,N'-dicyclohexylcarbodiimide (113 mg, 0.55 mmol) were allowed to react in N,N-dimethylformamide (10 ml) in the same manner as in Example 1 (3) and treated with 1 N hydrochloric acid to thereby give N'-[N-[N-(benzyloxycarbonyl)-L-valyl]-L-tyrosyl]-N-(4-pyridylmethyl)-L-isoleucinamide hydrochloride (130 mg).
   ¹H-NMR (DMSO-d₆, 400 MHz) δ (ppm);
      0.74 (d, J = 6.6 Hz, 3H), 0.75 (d, J = 6.4 Hz, 3H), 0.81 (t, J = 7.3 Hz, 3H), 0.82 (d, J = 6.9 Hz, 3H), 1.05 (m, 1H), 1.42 (m, 1H), 1.77 (m, 1H), 1.88 (m, 1H), 2.70 (dd, J = 9.0, 13.8 Hz, 1H), 2.87 (dd, J = 4.6, 13.8 Hz, 1H), 3.83 (dd, J = 7.3, 8.6 Hz, 1H), 4.18 (dd, J = 7.9, 7.9 Hz, 1H), 4.43-4.59 (m, 2H), 4.55 (m, 1H), 4.98 (d, J = 12.6 Hz, 1H), 5.05 (d, J = 12.6 Hz, 1H), 5.61 (br, 1H), 6.59 (d, J = 8.3 Hz, 2H), 7.02 (d, J = 8.3 Hz, 2H), 7.25 (d, J = 8.8 Hz, 1H), 7.26-7.41 (m, 5H), 7.78 (d, J = 6.0 Hz, 2H), 8.00 (d, J = 7.9 Hz, 1H), 8.58 (t, J = 5.8 Hz, 1H), 8.81 (d, J = 6.0 Hz, 2H), 9.16 (br, 1H)
(2) Under an argon gas stream, N'-[N-[N-(benzyloxycarbonyl)-L-valyl]-L-tyrosyl]-N-(4-pyridylmethyl)-L-isoleucinamide hydrochloride (80 mg, 0.12 mmol) was dissolved in methanol (15 ml) and tetrahydrofuran (20 ml). To the obtained solution was added 10% palladium-carbon (30 mg). After replacing the argon by hydrogen, the reaction mixture was stirred at room temperature until the starting compounds disappeared. After filtering off the solid matters through celite, a 4 N solution of hydrochloric acid/ethyl acetate (1.5 ml) was added. Then the solvent was distilled off under reduced pressure and the residue was concentrated and purified by thin layer chromatography to thereby give the title compound (40 mg).
   ¹H-NMR (DMSO-d₆, 400 MHz) δ (ppm);
      0.70 (d, J = 6.8 Hz, 3H), 0.80 (t, J = 7.3 Hz, 3H), 0.81 (d, J = 7.1 Hz, 3H), 0.83 (d, J = 7.3 Hz, 3H), 1.09 (m, 1H), 1.42 (m, 1H), 1.75 (m, 1H), 1.93 (m, 1H), 2.70 (dd, J = 8.9, 14.0 Hz, 1H), 2.86 (dd, J = 4.9, 14.0 Hz, 1H), 3.13 (m, 1H), 4.18 (dd, J = 8.6, 8.6 Hz, 1H), 4.26 (dd, J = 5.8, 16.2 Hz, 1H), 4.32 (dd, J = 6.4, 16.2 Hz, 1H), 4.59 (m, 1H), 6.61 (d, J = 8.5 Hz, 2H), 7.01 (d, J = 8.5 Hz, 2H), 7.23 (d, J = 6.0 Hz, 2H), 8.11 (d, J = 8.6 Hz, 1H), 8.17 (m, 1H), 8.48 (d, J = 6.0 Hz, 2H), 8.51 (t, J = 6.0 Hz, 1H), 9.18 (s, 1H)
   MASS (m/e):
      520 (M⁺)

### EXAMPLE 10

### Production of L-valyl-L-tyrosyl-N-(2-phenylethyl)-L-norleucinamide hydrochloride

(1) N-(tert-Butoxycarbonyl)-L-norleucine (462 mg, 2.0 mmol), 1-hydroxybenzotriazole monohydrate (337 mg, 2.2 mmol), N,N'-dicyclohexylcarbodiimide (453 mg, 2.2 mmol) and 2-phenylethylamine (242 mg, 2.0 mmol) were allowed to react in tetrahydrofuran (20 ml) in the same manner as in Example 3 (1) to thereby give N'-(tert-butoxycarbonyl)-N-(2-phenylethyl)-L-norleucinamide (668 mg).
   ¹H-NMR (CDCl₃, 200 MHz) δ (ppm);
      0.88 (t, J = 6.7 Hz, 3H), 1.14-1.42 (m, 4H), 1.42 (s, 9H), 2.81 (t, J = 7.0 Hz, 2H), 3.37-3.68 (m, 2H), 3.96 (m, 1H), 4.95 (m, 1H), 6.08 (m, 1H), 7.10-7.40 (m, 5H)
(2) N'-(tert-Butoxycarbonyl)-N-(2-phenylethyl)-L-norleucinamide (568 mg, 1.7 mmol) and a 4 N solution of hydrochloric acid/ethyl acetate (5.3 ml) were allowed to react in ethyl acetate (17 ml) in the same manner as in Example 3 (2) to thereby give N-(2-phenylethyl)-L-norleucinamide hydrochloride (460 mg).
   ¹H-NMR (CDCl₃, 200 MHz) δ (ppm);
      0.82 (t, J = 6.8 Hz, 3H), 0.99-1.37 (m, 4H), 2.75 (t, J = 6.9 Hz, 2H), 3.15-3.60 (m, 2H), 3.66 (m, 1H), 7.13-7.38 (m, 5H), 8.25 (bs, 3H), 8.65 (t, J = 5.5 Hz, 1H)
(3) N-[N-(Benzyloxycarbonyl)-L-valyl]-L-tyrosine (232 mg, 0.60 mmol), N-(2-phenylethyl)-L-norleucinamide (163 mg, 0.60 mmol), 1-hydroxybenzotriazole monohydrate (102 mg, 0.66 mmol) and N,N'-dicyclohexylcarbodiimide (136 mg, 0.66 mmol) were allowed to react in tetrahydrofuran (6.0 ml) in the same manner as in Example 1 (3) to thereby give N'-[N-[N-(benzyloxycarbonyl)-L-valyl]-L-tyrosyl]-N-(2-phenylethyl)-L-norleucinamide (206 mg).
   ¹H-NMR (DMSO-d₆, 400 MHz) δ (ppm);
      0.75 (d, J = 6.6 Hz, 6H), 0.81 (d, J = 7.0 Hz, 3H), 1.08-1.30 (m, 4H), 1.56-1.66 (m, 2H), 1.88 (m, 1H), 2.67 (t, J = 7.3 Hz, 2H), 2.69 (dd, 1H), 2.86 (dd, J = 5.1, 13.9 Hz, 1H), 3.16-3.30 (m, 2H), 3.83 (dd, J = 7.4, 8.7 Hz, 1H), 4.13 (m, 1H), 4.49 (m, 1H), 4.99 (d, J = 12.6 Hz, 1H), 5.05 (d, J = 12.6 Hz, 1H), 6.62 (d, J = 8.4 Hz, 2H), 7.02 (d, J = 8.4 Hz, 2H), 7.15-7.42 (m, 11H), 7.68 (t, J = 5.5 Hz, 1H), 7.89 (d, J = 8.1 Hz, 1H), 7.96 (d, J = 8.2 Hz, 1H), 9.16 (s, 1H)
(4) Under an argon gas stream, N'-[N-[N-(benzyloxycarbonyl)-L-valyl]-L-tyrosyl]-N-(2-phenylethyl)-L-norleucinamide (100 mg, 0.16 mmol) was dissolved in methanol (10 ml) and tetrahydrofuran (5.0 ml). To the obtained solution were added 10% palladium-carbon (30 mg) and a 4 N solution of hydrochloric acid/ethyl acetate (0.20 ml). After replacing argon by hydrogen, the reaction mixture was stirred at room temperature until the starting compounds disappeared. After filtering off the solid matters through celite, the solvent was distilled off under reduced pressure and the residue was reprecipitated from chloroform/diethyl ether to thereby give the title compound (60 mg).
   ¹H-NMR (DMSO-d₆, 400 MHz) δ (ppm);
      0.81 (t, J = 7.0 Hz, 3H), 0.88 (d, J = 6.9 Hz, 3H), 0.92 (d, J = 6.9 Hz, 3H), 1.06-1.28 (m, 4H), 1.42 (m, 1H), 1.56 (m, 1H), 2.09 (m, 1H), 2.68 (t, J = 7.2 Hz, 2H), 2.68 (dd, J = 9.1, 13.1 Hz, 1H), 2.88 (dd, J = 5.5, 13.1 Hz, 1H), 3.17-3.32 (m, 2H), 3.59 (m, 1H), 4.16 (m, 1H), 4.57 (m, 1H), 6.66 (d, J = 8.5 Hz, 2H), 7.07 (d, J = 8.5 Hz, 2H), 7.15-7.24 (m, 3H), 7.24-7.32 (m, 2H), 7.68 (t, J = 5.7 Hz, 1H), 8.02 (bs, 3H), 8.14 (d, J = 8.2 Hz, 1H), 8.54 (d, J = 8.2 Hz, 1H), 9.24 (s, 1H)
   MASS (m/e):
      496 (M⁺)

### EXAMPLE 11

### Production of L-valyl-L-tyrosyl-N,N-diethyl-L-isoleucinamide hydrochloride

(1) N-(tert-Butoxycarbonyl)-L-isoleucine (691 mg, 3.0 mmol), diethylamine (263 mg, 3.6 mmol), 1-hydroxybenzotriazole monohydrate (680 mg, 3.3 mmol) and N,N'-dicyclohexylcarbodiimide (680 mg, 3.3 mmol) were allowed to react in tetrahydrofuran (15 ml) in the same manner as in Example 3 (1) to thereby give N'-(tert-butoxycarbonyl)-N,N-diethyl-L-isoleucinamide (858 mg).
   ¹H-NMR (CDCl₃, 200 MHz) δ (ppm);
      0.87 (t, J = 7.0 Hz, 3H), 0.89 (d, J = 6.9 Hz, 3H), 1.02-1.82 (m, 3H), 1.10 (t, J = 7.1 Hz, 3H), 1.21 (t, J = 7.1 Hz, 3H), 1.41 (s, 9H), 3.15 (dq, J = 13.5, 7.1 Hz, 1H), 3.28-3.51 (m, 2H), 3.59 (dq, J = 13.6, 7.1 Hz, 1H), 4.37 (dd, J = 7.4, 9.4 Hz, 1H), 5.20 (d, J = 9.4 Hz, 1H)
(2) N'-(tert-Butoxycarbonyl)-N,N-diethyl-L-isoleucinamide (429 mg, 1.5 mmol) and a 4 N solution of hydrochloric acid/ethyl acetate (6.0 ml) were allowed to react in ethyl acetate (15 ml) in the same manner as in Example 3 (2) to thereby give N,N-diethyl-L-isoleucinamide hydrochloride (335 mg).
   ¹H-NMR (DMSO-d₆, 400 MHz) δ (ppm);
      0.85 (t, J = 7.3 Hz, 3H), 0.94 (d, J = 6.9 Hz, 3H), 1.04 (t, J = 7.0 Hz, 3H), 1.12 (m, 1H), 1.13 (t, J = 7.0 Hz, 3H), 1.45 (m, 1H), 1.77 (m, 1H), 3.01-3.67 (m, 4H)
(3) N-[N-(Benzyloxycarbonyl)-L-valyl]-L-tyrosine (231 mg, 0.60 mmol), 1-hydroxybenzotriazole monohydrate (101 mg, 0.66 mmol), N,N'-dicyclohexylcarbodiimide (136 mg, 0.66 mmol), N,N-diethyl-L-isoleucinamide (134 mg, 0.60 mmol) and triethylamine (180 mg, 0.18 mmol) were allowed to react in tetrahydrofuran (15 ml) in the same manner as in Example 1 (3) to thereby give N'-[N-[N-(benzyloxycarbonyl)-L-valyl]-L-tyrosyl]-N,N-diethyl-L-isoleucinamide (250 mg).
   ¹H-NMR (DMSO-d₆, 400 MHz) δ (ppm);
      0.70-0.85 (m, 12H), 1.01 (t, J = 6.9 Hz, 3H), 1.09 (t, J = 7.1 Hz, 3H), 1.25 (m, 1H), 1.46 (m, 1H), 1.74 (m, 1H), 1.89 (m, 1H), 2.68 (dd, J = 8.2, 13.9 Hz, 1H), 2.79 (dd, J = 5.2, 13.9 Hz, 1H), 3.10-3.48 (m, 4H), 3.83 (dd, J = 7.2, 9.0 Hz, 1H), 4.47 (dd, J = 9.2, 9.2 Hz, 1H), 4.52 (m, 1H), 5.00 (d, J = 12.5 Hz, 1H), 5.05 (d, J = 12.5 Hz, 1H), 6.58 (d, J = 8.4 Hz, 2H), 6.95 (d, J = 8.4 Hz, 2H), 7.25 (d, J = 9.0 Hz, 1H), 7.28-7.41 (m, 5H), 7.84 (d, J = 8.2 Hz, 1H), 8.03 (d, J = 9.2 Hz, 1H), 9.11 (s, 1H)
(4) Using N'-[N-[N-(benzyloxycarbonyl)-L-valyl]-L-tyrosyl]-N,N-diethyl-L-isoleucinamide (150 mg, 0.26 mmol) and 10% palladium-carbon (40 mg), a reaction was performed in methanol (30 ml) in the same manner as in Example 1 (2) to thereby give the title compound (118 mg).
   ¹H-NMR (DMSO-d₆, 400 MHz) δ (ppm);
      0.76 (d, J = 7.1 Hz, 3H), 0.77 (t, J = 7.6 Hz, 3H), 0.88 (d, J = 6.9 Hz, 3H), 0.92 (d, J = 6.9 Hz, 3H), 1.01 (t, J = 7.0 Hz, 3H), 1.05 (m, 1H), 1.09 (t, J = 7.1 Hz, 3H), 1.46 (m, 1H), 1.77 (m, 1H), 2.08 (m, 1H), 2.70 (dd, J = 8.2, 14.0 Hz, 1H), 2.79 (dd, J = 5.7, 14.0 Hz, 1H), 3.16 (m, 1H), 3.22-3.47 (m, 3H), 3.62 (b, 1H), 4.47 (dd, J = 9.3, 9.3 Hz, 1H), 4.61 (m, 1H), 6.62 (d, J = 8.5 Hz, 2H), 7.01 (d, J = 8.5 Hz, 2H), 8.06 (bs, 3H), 8.29 (d, J = 9.3 Hz, 1H), 8.45 (d, J = 8.0 Hz, 1H), 9.20 (s, 1H)
   MASS (m/e):
      448 (M⁺)

### EXAMPLE 12

### Production of L-valyl-L-tyrosyl-N-[5-(methoxycarbonyl)-pentyl]-L-isoleucinamide hydrochloride

(1) 6-Aminohexanoic acid (1.3 g, 10 mmol) and p-toluenesulfonic acid (2.3 g, 12 mmol) were dissolved in benzene (10 ml). To the reaction mixture was added benzyl alcohol (5.0 ml) and the obtained mixture was heated under reflux for 5 hours while removing water therefrom. After cooling, ether (16 ml) and hexane (16 ml) were added thereto. The solid matter thus formed was filtered and washed with ether. The solid matter thus obtained was dried under reduced pressure to thereby give benzyl 6-aminohexanoate p-toluenesulfonate (3.8 g).
   ¹H-NMR (DMSO-d₆, 200 MHz) δ (ppm);
      1.30 (m, 2H), 1.53 (m, 4H), 2.27 (s, 3H), 2.37 (t, J = 7.3 Hz, 2H), 2.75 (m, 2H), 5.08 (s, 2H), 7.10 (d, J = 8.0 Hz, 2H), 7.26-7.43 (m, 5H), 7.49 (d, J = 8.0 Hz, 2H), 7.62 (br, 3H)
(2) Under an argon gas stream, N-(tert-butoxycarbonyl)-L-isoleucine (462 mg, 2.0 mmol) and 1-hydroxybenzotriazole monohydrate (337 mg, 2.2 mmol) were dissolved in tetrahydrofuran (10 ml) and chloroform (10 ml). Then a solution of 1-ethyl-3-(3-dimethylaminopropyl)-carbodiimide hydrochloride (422 mg, 2.2 mmol) in chloroform (10 ml) was added thereto. After stirring at room temperature for 1 hour, a suspension of benzyl 6-aminohexanoate p-toluenesulfonate (786 mg, 2.0 mmol) and triethylamine (404 mg, 0.55 mmol) in tetrahydrofuran (10 ml) was added to the reaction mixture and the obtained mixture was stirred for 5 hours. After the reaction, the solvent was distilled off under reduced pressure. The residue was extracted with chloroform and washed with 1 N hydrochloric acid, a saturated agueous solution of sodium hydrogencarbonate, water and a saturated aqueous solution of sodium chloride. After drying over anhydrous sodium sulfate, the solvent was distilled off under reduced pressure to thereby give N'-(tert-butoxycarbonyl)-N-(5-carboxypentyl)-L-isoleucinamide (68 mg).
   ¹H-NMR (CDCl₃, 200 MHz) δ (ppm);
      0.88 (t, J = 7.0 Hz, 3H), 0.91 (d, J = 6.6 Hz, 3H), 1.12 (m, 1H), 1.23-1.76 (m, 7H), 1.43 (s, 9H), 1.78 (m, 1H), 2.35 (t, J = 7.4 Hz, 2H), 3.18-3.31 (m, 2H), 3.85 (dd, J = 6.4, 8.8 Hz, 1H), 5.00 (m, 1H), 5.11 (s, 2H), 5.93 (m, 1H), 7.35 (s, 5H)
(3) N'-(tert-Butoxycarbonyl)-N-(5-carboxypentyl)-L-isoleucinamide (781 mg, 1.8 mmol) and a 4 N solution of hydrochloric acid/ethyl acetate (6.0 ml) were allowed to react in ethyl acetate (18 ml) in the same manner as in Example 3 (2) to thereby give N-(5-carboxypentyl)-L-isoleucinamide hydrochloride (650 mg).
   ¹H-NMR (CDCl₃, 200 MHz) δ (ppm);
      0.92 (t, J = 6.9 Hz, 3H), 1.02 (d, J = 6.5 Hz, 3H), 1.12-1.46 (m, 3H), 1.46-1.80 (m, 5H), 2.07 (m, 1H), 2.34 (t, J = 7.3 Hz, 2H), 3.13 (m, 1H), 3.31 (m, 1H), 4.15 (m, 1H), 5.09 (s, 2H), 7.33 (s, 5H), 8.22 (br, 4H)
(4) N-[N-(Benzyloxycarbonyl)-L-valyl]-L-tyrosine (231 mg, 0.60 mmol), 1-hydroxybenzotriazole monohydrate (102 mg, 0.66 mmol), N-(5-carboxypentyl)-L-isoleucinamide hydrochloride (223 mg, 0.60 mmol), 1-ethyl-3-(3-dimethylaminopropyl)carbodiimide hydrochloride (127 mg, 0.66 mmol) and triethylamine (182 mg, 1.8 mmol) were allowed to react in tetrahydrofuran (9.0 ml) in the same manner as in Example 12 (2) to thereby give N'-[N-[N-(benzyloxycarbonyl)-L-valyl]-L-tyrosyl]-N-(5-carboxypentyl)-L-isoleucinamide (120 mg).
   ¹H-NMR (DMSO-d₆, 400 MHz) δ (ppm);
      0.71 (d, J = 7.7 Hz, 3H), 0.74 (d, J = 6.8 Hz, 3H), 0.77 (d, J = 6.8 Hz, 3H), 0.78 (d, J = 6.7 Hz, 3H), 1.05 (m, 1H), 1.19-1.31 (m, 2H), 1.31-1.45 (m, 3H), 1.47-1.60 (m, 2H), 1.68 (m, 1H), 1.88 (m, 1H), 2.32 (t, J = 7.4 Hz, 2H), 2.68 (dd, J = 9.0, 13.9 Hz, 1H), 2.85 (dd, J = 4.8, 13.9 Hz, 1H), 2.90-3.10 (m, 2H), 3.82 (dd, J = 7.3, 8.9 Hz, 1H), 4.08 (dd, J = 7.9, 8.4 Hz, 1H), 4.51 (m, 1H), 4.98 (d, J = 12.5 Hz, 1H), 5.05 (d, J = 12.5 Hz, 1H), 5.06 (s, 2H), 6.61 (d, J = 8.4 Hz, 2H), 7.00 (d, J = 8.4 Hz, 2H), 7.23 (d, J = 8.9 Hz, 1H), 7.25-7.43 (m, 10H), 7.76 (m, 1H), 7.82 (d, J = 8.8 Hz, 1H), 7.98 (d, J = 7.9 Hz, 1H), 9.18 (s, 1H)
(5) Using N'-[N-[N-(benzyloxycarbonyl)-L-valyl]-L-tyrosyl]-N-(5-carboxypentyl)-L-isoleucinamide (120 mg, 0.16 mmol), 10% palladium-carbon (40 mg) and a 4 N solution of hydrochloric acid/ethyl acetate (6.0 ml), a reaction was performed in methanol (10 ml) and tetrahydrofuran (10 ml) in the same manner as in Example 3 (2) to thereby give the title compound (90 mg).
   ¹H-NMR (DMSO-d₆, 400 MHz) δ (ppm);
      0.77 (d, J = 6.8 Hz, 3H), 0.77 (t, J = 7.3 Hz, 3H), 0.86 (d, J = 6.9 Hz, 3H), 0.91 (d, J = 6.9 Hz, 3H), 1.07 (m, 1H), 1.19-1.31 (m, 2H), 1.31-1.58 (m, 5H), 1.70 (m, 1H), 2.11 (m, 1H), 2.26 (t, J = 7.4 Hz, 2H), 2.70 (dd, J = 8.7, 13.9 Hz, 1H), 2.86 (dd, J = 5.3, 13.9 Hz, 1H), 2.91-3.10 (m, 2H), 3.57 (s, 3H), 3.61 (m, 1H), 4.08 (dd, J = 8.3, 8.6 Hz, 1H), 4.59 (m, 1H), 6.65 (d, J = 8.5 Hz, 2H), 7.07 (d, J = 8.5 Hz, 2H), 7.81 (t, J = 5.7 Hz, 1H), 8.05-8.21 (m, 4H), 8.56 (d, J = 7.8 Hz, 1H), 9.28 (s, 1H)
   MASS (m/e):
      520 (M⁺)

### EXAMPLE 13

### Production of L-valyl-L-tyrosyl-N-(5-carboxypentyl)-L-isoleucinamide

L-Valyl-L-tyrosyl-N-[5-(methoxycarbonyl)pentyl]-L-isoleucinamide (45 mg, 0.081 mmol) was dissolved in methanol (1.5 ml) and then allowed to react by using a 2 N aqueous solution sodium hydroxide in the same manner as in Example 5 (2). After purification by thin layer chromatography, the title compound (26 mg) was obtained.
¹H-NMR (DMSO-d₆, 400 MHz) δ (ppm);
   0.67 (d, J = 6.8 Hz, 3H), 0.78 (d, J = 6.8 Hz, 3H), 0.79 (t, J = 7.4 Hz, 3H), 0.81 (d, J = 6.8 Hz, 3H), 1.03 (m, 1H), 1.18-1.31 (m, 2H), 1.31-1.56 (m, 5H), 1.67 (m, 1H), 1.89 (m, 1H), 2.07 (t, J = 7.2 Hz, 2H), 2.74 (dd, J = 7.6, 13.8 Hz, 1H), 2.82 (dd, J = 5.5, 13.8 Hz, 1H), 2.91-3.12 (m, 2H), 2.94 (d, J = 4.8 Hz, 1H), 3.40 (br, 4H), 4.08 (dd, J = 8.1, 8.7 Hz, 1H), 4.54 (m, 1H), 6.59 (d, J = 8.4 Hz, 2H), 6.92 (d, J = 8.4 Hz, 2H), 7.79 (t, J = 5.6 Hz, 1H), 8.02 (d, J = 8.7 Hz, 1H), 8.09 (d, J = 8.1 Hz, 1H)
MASS (m/e):
   507 (M⁺+1)

### EXAMPLE 14

### Production of L-valyl-L-tyrosyl-N-(2-phenylethyl)-L-valinamide hydrochloride

(1) N-(tert-Butyoxycarbonyl)-L-valine (1.3 g, 6.0 mmol), 1-hydroxybenzotriazole monohydrate (1.0 g, 6.6 mmol), N,N'-dicyclohexylcarbodiimide (1.4 g, 6.6 mmol) and 2-phenylethylamine (0.73 g, 6.0 mmol) were allowed to react in tetrahydrofuran (60 ml) in the same manner as in Example 3 (1) to thereby give N'-(tert-butoxycarbonyl)-N-(2-phenylethyl)-L-valinamide (1.9 g).
   ¹H-NMR (CDCl₃, 200 MHz) δ (ppm);
      0.77 (d, J = 6.6 Hz, 3H), 0.78 (d, J = 6.7 Hz, 3H), 1.39 (s, 9H), 1.86 (m, 1H), 2.71 (t, J = 7.3 Hz, 2H), 3.17-3.47 (m, 2H), 3.73 (dd, 1H), 6.57 (d, J = 9.0 Hz, 1H), 7.13-7.35 (m, 5H), 7.92 (t, J = 5.3 Hz, 1H)

   (2) N'-(tert-Butoxycarbonyl)-N-(2-phenylethyl)-L-valinamide (1.9 g, 5.8 mmol) and a 4 N solution of hydrochloric acid/ethyl acetate (15 ml) were allowed to react in ethyl acetate (40 ml) in the same manner as in Example 3 (2) to thereby give N-(2-phenylethyl)-L-valinamide hydrochloride (5.0 g).
      ¹H-NMR (DMSO-d₆, 200 MHz) δ (ppm);
         0.84 (d, J = 6.8 Hz, 3H), 0.85 (d, J = 6.8 Hz, 3H), 2.03 (m, 1H), 2.78 (t, J = 7.2 Hz, 2H), 3.17-3.63 (m, 3H), 7.15-7.40 (m, 5H), 8.26 (bs, 3H), 8.71 (t, J = 5.5 Hz, 1H)
   (3) N-[N-(Benzyloxycarbonyl)-L-valyl]-L-tyrosine (308 mg, 0.80 mmol), N-(2-phenylethyl)-L-valinamide hydrochloride (206 mg, 0.80 mmol), 1-hydroxybenzotriazole monohydrate (135 mg, 0.88 mmol), N,N'-dicyclohexylcarbodiimide (181 mg, 0.88 mmol) and triethylamine (242 mg, 2.4 mmol) were allowed to react in tetrahydrofuran (15 ml) in the same manner as in Example 1 (2) to thereby give N'-[N-[N-(benzyloxycarbonyl)-L-valyl]-L-tyrosyl]-N-(2-phenylethyl)-L-valinamide (419 mg).
      ¹H-NMR (DMSO-d₆, 400 MHz) δ (ppm);
         0.68-0.84 (m, 12H), 1.79-1.95 (m, 2H), 2.69 (m, 1H), 2.69 (t, J = 7.3 Hz, 2H), 2.86 (dd, J = 5.1, 13.8 Hz, 1H), 3.19-3.42 (m, 2H), 3.83 (dd, J = 7.5, 8.5 Hz, 1H), 4.06 (dd, J = 7.2, 8.3 Hz, 1H), 4.53 (m, 1H), 4.99 (d, J = 12.6 Hz, 1H), 5.05 (d, J = 12.6 Hz, 1H), 6.61 (d, J = 8.3 Hz, 2H), 7.02 (d, J = 8.3 Hz, 2H), 7.15-7.41 (m, 11H), 7.77 (d, J = 8.9 Hz, 1H), 7.82 (t, J = 5.4 Hz, 1H), 7.97 (d, J = 8.3 Hz, 1H), 9.14 (s, 1H)
   (4) Using N'-[N-[N-(benzyloxycarbonyl)-L-valyl]-L-tyrosyl]-N-(2-phenylethyl)-L-valinamide (168 mg, 0.27 mmol), 10% palladium-carbon (40 mg) and a 4 N solution of hydrochloric acid/ethyl acetate (0.20 ml, 0.80 mmol), a reaction was performed in methanol (45 ml) and tetrahydrofuran (45 ml) in the same manner as in Example 10 (4) to thereby give the title compound (120 mg).
      ¹H-NMR (DMSO-d₆, 400 MHz) δ (ppm);
         0.76 (d, J = 6.7 Hz, 3H), 0.76 (d, J = 6.7 Hz, 3H), 0.87 (d, J = 6.9 Hz, 3H), 0.92 (d, J = 6.9 Hz, 3H), 1.89 (m, 1H), 2.11 (m, 1H), 2.70 (t, J = 7.1 Hz, 2H), 2.71 (dd, 1H), 2.86 (dd, J = 5.2, 14.0 Hz, 1H), 3.19-3.36 (m, 2H), 3.60 (d, J = 5.0 Hz, 1H), 4.07 (dd, J = 7.2, 9.1 Hz, 1H), 4.61 (m, 1H), 6.66 (d, J = 8.5 Hz, 2H), 7.09 (d, J = 8.5 Hz, 2H), 7.15-7.31 (m, 5H), 7.86 (t, J = 5.6 Hz, 1H), 8.07 (bs, 3H), 8.11 (d, J = 9.1 Hz, 1H), 8.55 (d, J = 8.0 Hz, 1H), 9.26 (s, 1H)
      MASS (m/e):
         482 (M⁺)

### EXAMPLE 15

### Production of L-valyl-L-tyrosyl-N-(2-phenylethyl)-L-phenylalaninamide hydrochloride

(1) N-(Benzyloxycarbonyl)-L-phenylalanine (12 g, 10 mmol), 1-hydroxybenzotriazole monohydrate (1.7 g, 11 mmol), N,N'-dicyclohexylcarbodiimide (2.3 g, 11 mmol) and 2-phenylethylamine (1.2 g, 10 mmol) were allowed to react in tetrahydrofuran (100 ml) in the same manner as in Example 1 (3) to thereby give N'-(benzyloxycarbonyl)-N-(2-phenylethyl)-L-phenylalaninamide (3.5 g).
   ¹H-NMR (CDCl₃, 200 MHz) δ (ppm);
      2.68 (t, J = 7.1 Hz, 2H), 2.70 (dd, 1H), 2.90 (dd, J = 4.3, 13.6 Hz, 1H), 3.13-3.40 (m, 1H), 4.18 (m, 1H), 4.94 (s, 2H), 7.12-7.41 (m, 15H), 7.47 (d, J = 8.7 Hz, 1H), 8.06 (t, J = 5.1 Hz, 1H)
(2) Using N'-(benzyloxycarbonyl)-N-(2-phenylethyl)-L-phenylalaninamide (2.8 g, 7.0 mmol), 10% palladium-carbon (400 mg) and a 4 N solution of hydrochloric acid/ethyl acetate (3.5 ml, 14 mmol), a reaction was performed in methanol (70 ml) in the same manner as in Example 10 (4) to thereby give N-(2-phenylethyl)-L-phenylalaninamide hydrochloride (2.1 g).
   ¹H-NMR (DMSO-d₆, 200 MHz) δ (ppm);
      2.62 (t, J = 7.2 Hz, 2H), 3.02 (d, J = 6.8 Hz, 2H), 3.10-3.50 (m, 2H), 3.98 (m, 1H), 7.05-7.40 (m, 10H), 8.38 (bs, 3H), 8.75 (t, J = 5.3 Hz, 1H)
(3) N-[N-(Benzyloxycarbonyl)-L-valyl]-L-tyrosine (308 mg, 0.80 mmol), N-(2-phenylethyl)-L-phenylalaninamide hydrochloride (244 mg, 0.80 mmol), 1-hydroxybenzotriazole monohydrate (135 mg, 0.88 mmol), N,N'-dicyclohexylcarbodiimide (181 mg, 0.88 mmol) and triethylamine (242 mg, 2.4 mmol) were allowed to react in tetrahydrofuran (23 ml) in the same manner as in Example 1 (3) to thereby give N'-[N-[N-(benzyloxycarbonyl)-L-valyl]-L-tyrosyl]-N-(2-phenylethyl)-L-phenylalaninamide (432 mg).
   ¹H-NMR (DMSO-d₆, 400 MHz) δ (ppm);
      0.71 (d, J = 6.7 Hz, 3H), 0.73 (d, J = 6.7 Hz, 3H), 1.86 (m, 1H), 2.62 (t, J = 7.4 Hz, 2H), 2.64 (dd, 1H), 2.73-2.85 (m, 2H), 2.91 (dd, J = 5.6, 13.7 Hz, 1H), 3.13-3.31 (m, 2H), 3.81 (dd, J = 7.2, 8.7 Hz, 1H), 4.37-4.50 (m, 2H), 4.99 (d, J = 12.6 Hz, 1H), 5.06 (d, J = 12.6 Hz, 1H), 6.60 (d, J = 8.4 Hz, 2H), 6.98 (d, J = 8.4 Hz, 2H), 7.11-7.43 (m, 16H), 7.85 (t, J = 5.6 Hz, 1H), 7.88 (d, J = 8.3 Hz, 1H), 8.10 (d, J = 8.2 Hz, 1H), 9.17 (s, 1H)
(4) Using N'-[N-[N-(benzyloxycarbonyl)-L-valyl]-L-tyrosyl]-N-(2-phenylethyl)-L-phenylalaninamide (214 mg, 0.32 mmol), 10% palladium-carbon (40 mg) and a 4 N solution of hydrochloric acid/ethyl acetate (0.20 ml, 0.80 mmol), a reaction was performed in methanol (40 ml) and tetrahydrofuran (40 ml) in the same manner as in Example 10 (4) to thereby give the title compound (75 mg).
   ¹H-NMR (DMSO-d₆, 400 MHz) δ (ppm);
      0.82 (d, J = 6.9 Hz, 3H), 0.86 (d, J = 6.9 Hz, 3H), 2.40 (m, 1H), 2.63 (t, J = 7.5 Hz, 2H), 2.68 (dd, J = 8.7, 14.1 Hz, 1H), 2.78 (dd, J = 8.7, 13.9 Hz, 1H), 2.85 (dd, J = 5.0, 14.1 Hz, 1H), 2.90 (dd, J = 5.5, 13.9 Hz, 1H), 3.17-3.30 (m, 2H), 3.58 (d, J = 5.2 Hz, 1H), 4.44 (m, 1H), 4.53 (m, 1H), 6.65 (d, J = 8.5 Hz, 2H), 7.04 (d, J = 8.5 Hz, 2H), 7.13-7.33 (m, 10H), 7.86 (t, J = 5.7 Hz, 1H), 8.02 (br, 3H), 8.33 (d, J = 8.3 Hz, 1H), 8.46 (d, J = 8.2 Hz, 1H), 9.24 (s, 1H)
   MASS (m/e):
      530 (M⁺)

### EXAMPLE 16

### Production of L-2-aminobutanoyl-L-tyrosyl-N-(2-phenylethyl)-L-leucinamide hydrochloride

(1) N-(Benzyloxycarbonyl)-L-tyrosine (2.5 g, 8.0 mmol), N-(2-phenylethyl)-L-leucinamide (1.9 g, 8.0 mmol), 1-hydroxybenzotriazole monohydrate (1.4 g, 8.8 mmol) and N,N'-dicyclohexylcarbodiimide (1.8 g, 8.0 mmol) were allowed to react in tetrahydrofuran (80 ml) in the same manner as in Example 1 (3) to thereby give N'-[N-(benzyloxycarbonyl)-L-tyrosyl]-N-(2-phenylethyl)-L-leucinamide (3.0 g).
   ¹H-NMR (DMSO-d₆, 400 MHz) δ (ppm);
      0.78 (d, J = 6.4 Hz, 3H), 0.82 (t, J = 7.7 Hz, 3H), 0.83 (d, J = 6.6 Hz, 3H), 1.05-1.55 (m, 9H), 1.36 (s, 9H), 2.68 (t, J = 7.4 Hz, 2H), 2.70 (dd, 1H), 2.85 (dd, J = 5.3, 14.0 Hz, 1H), 3.24 (m, 1H), 3.80 (m, 1H), 4.21 (m, 1H), 4.46 (m, 1H), 6.60 (d, J = 8.4 Hz, 2H), 6.90 (d, J = 8.0 Hz, 1H), 6.97 (d, J = 8.4 Hz, 2H), 7.15-7.24 (m, 3H), 7.24-7.32 (m, 2H), 7.70 (t, J = 5.8 Hz, 1H), 7.72 (d, J = 8.0 Hz, 1H), 7.96 (d, J = 8.3 Hz, 1H), 9.15 (s, 1H)
(2) Using N'-[N-(benzyloxycarbonyl)-L-tyrosyl]-N-(2-phenylethyl)-L-leucinamide (2.0 g, 3.8 mmol) and 10% palladium-carbon (150 mg), a reaction was performed in methanol (45 ml) and tetrahydrofuran (1.9 ml, 7.6 mmol) in the same manner as in Example 10 (4) to thereby give L-tyrosyl-N-(2-phenylethyl)-L-leucinamide hydrochloride (1.6 g).
   ¹H-NMR (DMSO-d₆, 400 MHz) δ (ppm);
      0.82 (d, J = 6.5 Hz, 3H), 0.86 (d, J = 6.5 Hz, 3H), 1.33-1.59 (m, 3H), 2.72 (t, J = 7.3 Hz, 2H), 2.85 (dd, J = 7.3, 14.1 Hz, 1H), 3.00 (dd, J = 5.6, 14.1 Hz, 1H), 3.20-3.36 (m, 2H), 3.97 (dd, J = 5.6, 7.3 Hz, 1H), 4.26 (m, 1H), 6.69 (d, J = 8.5 Hz, 2H), 7.05 (d, J = 8.5 Hz, 2H), 7.14-7.32 (m, 5H), 8.07 (d, J = 5.6 Hz, 1H), 8.15 (bs, 3H), 8.70 (d, J = 8.3 Hz, 1H), 9.40 (s, 1H)
(3) N-(tert-Butoxycarbonyl)-L-2-aminobutanoic acid (121 mg, 0.60 mmol), L-tyrosyl-N-(2-phenylethyl)-L-leucinamide hydrochloride (302 mg, 0.70 mmol), 1-hydroxybenzotriazole monohydrate (101 mg, 0.66 mmol), N,N'-dicyclohexylcarbodiimide (136 mg, 0.66 mmol) and triethylamine (121 mg, 1.2 mmol) were allowed to react in tetrahydrofuran (11 ml) in the same manner as in Example 3 (1) to thereby give N'-[N-[N-(tert-butoxycarbonyl)-L-2-aminobutanoyl]-L-tyrosyl]-N-(2-phenylethyl)-L-leucinamide (302 mg).
   ¹H-NMR (DMSO-d₆, 400 MHz) δ (ppm);
      0.76 (t, J = 7.4 Hz, 3H), 0.78 (d, J = 6.4 Hz, 3H), 0.83 (d, J = 6.4 Hz, 3H), 1.22-1.57 (m, 5H), 2.68 (t, J = 7.3 Hz, 2H), 2.70 (dd, 1H), 2.85 (dd, J = 5.3, 13.9 Hz, 1H), 3.18-3.31 (m, 2H), 3.76 (m, 1H), 4.21 (m, 1H), 4.46 (m, 1H), 6.61 (d, J = 8.4 Hz, 2H), 6.88 (d, J = 7.9 Hz, 1H), 6.97 (d, J = 8.4 Hz, 2H), 7.14-7.32 (m, 5H), 7.69 (t, J = 5.7 Hz, 1H), 7.72 (d, J = 8.1 Hz, 1H), 7.97 (d, J = 8.2 Hz, 1H), 9.15 (s, 1H)
(4) N'-[N-[N-(tert-Butoxycarbonyl)-L-2-aminobutanoyl]-L-tyrosyl]-N-(2-phenylethyl)-L-leucinamide (204 mg, 0.35 mmol) and a 4 N solution of hydrochloric acid/ethyl acetate (1.8 ml) were allowed to react in methanol (0.50 ml), ethyl acetate (4.0 ml) and chloroform (4.0 ml) in the same manner as in Example 2 (4) to thereby give the title compound (100 mg).
   ¹H-NMR (DMSO-d₆, 400 MHz) δ (ppm);
      0.78 (d, J = 6.5 Hz, 3H), 0.83 (d, J = 7.3 Hz, 3H), 0.85 (t, J = 6.3 Hz, 3H), 1.29-1.57 (m, 3H), 1.64-1.80 (m, 2H), 2.68 (t, J = 7.4 Hz, 2H), 2.69 (dd, 1H), 2.89 (dd, J = 5.1, 13.9 Hz, 1H), 3.17-3.30 (m, 2H), 3.67 (t, J = 5.9 Hz, 1H), 4.23 (m, 1H), 4.54 (m, 1H), 6.66 (d, J = 8.5 Hz, 2H), 7.07 (d, J = 8.5 Hz, 2H), 7.15-7.32 (m, 5H), 7.72 (t, J = 5.6 Hz, 1H), 7.97 (bs, 3H), 8.19 (d, J = 8.4 Hz, 1H), 8.58 (d, J = 8.2 Hz, 1H), 9.26 (s, 1H)
   MASS (m/e):
      482 (M⁺)

### EXAMPLE 17

### Production of L-ornithyl-L-tyrosyl-N-(2-phenylethyl)-L-leucinamide dihydrochloride

(1) To a solution of N,N'-bis(benzyloxycarbonyl)-L-ornithine-N-hydroxysuccinimide ester (279 mg, 0.60 mmol) in tetrahydrofuran (6.0 ml) was added under an argon gas stream a suspension of L-tyrosyl-N-(2-phenylethyl)-L-leucinamide (302 mg, 0.70 mmol) and triethylamine (121 mg, 1.2 mmol) in tetrahydrofuran (3.0 ml) at room temperature and the obtained mixture was stirred for 4 hours. After the reaction, it was extracted with methanol/chloroform, washed successively with 1 N hydrochloric acid, a saturated aqueous solution of sodium hydrogencarbonate water and an aqueous solution of sodium chloride and dried over anhydrous sodium sulfate. After distilling off the solvent under reduced pressure, the residue was purified by column chromatography to thereby give N'-[N-[N,N'-bis(benzyloxycarbonyl)-L-ornithyl]-L-tyrosyl]-N-(2-phenylethyl)-L-leucinamide (290 mg).
   ¹H-NMR (DMSO-d₆, 400 MHz) δ (ppm);
      0.78 (t, J = 6.4 Hz, 3H), 0.83 (d, J = 6.4 Hz, 3H), 1.28-1.62 (m, 7H), 2.67 (t, J = 7.3 Hz, 2H), 2.71 (dd, J = 8.4, 14.2 Hz, 1H), 2.86 (dd, J = 5.4, 14.2 Hz, 1H), 2.90-3.02 (m, 2H), 3.19-3.30 (m, 2H), 3.95 (m, 1H), 4.21 (m, 1H), 4.44 (m, 1H), 4.98 (d, J = 12.7 Hz, 1H), 5.00 (s, 2H), 5.03 (d, J = 12.7 Hz, 1H), 6.62 (d, J = 8.4 Hz, 2H), 6.99 (d, J = 8.4 Hz, 2H), 7.15-7.39 (m, 16H), 7.41 (d, J = 8.0 Hz, 1H), 7.69 (t, J = 5.6 Hz, 1H), 7.90 (d, J = 8.1 Hz, 2H), 9.17 (s, 1H)
(2) Using N'-[N-[N,N'-bis(benzyloxycarbonyl)-L-ornithyl]-L-tyrosyl]-N-(2-phenylethyl)-L-leucinamide (200 mg, 0.26 mmol), 10% palladium-carbon (40 mg) and a 4 N solution of hydrochloric acid/ethyl acetate (0.13 ml, 0.52 mmol), a reaction was performed in methanol (5.0 ml) in the same manner as in Example 10 (4) to thereby give the title compound (150 mg).
   ¹H-NMR (DMSO-d₆, 400 MHz) δ (ppm);
      0.80 (d, J = 6.5 Hz, 3H), 0.85 (d, J = 6.5 Hz, 3H), 1.30-1.58 (m, 3H), 1.60-1.90 (m, 4H), 2.69 (t, J = 7.4 Hz, 2H), 2.75 (dd, J = 9.3, 14.0 Hz, 1H), 2.77-2.89 (m, 2H), 2.91 (dd, J = 4.7, 14.0 Hz, 1H), 3.16-3.35 (m, 2H), 3.84 (m, 1H), 4.24 (m, 1H), 4.47 (m, 1H), 6.67 (d, J = 8.5 Hz, 2H), 7.10 (d, J = 8.5 Hz, 2H), 7.15-7.32 (m, 5H), 7.95 (t, J = 5.6 Hz, 1H), 8.08 (bs, 3H), 8.18 (d, J = 8.3 Hz, 1H), 8.29 (bs, 3H), 8.88 (d, J = 8.1 Hz, 1H), 9.28 (br, 1H)
   MASS (m/e):
      511 (M⁺)

### EXAMPLE 18

### Production of N'-[N-[L-2-amino-3-(ethylthio)propionyl]-L-tyrosyl]-N-(2-phenylethyl)-L-leucinamide hydrochloride

(1) L-2-(tert-Butoxycarbonylamino)-3-(ethylthio)propionic acid (158 mg, 0.60 mmol), L-tyrosyl-N-(2-phenylethyl)-L-leucinamide hydrochloride (302 mg, 0.70 mmol), 1-hydroxybenzotriazole monohydrate (101 mg, 0.66 mmol), N,N'-dicyclohexylcarbodiimide (136 mg, 0.66 mmol) and triethylamine (121 mg, 1.2 mmol) were allowed to react in tetrahydrofuran (11 ml) in the same manner as in Example 3 (1) to thereby give N'-[N-[L-2-(tert-butoxycarbonylamino)-3-(ethylthio)propionyl]-L-tyrosyl]-N-(2-phenylethyl)-L-leucinamide (385 mg).
   ¹H-NMR (DMSO-d₆, 400 MHz) δ (ppm);
      0.79 (d, J = 6.4 Hz, 3H), 0.84 (d, J = 6.4 Hz, 3H), 1.15 (t, J = 7.4 Hz, 3H), 1.21-1.29 (m, 2H), 1.36 (s, 9H), 1.46 (m, 1H), 1.64-1.80 (m, 2H), 2.32-2.48 (m, 2H), 2.46 (q, J = 7.4 Hz, 2H), 2.68 (t, J = 7.3 Hz, 2H), 2.70 (dd, 1H), 2.85 (dd, J = 5.2, 13.9 Hz, 1H), 3.25 (m, 1H), 3.94 (m, 1H), 4.21 (m, 1H), 4.46 (m, 1H), 6.61 (d, J = 8.4 Hz, 2H), 6.97 (d, J = 8.4 Hz, 2H), 7.05 (d, J = 7.7 Hz, 1H), 7.15-7.24 (m, 3H), 7.24-7.31 (m, 2H), 7.71 (t, J = 5.6 Hz, 1H), 7.73 (d, J = 7.9 Hz, 1H), 8.01 (d, J = 8.2 Hz, 1H), 9.16 (s, 1H)
(2) N'-[N-[L-2-(tert-Butoxycarbonylamino)-3-(ethylthio)propionyl]-L-tyrosyl]-N-(2-phenylethyl)-L-leucinamide (257 mg, 0.40 mmol) and a 4 N solution of hydrochloric acid/ethyl acetate (2.0 ml) were allowed to react in ethyl acetate (4.0 ml) in the same manner as in Example 2 (4) to thereby give the title compound (105 mg).
   ¹H-NMR (DMSO-d₆, 400 MHz) δ (ppm);
      0.79 (d, J = 6.4 Hz, 3H), 0.84 (d, J = 6.4 Hz, 3H), 1.17 (t, J = 7.3 Hz, 3H), 1.28-1.58 (m, 3H), 1.84-2.06 (m, 2H), 2.66 (dd, 1H), 2.68 (t, J = 6.9 Hz, 2H), 2.90 (dd, J = 4.6, 13.9 Hz, 1H), 3.16-3.32 (m, 2H), 3.79 (b, 1H), 4.25 (m, 1H), 4.53 (m, 1H), 6.66 (d, J = 8.4 Hz, 2H), 7.09 (d, J = 8.4 Hz, 2H), 7.14-7.33 (m, 5H), 7.78 (t, J = 5.1 Hz, 1H), 8.21 (br, 3H), 8.24 (d, J = 8.5 Hz, 1H), 8.63 (d, J = 7.9 Hz, 1H), 9.27 (s, 1H)
   MASS (m/e):
      542 (M⁺)

### EXAMPLE 19

### Production of L-norleucyl-L-tyrosyl-N-(2-phenylethyl)-L-leucinamide hydrochloride

(1) N-(tert-Butoxycarbonyl)-L-norleucine (139 mg, 0.60 mmol), L-tyrosyl-N-(2-phenylethyl)-L-leucinamide hydrochloride (302 mg, 0.70 mmol), 1-hydroxybenzotriazole monohydrate (101 mg, 0.66 mmol), N,N'-dicyclohexylcarbodiimide (136 mg, 0.66 mmol) and triethylamine (121 mg, 1.2 mmol) were allowed to react in tetrahydrofuran (16 ml) in the same manner as in Example 3 (1) to thereby give N'-[N-[N-(tert-butoxycarbonyl)-L-norleucyl]-L-tyrosyl]-N-(2-phenylethyl)-L-leucinamide (300 mg).
   ¹H-NMR (DMSO-d₆, 400 MHz) δ (ppm);
      0.78 (d, J = 6.4 Hz, 3H), 0.82 (t, J = 7.7 Hz, 3H), 0.83 (d, J = 6.6 Hz, 3H), 1.05-1.55 (m, 9H), 1.36 (s, 9H), 2.68 (t, J = 7.4 Hz, 2H), 2.70 (dd, 1H), 2.85 (dd, J = 5.3, 14.0 Hz, 1H), 3.24 (m, 1H), 3.80 (m, 1H), 4.21 (m, 1H), 4.46 (m, 1H), 6.60 (d, J = 8.4 Hz, 2H), 6.90 (d, J = 8.0 Hz, 1H), 6.97 (d, J = 8.4 Hz, 2H), 7.15-7.24 (m, 3H), 7.24-7.32 (m, 2H), 7.70 (t, J = 5.8 Hz, 1H), 7.72 (d, J = 8.0 Hz, 1H), 7.96 (d, J = 8.3 Hz, 1H), 9.15 (s, 1H)
(2) N'-[N-[N-(tert-Butoxycarbonyl)-L-norleucyl]-L-tyrosyl]-N-(2-phenylethyl)-L-leucinamide (200 mg, 0.33 mmol), a 4 N solution of hydrochloric acid/ethyl acetate (4.0 ml) were allowed to react in methanol (0.50 ml) and ethyl acetate (4.0 ml) in the same manner as in Example 3 (2) to thereby give the title compound (130 mg).
   ¹H-NMR (DMSO-d₆, 400 MHz) δ (ppm);
      0.79 (d, J = 6.6 Hz, 3H), 0.84 (d, J = 6.4 Hz, 3H), 0.85 (t, J = 6.2 Hz, 3H), 1.16-1.30 (m, 4H), 1.30-1.44 (m, 2H), 1.49 (m, 1H), 1.59-1.75 (m, 2H), 2.68 (t, J = 7.3 Hz, 2H), 2.68 (dd, 1H), 2.88 (dd, J = 5.3, 13.9 Hz, 1H), 3.17-3.30 (m, 2H), 3.71 (t, J = 5.9 Hz, 1H), 4.24 (m, 1H), 4.55 (m, 1H), 6.66 (d, J = 8.5 Hz, 2H), 7.07 (d, J = 8.5 Hz, 2H), 7.15-7.32 (m, 5H), 7.71 (t, J = 5.6 Hz, 1H), 8.09 (bs, 3H), 8.18 (d, J = 8.5 Hz, 1H), 8.62 (d, J = 8.3 Hz, 1H), 9.27 (s, 1H)
   MASS (m/e):
      510 (M⁺)

### EXAMPLE 20

### Production of L-valyl-L-phenylalanyl-N-benzyl-L-isoleucinamide hydrochloride

(1) To a solution of N-benzyl-L-isoleucinamide (660 mg, 3.0 ml) in tetrahydrofuran (20 ml) was added N-(tert-butoxycarbonyl)-L-phenylalanine-N-hydroxysuccinimide ester (1.09 g, 3.0 mmol) and the obtained mixture was stirred at room temperature for 4 hours. Then the reaction mixture was poured into water, extracted with ethyl acetate, washed with water, dilute hydrochloric acid, a saturated aqueous solution of sodium hydrogencarbonate and an aqueous solution of sodium chloride and dried. After distilling off the solvent under reduced pressure, the obtained residue was recrystallized from isopropyl ether/tetrahydrofuran to thereby give N'-[N-(tert-butoxycarbonyl)-L-phenylalanyl]-N-benzyl-L-isoleucinamide (1.07 g).
(2) A solution of N'-[N-(tert-butoxycarbonyl)-L-phenylalanyl]-N-benzyl-L-isoleucinamide (934 mg, 2.0 mmol) in a 4 N solution of hydrochloric acid/ethyl acetate (5 ml) was stirred at room temperature for 4 hours. Then the reaction mixture was diluted with ethyl acetate (20 ml). The crystals thus obtained were collected by filtration and dried under reduced pressure to thereby give L-phenylalanyl-N-benzyl-L-isoleucinamide hydrochloride (0.80 g).
   ¹H-NMR (DMSO-d₆, 200 MHz) δ (ppm);
      0.83 (t, 3H, J = 7 Hz, 3H), 0.84 (d, J = 7 Hz, 3H), 0.98-1.20 (m, 1H), 1.40-1.58 (m, 1H), 1.65-1.82 (m, 1H), 2.96 (dd, J = 7.18 Hz, 1H), 3.11 (dd, J = 6.18 Hz, 1H), 4.14 (t, J = 6 Hz, 1H), 4.23 (t, J = 8 Hz, 1H), 4.29 (d, J = 6 Hz, 2H), 7.18-7.36 (m, 10H), 8.22 (bs, 3H), 8.62 (t, J = 6 Hz, 1H), 8.69 (d, J = 8 Hz, 1H)
(3) To a solution of L-phenylalanyl-N-benzyl-L-isoleucinamide hydrochloride (0.70 g, 1.7 mmol) and triethylamine (0.20 g, 2.0 mmol) in tetrahydrofuran (50 ml) was added N-(tert-butoxycarbonyl) valine-N-hydroxysuccinimide ester (0.54 g, 1.7 mmol) and the obtained mixture was stirred at room temperature for 2 hours. After allowing it to stand overnight, the solvent was distilled off under reduced pressure. Then water (30 ml) was added to the residue and the mixture was stirred for 30 minutes. The crystals thus precipitated were collected by filtration, washed with methanol and dried under reduced pressure to thereby give N'-[N-[N-(tert-butoxycarbonyl)-L-valyl]-L-phenylalanyl]-N-benzyl-L-isoleucinamide (0.80 g).
(4) A solution of N'-[N-[N-(tert-butoxycarbonyl)-L-valyl]-L-phenylalanyl]-N-benzyl-L-isoleucinamide (0.60 g, 1.1 mmol) in a 4 N solution of hydrochloric acid/ethyl acetate (10 ml) was stirred at room temperature for 1 hour. Then the reaction mixture was diluted with ethyl acetate (20 ml) and the crystals thus obtained were collected by filtration and dried under reduced pressure to thereby give the title compound (0.48 g).
   ¹H-NMR (DMSO-d₆, 200 MHz) δ (ppm);
      0.78 (t, J = 6 Hz, 3H), 0.79 (d, J = 6 Hz, 3H), 0.87 (d, J = 7 Hz, 3H), 0.92 (d, J = 7 Hz, 3H), 1.31-1.48 (m, 1H), 1.65-1.85 (m, 1H), 2.02-2.18 (m, 1H), 2.81 (dd, J = 9.13 Hz, 1H), 2.99 (dd, J = 5.13 Hz, 1H), 3.60 (d, J = 5 Hz, 1H), 4.14-4.30 (m, 3H), 4.63-4.77 (m, 1H), 7.15-7.36 (m, 10H), 8.07 (bs, 3H), 8.28 (d, J = 9 Hz, 1H), 8.44 (t, J = 5 Hz, 1H), 8.61 (d, J = 8 Hz, 1H)
   MASS (m/e):
      467 (M⁺+1)

### EXAMPLE 21

### Production of L-prolyl-L-tyrosyl-N-benzyl-L-isoleucinamide hydrochloride

(1) From N-benzyl-L-isoleucinamide and N-(tert-butoxycarbonyl)tyrosine-N-hydroxysuccinimide ester, L-tyrosyl-N-benzyl-L-isoleucinamide hydrochloride was obtained in the same manner as in Example 20 (1) and (2).
(2) To a solution of L-tyrosyl-N-benzyl-L-isoleucinamide hydrochloride (420 mg, 1.0 mmol) and triethylamine (110 mg, 1.1 mmol) in tetrahydrofuran (20 ml) was added N-(tert-butoxycarbonyl)proline-N-hydroxysuccinimide ester (320 mg, 1.0 mmol) and the obtained mixture was stirred at room temperature for 4 hours. After allowing to stand overnight, the reaction mixture was poured into water and extracted with ethyl acetate. The organic layer was washed with 10% hydrochloric acid and water, and dried. After distilling off the solvent under reduced pressure, the residue was purified by silica gel column chromatography [eluent: methanol/chloroform (1 : 20)] to thereby give N'-[N-[N-(tert-butoxycarbonyl)-L-prolyl]-L-tyrosyl]-N-benzyl-L-isoleucinamide (570 mg).
(3) A solution of N'-[N-[N-(tert-butoxycarbonyl)-L-prolyl]-L-tyrosyl]-N-benzyl-L-isoleucinamide (300 mg, 0.52 mmol) in a 4 N solution of hydrochloric acid/ethyl acetate (6 ml) was stirred at room temperature for 2 hours. Then the reaction mixture was diluted with ethyl acetate (20 ml) and the crystals thus obtained were collected by filtration and dried under reduced pressure to thereby give the title compound (260 mg).
   ¹H-NMR (DMSO-d₆, 300 MHz) δ (ppm);
      0.76-0.86 (m, 1H), 1.00-1.17 (m, 1H), 1.35-1.50 (m, 1H), 1.70-1.90 (m, 4H), 2.20-2.36 (m, 1H), 2.67 (dd, J = 9.15 Hz, 1H), 2.93 (dd, J = 5.15 Hz, 1H), 3.07-3.23 (m, 2H), 4.06-4.30 (m, 4H), 4.53-4.62 (m, 1H), 6.65 (d, J = 8 Hz, 2H), 7.07 (d, J = 8 Hz, 2H), 7.20-7.35 (m, 5H), 8.12 (d, J = 8 Hz, 1H), 8.30-8.67 (m, 1H), 8.48 (t, J = 6 Hz, 1H), 8.74 (d, J = 8 Hz, 1H), 9.25 (s, 1H), 9.40-9.70 (m, 1H)
   MASS (m/e):
      481 (M⁺+1)

### EXAMPLE 22

### Production of β-alanyl-l-tyrosyl-N-benzyl-L-isoleucinamide hydrochloride

(1) To a solution of L-tyrosyl-N-benzyl-L-isoleucinamide hydrochloride (420 mg, 1.0 mmol) and triethylamine (140 mg, 1.4 mmol) in acetone (20 ml) was added N-(tert-butoxycarbonyl) β-alanine-N-hydroxysuccinimide ester (290 mg, 1.0 mmol) and the obtained mixture was stirred at room temperature for 4 hours. After allowing to stand overnight, the reaction mixture was poured into water and extracted with ethyl acetate. The organic layer was washed with 10% hydrochloric acid and water and dried. After distilling off the solvent under reduced pressure, the residue was purified by silica gel column chromatography [eluent: methanol/chloroform (1 : 20)] to thereby give N'-[N-[N-(tert-butoxycarbonyl)-β-alanyl]-L-tyrosyl]-N-benzyl-L-isoleucinamide (394 mg).
(2) A solution of N'-[N-[N-(tert-butoxycarbonyl)-β-alanyl]-L-tyrosyl]-N-benzyl-L-isoleucinamide (286 mg, 0.52 mmol) in a 4 N solution of hydrochloric acid/ethyl acetate (6 ml) was stirred at room temperature for 4 hours. Then the reaction mixture was diluted with ethyl acetate (20 ml) and the crystals thus obtained were collected by filtration and dried under reduced pressure to thereby give the title compound (240 mg).
   ¹H-NMR (DMSO-d₆, 300 MHz) δ (ppm);
      0.77-0.86 (m, 6H), 1.00-1.15 (m, 1H), 1.37-1.50 (m, 1H), 1.67-1.82 (m, 1H), 2.34-2.68 (m, 3H), 2.81-2.93 (m, 3H), 4.18 (t, J = 8 Hz, 1H), 4.28 (d, J = 6 Hz, 2H), 4.47-4.57 (m, 1H), 6.64 (d, J = 8 Hz, 2H), 7.04 (d, J = 8 Hz, 2H), 7.20-7.34 (m, 5H), 7.86 (bs, 3H), 8.06 (d, J = 9 Hz, 1H), 8.34 (d, J = 8 Hz, 1H), 8.49 (t, J = 6 Hz, 1H)
   MASS (m/e):
      455 (M⁺+1)

### EXAMPLE 23

### Production of N'-[L-valyl-4-methoxy-L-phenylalanyl]-N-benzylisoleucinamide hydrochloride

(1) To a solution of N'-[N-(tert-butoxycarbonyl)-L-tyrosyl]-N-benzyl-L-isoleucinamide (1.00 g, 2.1 mmol) in acetone (50 ml) were added dimethyl sulfate (0.30 g, 2.4 mmol) and potassium carbonate (0.57 g, 4.1 mmol) and the resulting mixture was stirred at room temperature for 3 hours. Then the reaction mixture was poured into water, extracted with ethyl acetate, washed with water and dried. After distilling off the solvent, the residue was purified by silica gel column chromatography [eluent: chloroform] to thereby give N'-[N-(tert-butoxycarbonyl)-4-methoxy-L-phenylalanyl]-N-benzyl-L-isoleucinamide (0.82 g).
(2) A solution of N'-[N-(tert-butoxycarbonyl)-4-methoxy-L-phenylalanyl]-N-benzyl-L-isoleucinamide (0.70 g, 1.4 mmol) in a 4 N solution of hydrochloric acid/ethyl acetate (6 ml) was stirred at room temperature for 1 hour. Then the reaction mixture was diluted with ethyl acetate (20 ml) and the crystals thus obtained were collected by filtration and dried under reduced pressure to thereby give N'-(4-methoxy-L-phenylalanyl)-N-benzyl-L-isoleucinamide hydrochloride (0.60 g).
(3) To a solution of N'-(4-methoxy-L-phenylalanyl)-N-benzyl-L-isoleucinamide hydrochloride (434 mg, 1.0 mmol) and triethylamine (110 mg, 1.1 mmol) in tetrahydrofuran (20 ml) and N,N-dimethylformamide (20 ml) was added N-(tert-butoxycarbonyl)-L-valine-N-hydroxysuccinimide ester (314 mg, 1.0 mmol) and the obtained mixture was stirred at room temperature for 3 hours. The reaction mixture was poured into water and extracted with ethyl acetate. The organic layer was washed with 10% hydrochloric acid and water and dried. After distilling off the solvent, the obtained crude crystals were washed with methanol and dried to thereby give N'-[N-[N-(tert-butoxycarbonyl)-L-valyl]-4-methoxy-L-phenylalanyl]-N-benzyl-L-isoleucinamide (400 mg).
(4) A solution of N'-[N-[N-(tert-butoxycarbonyl)-L-valyl]-4-methoxy-L-phenylalanyl]-N-benzyl-L-isoleucinamide (308 mg, 0.52 mmol) in a 4 N solution of hydrochloric acid/ethyl acetate (4 ml) was stirred at room temperature for 2 hours. Then the reaction mixture was diluted with ethyl acetate (20 ml) and the crystals thus obtained were collected by filtration and dried under reduced pressure to thereby give the title compound (250 mg).
   ¹H-NMR (DMSO-d₆, 300 MHz) δ (ppm);
      0.79 (t, J = 7 Hz, 3H), 0.81 (d, J = 7 Hz, 3H), 0.88 (d, J = 7 Hz, 3H), 0.93 (d, J = 7 Hz, 3H), 1.00-1.17 (m, 1H), 1.35-1.50 (m, 1H), 1.68-1.81 (m, 1H), 2.04-2.17 (m, 1H), 2.74 (dd, J = 9.15 Hz, 1H), 2.92 (dd, J = 6.15 Hz, 1H), 3.58 (d, J = 5 Hz, 1H), 3.70 (s, 3H), 4.16-4.36 (m, 3H), 4.62-4.70 (m, 1H), 6.80 (d, J = 8 Hz, 2H), 7.17-7.33 (m, 7H), 7.96 (bs, 3H), 8.25 (d, J = 8 Hz, 1H), 8.42 (t, J = 5 Hz, 1H), 8.53 (d, J = 8 Hz, 1H)
   MASS (m/e):
      497 (M⁺+1)

### EXAMPLE 24

### Production of L-norvalyl-L-tyrosyl-N-benzyl-L-isoleucinamide hydrochloride

(1) To a solution of L-tyrosyl-N-benzyl-L-isoleucinamide hydrochloride (420 mg, 1.0 mmol) and triethylamine (150 mg, 1.5 mmol) in N,N-dimethylformamide (20 ml) was added N-(tert-butoxycarbonyl)-L-norvaline-N-hydroxysuccinimide ester (315 mg, 1.0 mmol) and the obtained mixture was stirred at room temperature for 5 hours. The reaction mixture was poured into water and extracted with ethyl acetate. The organic layer was washed with 10% hydrochloric acid and water, and dried. After distilling off the solvent, the obtained residue was purified by silica gel column chromatography [eluent: methanol/chloroform (1 : 20)] to thereby give N'-[N-[N-(tert-butoxycarbonyl)-L-norvalyl]-N-tyrosyl]-N-benzyl-L-isoleucinamide (492 mg).
(2) A solution of N'-[N-[N-(tert-butoxycarbonyl)-L-norvalyl]-N-tyrosyl]-N-benzyl-L-isoleucinamide (390 mg, 0.67 mmol) in a 4 N solution of hydrochloric acid/ethyl acetate (6 ml) was stirred at room temperature for 2 hours. Then the reaction mixture was diluted with ethyl acetate (20 ml) and the crystals thus obtained were collected by filtration and dried under reduced pressure to thereby give the title compound (320 mg).
   ¹H-NMR (DMSO-d₆, 200 MHz) δ (ppm);
      0.74-0.87 (m, 9H), 0.96-1.84 (m, 7H), 2.69 (dd, J = 9.14 Hz, 1H), 2.89 (dd, J = 5.14 Hz, 1H), 4.15-4.33 (m, 3H), 4.53-4.66 (m, 1H), 6.65 (d, J = 8 Hz, 2H), 7.08 (d, J = 8 Hz, 2H), 7.17-7.37 (m, 5H), 8.13 (s, 3H), 8.15 (d, J = 8 Hz, 1H), 8.44 (t, J = 6 Hz, 1H), 8.63 (d, J = 8 Hz, 1H), 9.26 (s, 1H)
   MASS (m/e):
      483 (M⁺+1)

### EXAMPLE 25

### Production of L-norleucyl-L-tyrosyl-N-benzyl-L-isoleucinamide hydrochloride

The title compound was obtained in the same manner as in Example 24 (1) and (2) but starting with L-tyrosylN-benzyl-L-isoleucinamide hydrochloride and N-(tert-butoxycarbonyl)-L-norleucine-N-hydroxysuccinimide ester.
¹H-NMR (DMSO-d₆, 200 MHz) δ (ppm);
   0.73-0.88 (m, 9H), 1.00-1.48 (m, 6H), 1.60-1.82 (m, 3H), 2.69 (dd, J = 9.14 Hz, 1H), 2.89 (dd, J = 5.14 Hz, 1H), 3.72 (t, J = 7 Hz, 1H), 4.15-4.29 (m, 3H), 4.55-4.67 (m, 1H), 6.65 (d, J = 9 Hz, 2H), 7.07 (d, J = 9 Hz, 2H), 7.16-7.36 (m, 5H), 8.13 (bs, 3H), 8.16 (d, J = 8 Hz, 1H), 8.41 (t, J = 6 Hz, 1H), 8.63 (d, J = 8 Hz, 1H), 9.26 (s, 1H)
MASS (m/e):
   497 (M⁺+1)

### EXAMPLE 26

### Production of L-isoleucyl-L-tyrosyl-N-benzyl-L-isoleucinamide hydrochloride

The title compound was obtained in the same manner as in Example 24 but starting with L-tyrosyl-N-benzyl-L-isoleucinamide hydrochloride and N-(tert-butoxycarbonyl)-L-isoleucine-N-hydroxysuccinimide ester.
¹H-NMR (DMSO-d₆, 200 MHz) δ (ppm);
   0.73-0.92 (m, 12H), 0.96-1.17 (m, 2H), 1.30-1.52 (m, 2H), 1.67-1.93 (m, 2H), 2.70 (dd, J = 9.15 Hz, 1H), 2.87 (dd, J = 5.15 Hz, 1H), 3.63 (d, J = 5 Hz, 1H), 4.14-4.30 (m, 3H), 4.55-4.68 (m, 1H), 6.65 (d, J = 9 Hz, 2H), 7.07 (d, J = 9 Hz, 2H), 7.18-7.35 (m, 5H), 8.07 (bs, 3H), 8.22 (d, J = 8 Hz, 1H), 8.37 (t, J = 6 Hz, 1H), 8.53 (d, J = 7 Hz, 1H), 9.26 (s, 1H)
MASS (m/e):
   497 (M⁺+1)

### EXAMPLE 27

### Production of N'-[N-[O-benzyl-L-seryl]-L-tyrosyl]-N-benzyl-L-isoleucinamide hydrochloride

The title compound was obtained in the same manner as in Example 24 but starting with L-tyrosyl-N-benzyl-L-isoleucinamide hydrochloride and N-(tert-butoxycarbonyl)-O-benzylserine-N-hydroxysuccinimide ester.
¹H-NMR (DMSO-d₆, 200 MHz) δ (ppm);
   0.76 (t, J = 7 Hz, 3H), 0.78 (d, J = 7 Hz, 3H), 0.94-1.13 (m, 1H), 1.31-1.48 (m, 1H), 1.66-1.81 (m, 1H), 2.70 (dd, J = 9.14 Hz, 1H), 2.93 (dd, J = 5.14 Hz, 1H), 3.68 (dd, J = 5.10 Hz, 1H), 3.82 (dd, J = 3.10 Hz, 1H), 3.95-4.03 (m, 1H), 4.16-4.31 (m, 3H), 4.51 (s, 2H), 4.57-4.68 (m, 1H), 6.65 (d, J = 9 Hz, 2H), 7.08 (d, J = 9 Hz, 2H), 7.17-7.38 (m, 10H), 8.21 (d, J = 8 Hz, 1H), 8.28 (bs, 3H), 8.49 (t, J = 5 Hz, 1H), 8.73 (d, J = 7 Hz, 1H), 9.29 (s, 1H)
MASS (m/e):
   561 (M⁺+1)

### EXAMPLE 28

### Production of L-methionyl-L-tyrosyl-N-benzyl-L-isoleucinamide hydrochloride

The title compound was obtained in the same manner as in Example 24 but starting with L-tyrosyl-N-benzyl-L-isoleucinamide hydrochloride and N-(tert-butoxycarbonyl)-L-methionine-N-hydroxysuccinimide ester.
¹H-NMR (DMSO-d₆, 200 MHz) δ (ppm);
   0.75-0.86 (m, 6H), 1.00-1.22 (m, 1H), 1.33-1.52 (m, 1H), 1.67-1.85 (m, 1H), 1.90-2.10 (m, 2H), 2.04 (s, 3H), 2.40-2.58 (m, 2H), 2.69 (dd, J = 10, 15 Hz, 1H), 2.90 (dd, J = 5.15 Hz, 1H), 3.73-3.86 (m, 1H), 4.15-4.32 (m, 3H), 4.52-4.65 (m, 1H), 6.66 (d, J = 8 Hz, 2H), 7.10 (d, J = 8 Hz, 2H), 7.16-7.36 (m, 5H), 8.24 (d, J = 9 Hz, 1H), 8.29 (bs, 3H), 8.47 (t, J = 6 Hz, 1H), 8.66 (d, J = 8 Hz, 1H), 9.28 (bs, 1H)
MASS (m/e):
   515 (M⁺+1)

### EXAMPLE 29

### Production of L-lysyl-L-tyrosyl-N-benzyl-L-isoleucinamide dihydrochloride

(1) To a solution of L-tyrosyl-N-benzyl-L-isoleucinamide hydrochloride (420 mg, 1.0 mmol), 1-ethyl-3-(3-dimethylaminopropyl)carbodiimide hydrochloride (168 mg, 1.1 mmol) and 1-hydroxybenzotriazole monohydrate (383 mg, 2.0 mmol) in tetrahydrofuran (20 ml) was added N,N'-bis (tert-butoxycarbonyl)-L-lysinedicyclohexylamine salt (580 mg, 1.1 mmol) and the resulting mixture was stirred at room temperature for 4 hours. The reaction mixture was poured into water, extracted with ethyl acetate, washed with water, diluted hydrochloric acid, a saturated aqueous solution of sodium hydrogencarbonate and a saturated aqueous solution of sodium chloride and dried. After distilling off the solvent, the obtained residue was purified by silica gel column chromatography [eluent: methanol/chloroform (1 : 20)] to thereby give N'-[N-[N,N'-bis(tert-butoxycarbonyl)-L-lysyl]-L-tyrosyl]-N-benzyl-L-isoleucinamide (550 mg).
(2) A solution of N'-[N-[N,N'-bis(tert-butoxycarbonyl)-L-lysyl]-L-tyrosyl]-N-benzyl-L-isoleucinamide (420 mg, 0.59 mmol) in a 4 N solution of hydrochloric acid/ethyl acetate (8 ml) was stirred at room temperature for 2 hours. Then the reaction mixture was diluted with ethyl acetate (20 ml) and the crystals thus obtained were collected by filtration and dried under reduced pressure to thereby give the title compound (340 mg).
   ¹H-NMR (DMSO-d₆, 200 MHz) δ (ppm);
      0.75-0.93 (m, 6H), 1.02-1.87 (m, 9H), 2.65-2.73 (m, 3H), 2.94 (dd, J = 5.15 Hz, 1H), 3.65-3.85 (m, 1H), 4.17-4.34 (m, 3H), 4.50-4.67 (m, 1H), 6.69 (d, J = 8 Hz, 2H), 7.13 (d, J = 8 Hz, 2H), 7.20-7.38 (m, 5H), 8.11 (bs, 3H), 8.22 (d, J = 9 Hz, 1H), 8.26 (bs, 3H), 8.51 (t, J = 5 Hz, 1H), 8.78 (d, J = 8 Hz, 1H), 9.31 (bs, 1H)
   MASS (m/e):
      512 (M⁺+1)

### EXAMPLE 30

### Production of N'-[N-[3-(methoxycarbonyl)-L-alanyl]-L-tyrosyl]-N-benzyl-L-isoleucinamide hydrochloride

The title compound was obtained in the same manner as in Example 24 but starting with L-tyrosyl-N-benzyl-L-isoleucinamide hydrochloride and N-(tert-butoxycarbonyl)-3-(methoxycarbonyl)-L-alanine-N-hydroxysuccinimide ester.
¹H-NMR (DMSO-d₆, 200 MHz) δ (ppm);
   0.74-0.88 (m, 6H), 0.95-1.21 (m, 1H), 1.32-1.54 (m, 1H), 1.66-1.86 (m, 1H), 2.57-3.02 (m, 4H), 3.65 (s, 3H), 3.98-4.33 (m, 4H), 4.47-4.62 (m, 1H), 6.66 (d, J = 8 Hz, 2H), 7.07 (d, J = 8 Hz, 2H), 7.16-7.37 (m, 5H), 8.17 (d, J = 9 Hz, 1H), 8.28 (bs, 3H), 8.47 (t, J = 5 Hz, 1H), 8.66 (d, J = 8 Hz, 1H), 9.28 (s, 1H)
MASS (m/e):
   513 (M⁺+1)

### EXAMPLE 31

### Production of L-phenylalanyl-L-tyrosyl-N-benzyl-L-isoleucinamide hydrochloride

The title compound was obtained in the same manner as in Example 24 but starting with L-tyrosyl-N-benzyl-L-isoleucinamide hydrochloride and N-(tert-butoxycarbonyl)-L-phenylalanine-N-hydroxysuccinimide ester.
¹H-NMR (DMSO-d₆, 200 MHz) δ (ppm);
   0.82 (t, J = 7 Hz, 3H), 0.83 (d, J = 8 Hz, 3H), 1.00-1.22 (m, 1H), 1.35-1.55 (m, 1H), 1.66-1.86 (m, 1H), 2.74 (dd, J = 9.14 Hz, 1H), 2.85-3.04 (m, 2H), 3.18 (dd, J = 5.14 Hz, 1H), 3.96-4.08 (m, 1H), 4.18-4.32 (m, 3H), 4.56-4.67 (m, 1H), 6.66 (d, J = 8 Hz, 2H), 7.10 (d, J = 8 Hz, 2H), 7.16-7.35 (m, 10H), 8.14 (bs, 3H), 8.27 (d, J = 8 Hz, 1H), 8.48 (t, J = 6 Hz, 1H), 8.83 (t, J = 8 Hz, 1H), 9.28 (s, 1H)
MASS (m/e):
   531 (M⁺+1)

### EXAMPLE 32

### Production of L-norleucyl-L-tryptophyl-N-(2-phenylethyl)-L-leucinamide hydrochloride

(1) To a solution of N-(2-phenylethyl)-L-leucinamide (478 mg, 2.0 mmol) in N,N-dimethylformamide (20 ml) was added L-tryptophane-N-hydroxysuccinimide ester (820 mg, 2.0 mmol) and the resulting mixture was stirred at room temperature for 2 hours. The reaction mixture was poured into water, extracted with ethyl acetate, washed with water, diluted hydrochloric acid and an aqueous solution of sodium chloride and dried. After distilling off the solvent, the obtained residue was purified by silica gel column chromatography [eluent: methanol/chloroform (1 : 20)] to thereby give N'-[N-(tert-butoxycarbonyl)-L-tryptophyl]-N-(2-phenylethyl)-L-leucinamide (960 mg).
(2) A solution of N'-[N-(tert-butoxycarbonyl)-L-tryptophyl]-N-(2-phenylethyl)-L-leucinamide (960 mg) in a 4 N solution of hydrochloric acid/ethyl acetate (10 ml) was stirred at room temperature for 2 hours. Then the reaction mixture was diluted with ethyl acetate (10 ml) and hexane (20 ml) and the crystals thus obtained were collected by filtration and dried under reduced pressure to thereby give L-tryptophyl-N-(2-phenylethyl)-L-leucinamide hydrochloride (766 mg).
   ¹H-NMR (DMSO-d₆, 200 MHz) δ (ppm);
      0.83 (d, J = 7 Hz, 3H), 0.87 (d, J = 7 Hz, 3H), 1.46-1.61 (m, 3H), 2.71 (t, J = 7 Hz, 2H), 3.08 (dd, J = 8.15 Hz, 1H), 3.17-3.48 (m, 3H), 3.97-4.10 (m, 1H), 4.24-4.36 (m, 1H), 6.94-7.40 (m, 9H), 7.74 (d, J = 8 Hz, 1H), 8.11 (t, J = 6 Hz, 1H), 8.16 (bs, 3H), 8.80 (d, J = 8 Hz, 1H), 11.07 (s, 1H)
(3) The title compound was obtained in the same manner as in Example 24 but starting with L-tryptophyl-N-(2-phenylethyl)-L-leucinamide hydrochloride and N-(tert-butoxycarbonyl)-L-norleucine-N-hydroxysuccinimide ester.
   ¹H-NMR (DMSO-d₆, 200 MHz) δ (ppm);
      0.81 (t, J = 7 Hz, 3H), 0.84 (d, J = 7 Hz, 6H), 1.15-1.78 (m, 9H), 2.65 (t, J = 7 Hz, 2H), 2.96 (dd, J = 8.15 Hz, 1H), 3.07-3.28 (m, 3H), 3.67-3.80 (m, 1H), 4.21-4.33 (m, 1H), 4.63-4.77 (m, 1H), 6.94-7.36 (m, 10H), 7.64-7.77 (m, 2H), 8.13-8.29 (m, 4H), 8.73 (d, J = 8 Hz, 1H), 10.94 (s, 1H)
   MASS (m/e):
      534 (M⁺+1)

### EXAMPLE 33

### Production of N'-[N-(L-norleucyl)-N^{im}-benzyl-L-histidyl]-N-(2-phenylethyl)-L-leucinamide dihydrochloride

(1) To a solution of N-(2-phenylethyl)-L-leucinamide (450 mg, 1.9 mmol) and N-(tert-butoxycarbonyl)-N^{im}-benzyl-L-histidine (670 mg, 1.9 mmol) in N,N-dimethylformamide (20 ml) were added 1-ethyl-3-(3-dimethylaminopropyl)carbodiimide hydrochloride (410 mg, 2.1 mmol) and 1-hydroxybenzotriazole monohydrate (590 mg, 3.9 mmol) and the resulting mixture was stirred at room temperature for 3 hours. The reaction mixture was poured into water, extracted with ethyl acetate, washed with water, diluted hydrochloric acid and an aqueous solution of sodium chloride, and dried. After distilling off the solvent, the obtained residue was purified by silica gel column chromatography [eluent: methanol/chloroform (1 : 20)] to thereby give N'-[N-(tert-butoxycarbonyl)-N^{im}-benzyl-L-histidyl]-N-(2-phenylethyl)-L-leucinamide (900 mg).
(2) A solution of N'-[N-(tert-butoxycarbonyl)-N^{im}-benzyl-L-histidyl]-N-(2-phenylethyl)-L-leucinamide (900 mg) in a 4 N solution of hydrochloric acid/ethyl acetate (8 ml) was stirred at room temperature for 2 hours. Then the reaction mixture was diluted with ethyl acetate (20 ml) and the crystals thus obtained were collected by filtration and dried under reduced pressure to thereby give N'-(N^{im}-benzyl-L-histidyl)-N-(2-phenylethyl)-L-leucinamide dihydrochloride (660 mg).
   ¹H-NMR (DMSO-d₆, 200 MHz) δ (ppm);
      0.83 (d, J = 7 Hz, 3H), 0.87 (d, J = 7 Hz, 3H), 1.26-1.70 (m, 3H), 2.73 (t, J = 7 Hz, 2H), 3.14-3.46 (m, 4H), 4.13-4.33 (m, 2H), 5.40 (s, 2H), 7.13-7.47 (m, 10H), 7.60 (s, 1H), 8.42 (t, J = 6 Hz, 1H), 8.60 (bs, 3H), 8.83 (d, J = 7 Hz, 1H), 9.32 (s, 1H)
(3) The title compound was obtained in the same manner as in Example 24 but starting with N'-(N^{im}-benzyl-L-histidyl)-N-(2-phenylethyl)-L-leucinamide dihydrochloride and N-(butoxycarbonyl)-L-norleucine-N-hydroxysuccinimide ester.
   ¹H-NMR (DMSO-d₆, 200 MHz) δ (ppm);
      0.76-0.87 (m, 9H), 1.16-1.74 (m, 9H), 2.72 (t, J = 7 Hz, 2H), 2.93-3.42 (m, 4H), 3.76-3.92 (m, 1H), 4.15-4.29 (m, 1H), 4.62-4.74 (m, 1H), 5.41 (d, J = 14 Hz, 1H), 5.51 (d, J = 14 Hz, 1H), 7.13-7.49 (m, 10H), 7.67 (s, 1H), 8.24 (t, J = 6 Hz, 1H), 8.32 (d, J = 8 Hz, 1H), 8.49 (bs, 3H), 8.96 (d, J = 7 Hz, 1H), 9.31 (s, 1H), 14.93 (bs, 1H)
   MASS (m/e):
      575 (M⁺+1)

### EXAMPLE 34

### Production of L-norleucyl-L-isoleucyl-N-(2-phenylethyl)-L-leucinamide hydrochloride

L-Isoleucyl-N-(2-phenylethyl)-L-leucinamide hydrochloride was obtained in the same manner as in Example 32 (1) and (2) but starting with N-(2-phenylethyl)-L-leucinamide and N-(tert-butoxycarbonyl)-isoleucine-N-hydroxysuccinimide ester. Next, the title compound was obtained in the same manner as in Example 24 but starting with L-isoleucyl-N-(2-phenylethyl)-L-leucinamide hydrochloride and N-(tert-butoxycarbonyl)norleucine-N-hydroxysuccinimide ester.
¹H-NMR (DMSO-d₆, 200 MHz) δ (ppm);
   0.75-0.88 (m, 15H), 0.95-1.77 (m, 12H), 2.69 (t, J = 7 Hz, 2H), 3.22-3.36 (m, 2H), 3.83 (bs, 1H), 4.17-4.33 (m, 2H), 7.13-7.32 (m, 5H), 8.00 (t, J = 6 Hz, 1H), 8.13 (d, J = 7 Hz, 1H), 8.24 (bs, 3H), 8.50 (d, J = 7 Hz, 1H)
MASS (m/e):
   461 (M⁺+1)

### EXAMPLE 35

### Production of N'-[N-[L-2-amino-4-(benzyloxy)butanoyl]-L-tyrosyl]-N-(2-phenylethyl)-L-leucinamide hydrochloride

(1) L-2-(tert-Butoxycarbonylamino)-4-(benzyloxy)butanoic acid (155 mg, 0.50 mmol), L-tyrosyl-N-(2-phenylethyl)-L-leucinamide hydrochloride (252 mg, 0.50 mmol), 1-hydroxybenzotriazole monohydrate (84 mg, 0.55 mmol), N,N'-dicyclohexylcarbodiimide (113 mg, 0.55 mmol) and triethylamine (101 mg, 1.0 mmol) were allowed to react in tetrahydrofuran (13 ml) in the same manner as in Example 3 (1) to thereby give N'-[N-[2-(tert-butoxycarbonylamino)-4-(benzyloxy)butanoyl]-L-tyrosyl]-N-(2-phenylethyl)-L-leucinamide (220 mg).
   ¹H-NMR (DMSO-d₆, 400 MHz) δ (ppm);
      0.78 (d, J = 6.4 Hz, 3H), 0.83 (d, J = 6.5 Hz, 3H), 1.18-1.90 (m, 5H), 1.36 (s, 9H), 2.68 (t, J = 7.3 Hz, 2H), 2.72 (dd, J = 9.4, 13.9 Hz, 1H), 2.85 (dd, J = 5.2, 13.9 Hz, 1H), 3.18-3.44 (m, 4H), 3.99 (m, 1H), 4.22 (m, 1H), 4.37 (d, J = 12.5 Hz, 1H), 4.44 (m, 1H), 4.44 (d, J = 12.5 Hz, 1H), 6.60 (d, J = 8.4 Hz, 2H), 6.96 (d, J = 8.4 Hz, 2H), 7.14-7.40 (m, 10H), 7.69 (d, J = 7.8 Hz, 1H), 7.74 (t, J = 5.5 Hz, 1H), 7.99 (d, J = 8.4 Hz, 1H), 9.15 (s, 1H)
(2) N'-[N-[2-(tert-Butoxycarbonylamino)-4-(benzyloxy)butanoyl]-L-tyrosyl]-N-(2-phenylethyl)-L-leucinamide (180 mg, 0.26 mmol) and a 4 N solution of hydrochloric acid/ethyl acetate (4.0 ml) were allowed to react in chloroform (5.0 ml) and ethyl acetate (5.0 ml) in the same manner as in Example 3 (2) to thereby give the title compound (120 mg).
   ¹H-NMR (DMSO-d₆, 400 MHz) δ (ppm);
      0.78 (d, J = 6.5 Hz, 3H), 0.83 (d, J = 6.5 Hz, 3H), 1.34-1.59 (m, 3H), 1.89-2.08 (m, 2H), 2.68 (t, J = 7.4 Hz, 2H), 2.69 (dd, J = 9.0, 13.9 Hz, 1H), 2.90 (dd, J = 4.9, 13.9 Hz, 1H), 3.19-3.31 (m, 2H), 3.51 (t, J = 6.7 Hz, 2H), 3.81 (dd, J = 5.8, 6.9 Hz, 1H), 4.25 (m, 1H), 4.45 (s, 2H), 4.53 (m, 1H), 6.66 (d, J = 8.4 Hz, 2H), 7.07 (d, J = 8.4 Hz, 2H), 7.15-7.40 (m, 10H), 7.80 (t, J = 5.6 Hz, 1H), 8.06 (br, 3H), 8.21 (d, J = 8.3 Hz, 1H), 8.60 (d, J = 8.1 Hz, 1H), 9.27 (s, 1H)
   MASS (m/e):
      588 (M⁺)

### EXAMPLE 36

### Production of N'-[N-(L-norleucyl)-3,4-dihydroxy-L-phenylalanyl]-N-(2-phenylethyl)-L-leucinamide hydrochloride

(1) 3,4-Dihydroxy-L-phenylalanine (3.9 g, 20 mmol) was dissolved in 1,4-dioxane (50 ml) and a 1 N aqueous solution of sodium hydroxide (50 ml) and di-tert-butyl dicarbonate (4.8 g, 22 mmol) was added thereto. After stirring at room temperature for 40 hours, 1,4-dioxane was distilled off under reduced pressure. An aqueous solution of citric acid was added to the residue so as to adjust the pH value to about 4. Then the reaction mixture was extracted with chloroform and the organic layer was washed with water and a saturated aqueous solution of sodium chloride and dried over anhydrous sodium sulfate. Then the solvent was distilled off under reduced pressure to thereby give N-(tert-butoxycarbonyl)-3,4-dihydroxy-L-phenylalanine (4.5 g).
   ¹H-NMR (DMSO-d₆, 200 MHz) δ (ppm);
      1.33 (s, 9H), 2.57 (dd, J = 9.7, 12.9 Hz, 1H), 2.77 (dd, J = 4.5, 12.9 Hz, 1H), 3.96 (m, 1H), 6.47 (d, J = 7.8 Hz, 1H), 6.61 (s, 1H), 6.61 (d, J = 7.8 Hz, 1H), 6.94 (d, J = 8.4 Hz, 1H), 8.67 (s, 1H), 8.71 (s, 1H), 12.50 (br, 1H)
(2) N-(tert-Butoxycarbonyl)-3,4-dihydroxy-L-phenylalanine (591 mg, 3.0 mmol), N-(2-phenylethyl)-L-leucinamide hydrochloride (813 mg, 3.0 mmol), 1-hydroxybenzotriazole monohydrate (505 mg, 3.3 mmol), N,N'-dicyclohexylcarbodiimide (680 mg, 3.3 mmol) and triethylamine (606 mg, 6.0 mmol) were allowed to react in tetrahydrofuran (30 ml) in the same manner as in Example 3 (1) to thereby give N'-[N-(tert-butoxycarbonyl)-3,4-dihydroxy-L-phenylalanyl]-N-(2-phenylethyl)-L-leucinamide (450 mg).
   ¹H-NMR (DMSO-d₆, 400 MHz) δ (ppm);
      0.80 (d, J = 6.5 Hz, 3H), 0.84 (d, J = 6.5 Hz, 3H), 1.32 (s, 9H), 1.30-1.59 (m, 3H), 2.57 (dd, J = 9.8, 13.7 Hz, 1H), 2.67 (t, J = 7.2 Hz, 2H), 2.75 (dd, J = 4.7, 13.7 Hz, 1H), 3.16-3.32 (m, 2H), 4.04 (m, 1H), 4.24 (m, 1H), 6.48 (dd, J = 1.5, 8.1 Hz, 1H), 6.60 (d, J = 8.1 Hz, 1H), 6.62 (d, J = 1.5 Hz, 1H), 6.87 (d, J = 8.3 Hz, 1H), 7.16-7.23 (m, 3H), 7.23-7.32 (m, 2H), 7.72-7.81 (m, 2H), 8.67 (s, 1H), 8.68 (s, 1H)
(3) N'-[N-(tert-Butoxycarbonyl)-3,4-dihydroxy-L-phenylalanyl]-N-(2-phenylethyl)-L-leucinamide (400 mg, 0.78 mmol) and a 4 N solution of hydrochloric acid/ethyl acetate (4.0 ml) were allowed to react in ethyl acetate (10 ml) in the same manner as in Example 2 (4) to thereby give the N'-(3,4-dihydroxy-L-phenylalanyl)-N-(2-phenylethyl)-L-leucinamide hydrochloride (300 mg).
   ¹H-NMR (DMSO-d₆, 200 MHz) δ (ppm);
      0.83 (d, J = 6.5 Hz, 3H), 0.87 (d, J = 6.7 Hz, 3H), 1.30-1.65 (m, 3H), 2.70 (dd, 1H), 2.71 (t, J = 7.2 Hz, 2H), 2.94 (dd, J = 4.9, 14.1 Hz, 1H), 3.30-3.45 (m, 2H), 3.91 (m, 1H), 4.27 (m, 1H), 6.50 (dd, J = 1.8, 8.1 Hz, 1H), 6.66 (d, J = 8.1 Hz, 1H), 6.68 (d, J = 1.8 Hz, 1H), 7.14-7.34 (m, 5H), 8.01 (t, J = 5.6 Hz, 1H), 8.04 (m, 3H), 8.64 (d, J = 8.2 Hz, 1H), 8.82 (s, 1H), 8.92 (s, 1H)
(4) N-(tert-Butoxycarbonyl)-L-norleucine (139 mg, 0.60 mmol), N'-(3,4-dihydroxy-L-phenylalanyl)-N-(2-phenylethyl)-L-leucinamide hydrochloride (271 mg, 0.60 mmol), 1-hydroxybenzotriazole monohydrate (101 mg, 0.66 mmol), N,N'-dicyclohexylcarbodiimide (136 mg, 0.66 mmol) and triethylamine (121 mg, 1.2 mmol) were allowed to react in tetrahydrofuran (20 ml) in the same manner as in Example 3 (1) to thereby give N'-[N-[N-(tert-butoxycarbonyl)-L-norleucyl]-3,4-dihydroxy-L-phenylalanyl]-N-(2-phenylethyl)-L-leucinamide (220 mg).
   ¹H-NMR (DMSO-d₆, 400 MHz) δ (ppm);
      0.78 (d, J = 6.4 Hz, 3H), 0.82 (t, J = 7.3 Hz, 3H), 0.83 (d, J = 6.6 Hz, 3H), 0.96-1.67 (m, 9H), 1.37 (s, 9H), 2.62 (dd, J = 8.5, 13.8 Hz, 1H), 2.67 (t, J = 7.4 Hz, 2H), 2.79 (dd, J = 5.7, 13.8 Hz, 1H), 3.16-3.30 (m, 2H), 3.82 (m, 1H), 4.20 (m, 1H), 4.44 (m, 1H), 6.45 (dd, J = 1.8, 8.0 Hz, 1H), 6.58 (d, J = 8.0 Hz, 1H), 6.61 (d, J = 1.8 Hz, 1H), 6.84 (d, J = 8.0 Hz, 1H), 7.16-7.32 (m, 5H), 7.54 (t, J = 5.5 Hz, 1B), 7.77 (d, J = 8.1 Hz, 1H), 7.92 (d, J = 8.3 Hz, 1H), 8.62 (s, 1H), 8.69 (s, 1H)
(5) N'-[N-[N-(tert-Butoxycarbonyl)-L-norleucyl]-3,4-dihydroxy-L-phenylalanyl]-N-(2-phenylethyl)-L-leucinamide (200 mg, 0.32 mmol) and a 4 N solution of hydrochloric acid/ethyl acetate (2.0 ml) were allowed to react in chloroform (6.0 ml) and ethyl acetate (3.0 ml) in the same manner as in Example 3 (2) to thereby give the title compound (160 mg).
   ¹H-NMR (DMSO-d₆, 400 MHz) δ (ppm);
      0.78 (d, J = 6.5 Hz, 3H), 0.84 (d, J = 6.8 Hz, 3H), 0.85 (t, J = 6.8 Hz, 3H), 1.16-1.56 (m, 7H), 1.61-1.74 (m, 2H), 2.60 (dd, J = 9.4, 14.0 Hz, 1H), 2.67 (t, J = 7.4 Hz, 2H), 2.82 (dd, J = 5.2, 14.0 Hz, 1H), 3.20-3.29 (m, 2H), 3.70 (m, 1H), 4.24 (m, 1H), 4.54 (m, 1H), 6.53 (dd, J = 2.0, 8.0 Hz, 2H), 6.63 (d, J = 8.0 Hz, 1H), 6.71 (d, J = 2.0 Hz, 1H), 7.15-7.32 (m, 5H), 7.60 (t, J = 5.7 Hz, 1H), 8.10 (bs, 3H), 8.17 (d, J = 8.4 Hz, 1H), 8.61 (d, J = 8.3 Hz, 1H), 8.74 (s, 1H), 8.78 (s, 1H)
   MASS (m/e):
      526 (M⁺)

### EXAMPLE 37

### Production of L-valyl-L-tyrosyl-N-[2-(4-chlorophenyl)-ethyl]-L-isoleucinamide hydrochloride

(1) N-[N-[N-(Benzyloxycarbonyl)-L-valyl]-L-tyrosyl]-L-isoleucine (211 mg, 0.40 mmol), 1-hydroxybenzotriazole monohydrate (54 mg, 0.44 mmol), N,N'-dicyclohexylcarbodiimide (0.82 mg, 0.44 mmol) and 2-(4-chlorophenyl)ethylamine (62 mg, 0.40 mmol) were allowed to react in N,N-dimethylformamide (10 ml) in the same manner as in Example 1 (3) to thereby give N'-[N-[N-(benzyloxycarbonyl)-L-valyl]-L-tyrosyl]-N-[2-(4-chlorophenyl)ethyl]-L-isoleucinamide (95 mg).
   ¹H-NMR (DMSO-d₆, 400 MHz) δ (ppm);
      0.75 (d, J = 6.7 Hz, 3H), 0.75 (d, J = 6.8 Hz, 3H), 0.78 (d, J = 6.6 Hz, 3H), 0.79 (t, J = 7.1 Hz, 3H), 1.06 (m, 1H), 1.40 (m, 1H), 1.71 (m, 1H), 1.88 (m, 1H), 2.68 (dd, J = 9.0, 13.8 Hz, 1H), 2.85 (dd, J = 4.9, 13.8 Hz, 1H), 3.83 (dd, J = 7.2, 8.9 Hz, 1H), 4.17 (dd, J = 8.2, 8.2 Hz, 1H), 4.14-4.26 (m, 1H), 4.53 (m, 1H), 4.98 (d, J = 12.4 Hz, 1H), 5.05 (d, J = 12.4 Hz, 1H), 6.60 (d, J = 8.4 Hz, 2H), 7.00 (d, J = 8.4 Hz, 2H), 7.10 (d, J = 8.4 Hz, 2H), 7.13 (d, J = 8.4 Hz, 2H), 7.21 (d, J = 8.9 Hz, 1H), 7.26-7.40 (m, 5H), 7.85 (d, J = 8.9 Hz, 1H), 7.96 (d, J = 8.2 Hz, 1H), 8.24 (t, J = 5.9 Hz, 1H), 9.16 (s, 1H)
(2) Using N'-[N-[N-(benzyloxycarbonyl)-L-valyl]-L-tyrosyl]-N-[2-(4-chlorophenyl)ethyl]-L-isoleucinamide (80 mg, 0.12 mmol), 10% palladium-carbon (25 mg) and a 4 N solution of hydrochloric acid/ethyl acetate (0.060 ml), a reaction was performed in methanol (10 ml) and tetrahydrofuran (10 ml) in the same manner as in Example 10 (4) to thereby give the title compound (48 mg).
   ¹H-NMR (DMSO-d₆, 400 MHz) δ (ppm);
      0.70 (d, J = 6.8 Hz, 3H), 0.75 (t, J = 7.4 Hz, 3H), 0.86 (d, J = 6.9 Hz, 3H), 0.92 (d, J = 6.9 Hz, 3H), 1.01 (m, 1H), 1.31 (m, 1H), 1.64 (m, 1H), 2.09 (m, 1H), 2.67 (dd, 1H), 2.68 (t, J = 7.2 Hz, 2H), 2.84 (dd, J = 5.2, 13.8 Hz, 1H), 3.19-3.39 (m, 2H), 3.57 (d, J = 4.6 Hz, 1H), 4.08 (dd, J = 8.3, 8.9 Hz, 1H), 4.61 (m, 1H), 6.64 (d, J = 8.5 Hz, 2H), 7.07 (d, J = 8.5 Hz, 2H), 7.23 (d, J = 8.5 Hz, 2H), 7.32 (d, J = 8.5 Hz, 2H), 7.83 (t, J = 5.7 Hz, 1H), 7.93 (br, 3H), 8.09 (d, J = 8.9 Hz, 1H), 8.49 (d, J = 7.8 Hz, 1H), 9.21 (s, 1H)
   MASS (m/e):
      530 (M⁺)

### EXAMPLE 38

### Production of N'-[N-(L-Norleucyl)-4-nitro-L-phenylalanyl]-N-(2-phenylethyl)-L-leucinamide hydrochloride

(1) To a suspension of 4-nitro-L-phenylalanine (2.28 g, 10 mmol) in dioxane/water (2 : 1) (30 ml), a 1 N aqueous solution of sodium hydroxide (10 ml) was added and dissolved therein. After adding di-tert-butyl dicarbonate (2.24 g, 10 mmol), the reaction mixture was stirred at room temperature for 3 hours. After allowing to stand overnight, the solvent was distilled off under reduced pressure. The residue was dissolved in water, acidified with hydrochloric acid and then extracted with ethyl acetate. After drying, the solvent was distilled off under reduced pressure to thereby give N-(tert-butoxycarbonyl)-4-nitro-L-phenylalanine (3.22 g).
(2) To a solution of N-(2-phenylethyl)-L-leucinamide (702 mg, 3.0 mmol) and N-(tert-butoxycarbonyl)-4-nitro-L-phenylalanine (984 mg, 3.0 mmol) in N,N-dimethylformamide (20 ml) were added 1-ethyl-3-(3-dimethylaminopropyl)carbodiimide hydrochloride (632 mg, 3.3 mmol) and 1-hydroxybenzotriazole monohydrate (918 mg, 6.0 mmol) and the mixture was stirred at room temperature for 5 hours. The reaction mixture was poured into water and extracted with ethyl acetate. Then the organic layer was washed with water, diluted hydrochloric acid, a saturated aqueous solution of sodium hydrogencarbonate and an aqueous solution of sodium chloride. After drying, the solvent was distilled off under reduced pressure and the obtained residue was purified by silica gel column chromatography [eluent: methanol/chloroform (1 : 20)] to thereby give N'-[N-(tert-butoxycarbonyl)-4-nitro-L-phenylalanyl]-N-(2-phenylethyl)-L-leucinamide (1.30 g).
(3) A solution of N'-[N-(tert-butoxycarbonyl)-4-nitro-L-phenylalanyl]-N-(2-phenylethyl)-L-leucinamide (1.11 g, 14.9 mmol) in a 4 N solution of hydrochloric acid/ethyl acetate (14 ml) was stirred at room temperature for 2 hours. Then n-hexane was added to the reaction mixture and the crystals thus obtained were collected by filtration and dried to thereby give N'-(4-nitro-L-phenylalanyl)-N-(2-phenylethyl)-L-leucinamide hydrochloride (0.98 g).
   ¹H-NMR (DMSO-d₆, 200 MHz) δ (ppm);
      0.82 (d, J = 6 Hz, 3H), 0.85 (d, J = 6 Hz, 3H), 1.32-1.64 (m, 3H), 2.71 (t, J = 7 Hz, 2H), 3.06-3.37 (m, 4H), 4.05-4.30 (m, 2H), 7.13-7.32 (m, 5H), 7.56 (d, J = 8 Hz, 2H), 8.16 (d, J = 8 Hz, 2H), 8.21 (t, J = 6 Hz, 1H), 8.37 (bs, 3H), 8.74 (d, J = 8 Hz, 1H)
(4) To a solution of N'-(4-nitro-L-phenylalanyl)-N-(2-phenylethyl)-L-leucinamide hydrochloride (0.70 g, 1.5 mmol) and triethylamine (0.15 g, 1.5 mmol) in N,N-dimethylformamide (20 ml) were added N-(tert-butoxycarbonyl)-L-norleucine-N-hydroxysuccinimide ester (0.48 g, 1.5 mmol) and the mixture was stirred at room temperature for 1 hour. The reaction mixture was poured into water, extracted with ethyl acetate and washed with diluted hydrochloric acid, a saturated aqueous solution of sodium hydrogencarbonate and an aqueous solution of sodium chloride. After drying, the solvent was distilled off under reduced pressure and the obtained residue was purified by silica gel column chromatography [eluent: methanol/chloroform (1 : 20)] to thereby give N'-[N'-[N-(tert-butoxycarbonyl)-L-norleucyl]-4-nitro-L-phenylalanyl]-N-(2-phenylethyl)-L-leucinamide (0.89 g).
   ¹H-NMR (CDCl₃, 200 MHz) δ (ppm);
      0.82-0.93 (m, 9H), 1.15-1.80 (m, 9H), 1.37 (s, 9H), 2.84 (t, J = 7 Hz, 2H), 3.13 (d, J = 6 Hz, 2H), 3.38-3.61 (m, 2H), 3.91-4.02 (m, 1H), 4.40-4.56 (m, 1H), 4.75 (q, J = 8 Hz, 1H), 5.04 (d, J = 6 Hz, 1H), 6.82 (bs, 1H), 6.93-7.33 (m, 9H), 8.08 (d, J = 9 Hz, 2H)
(5) A solution of N'-[N-[N-(tert-butoxycarbonyl)-L-norleucyl]-4-nitro-L-phenylalanyl]-N-(2-phenylethyl)-L-leucinamide (205 mg, 0.31 mmol) in a 4 N solution of hydrochloric acid/ethyl acetate (6 ml) was stirred at room temperature for 2 hours. After allowing to stand overnight, it was diluted with ethyl acetate and the crystals thus precipitated were collected by filtration and dried to thereby give the title compound (160 mg).
   ¹H-NMR (DMSO-d₆, 200 MHz) δ (ppm);
      0.77-0.88 (m, 9H), 1.15-1.76 (m, 9H), 2.70 (t, J = 7 Hz, 2H), 2.94 (dd, J = 9.14 Hz, 1H), 3.15 (dd, J = 5.14 Hz, 1H), 3.22-3.36 (m, 2H), 3.72 (t, J = 6 Hz, 1H), 4.20-4.34 (m, 1H), 4.65-4.77 (m, 1H), 7.13-7.32 (m, 5H), 7.60 (d, J = 9 Hz, 2H), 8.06 (t, J = 6 Hz, 1H), 8.14 (d, J = 9 Hz, 2H), 8.19 (bs, 3H), 8.33 (d, J = 8 Hz, 1H), 8.78 (d, J = 8 Hz, 1H)

### EXAMPLE 39

### Production of N'-[N-(L-norleucyl)-4-amino-L-phenylalanyl]-N-(2-phenylethyl)-L-leucinamide dihydrochloride

(1) 5% Palladium-carbon (100 mg) was added to a solution of N'-[N-[N-(tert-butoxycarbonyl)-L-norleucyl]-4-nitro-L-phenylalanyl]-N-(2-phenylethyl)-L-leucinamide (400 mg, 0.6 mmol) in methanol (70 ml) and catalytic reduction was performed under a hydrogen atmosphere. Then the reaction mixture was filtered and the solvent was distilled off under reduced pressure. The residue was purified by silica gel column chromatography [eluent: methanol/chloroform (1 : 20)] to thereby give N'-[N-[N-(tert-butoxycarbonyl)-L-norleucyl]-4-amino-L-phenylalanyl]-N-(2-phenylethyl)-L-leucinamide (0.24 g).
(2) To a solution of N'-[N-[N-(tert-butoxycarbonyl)-L-norleucyl]-4-amino-L-phenylalanyl]-N-(2-phenylethyl)-L-leucinamide (0.24 g, 0.4 mmol) in methanol (10 ml) was added a 4 N solution of hydrochloric acid/ethyl acetate (8 ml) and the mixture was stirred at room temperature for 2 hours. After allowing to stand overnight, the solvent was distilled off under reduced pressure and ethyl acetate was added to the residue. The crystals thus precipitated were collected by filtration and dried to thereby give the title compound (0.22 g).
   ¹H-NMR (DMSO-d₆, 200 MHz) δ (ppm);
      0.78-0.90 (m, 9H), 1.20-1.78 (m, 9H), 2.73 (t, J = 7 Hz, 2H), 2.83 (dd, J = 10, 14 Hz, 1H), 3.06 (dd, J = 4.14 Hz, 1H), 3.20-3.40 (m, 2H), 3.67-3.80 (m, 1H), 4.23-4.35 (m, 1H), 4.58-4.70 (m, 1H), 7.14-7.28 (m, 5H), 7.31 (d, J = 8 Hz, 2H), 7.45 (d, J = 8 Hz, 2H), 8.09 (t, J = 6 Hz, 1H), 8.21 (bs, 3H), 8.35 (d, J = 8 Hz, 1H), 8.74 (d, J = 8 Hz, 1H), 10.46 (bs, 3H)

### EXAMPLE 40

### Production of N'-[N-(L-norleucyl)-4-chloro-L-phenylalanyl]-N-(2-phenylethyl)-L-leucinamide hydrochloride

(1) N'-(4-Chloro-L-phenylalanyl)-N-(2-phenylethyl)-L-leucinamide hydrochloride was obtained in the same manner as in Example 38 (1), (2) and (3) but starting with 4-chloro-L-phenylalanine and N-(2-phenylethyl)-L-leucinamide.
   ¹H-NMR (DMSO-d₆, 200 MHz) δ (ppm);
      0.83 (d, J = 6 Hz, 3H), 0.86 (d, J = 6 Hz, 3H), 1.35-1.60 (m, 3H), 2.72 (t, J = 7 Hz, 2H), 2.96 (dd, J = 8.14 Hz, 1H), 3.13 (dd, J = 5.14 Hz, 1H), 3.20-3.40 (m, 2H), 3.98-4.12 (m, 1H), 4.26 (q, J = 8 Hz, 1H), 7.13-7.37 (m, 9H), 8.18 (t, J = 6 Hz, 1H), 8.26 (bs, 3H), 8.73 (d, J = 8 Hz, 1H)
(2) The title compound was obtained in the same manner as in Example 38 (4) and (5) but using N'-(4-chloro-L-phenylalanyl)-N-(2-phenylethyl)-L-leucinamide hydrochloride and N-(tert-butoxycarbonyl)-L-norleucine-N-hydroxysuccinimide ester.
   ¹H-NMR (DMSO-d₆, 200 MHz) δ (ppm);
      0.77-0.88 (m, 9H), 1.17-1.75 (m, 9H), 2.70 (t, J = 7 Hz, 2H), 2.79 (dd, J = 9.14 Hz, 1H), 3.00 (dd, J = 5.14 Hz, 1H), 3.20-3.45 (m, 1H), 3.71 (t, J = 6 Hz, 1H), 4.18-4.32 (m, 1H), 4.55-4.68 (m, 1H), 7.13-7.37 (m, 9H), 7.95 (t, J = 6 Hz, 1H), 8.17 (bs, 3H), 8.27 (d, J = 8 Hz, 1H), 8.71 (d, J = 8 Hz, 1H)

### EXAMPLE 41

### Production of N'-[N-(L-norleucyl)-4-fluoro-DL-phenylalanyl]-N-(2-phenylethyl)-L-leucinamide hydrochloride

(1) N'-(4-Fluoro-DL-phenylalanyl)-N-(2-phenylethyl)-L-leucinamide hydrochloride was obtained in the same manner as in Example 38 (1), (2) and (3) but starting with 4-fluoro-DL-phenylalanine and N-(2-phenylethyl)-L-leucinamide.
(2) The title compound was obtained in the same manner as in Example 38 (4) and (5) but using N'-(4-fluoro-DL-phenylalanyl)-N-(2-phenylethyl)-L-leucinamide hydrochloride and N-(tert-butoxycarbonyl)-L-norleucine-N-hydroxysuccinimide ester.
   ¹H-NMR (DMSO-d₆, 200 MHz) δ (ppm);
      0.78-0.88 (m, 9H), 1.14-1.75 (m, 9H), 2.70 (t, J = 7 Hz, 2H), 2.79 (dd, J = 9.14 Hz, 1H), 3.00 (dd, J = 5.14 Hz, 1H), 3.20-3.35 (m, 2H), 3.71 (t, J = 6 Hz, 1H), 4.20-4.33 (m, 1H), 4.54-4.67 (m, 1H), 7.02-7.37 (m, 9H), 7.93 (t, J = 6 Hz, 1H), 8.16 (bs, 3H), 8.25 (d, J = 8 Hz, 1H), 8.70 (d, J = 8 Hz, 1H)

### EXAMPLE 42

### Production of N'-(L-valyl)-N-[(1S,2S)-1-(hydroxymethyl)-2-methylbutyl]-L-tyrosinamide hydrochloride

(1) To a solution of a mixture of tyrosine benzyl ester p-toluenesulfonate (8.86 g, 20 mmol) and triethylamine (2.02 g, 20 mmol) in tetrahydrofuran (150 ml)/methylene chloride (100 ml) was added N-(tert-butoxycarbonyl)-L-valine-N-hydroxysuccinimide ester (6.28 g, 20 mmol) and the obtained mixture was stirred at room temperature for 24 hours. Then the reaction mixture was poured into water and extracted with methylene chloride. The organic layer was washed successively with a saturated aqueous solution of sodium hydrogencarbonate, 10% hydrochloric acid and a saturated aqueous solution of sodium chloride. After drying, the solvent was distilled off under reduced pressure and the obtained residue was crystallized from chloroform/n-hexane to thereby give N-[N-(tert-butoxycarbonyl)-L-valyl]-L-tyrosine benzyl ester (7.03 g).
   ¹H-NMR (CDCl₃, 200 MHz) δ (ppm);
      0.85 (d, J = 7 Hz, 3H), 0.89 (d, J = 7 Hz, 3H), 1.44 (s, 9H), 1.93-2.12 (m, 1H), 3.02 (d, J = 5 Hz, 2H), 3.86 (dd, J = 7.9 Hz, 1H), 4.88 (dt, J = 5.8 Hz, 1H), 5.02-5.20 (m, 1H), 5.10 (d, J = 15 Hz, 1H), 5.19 (d, J = 15 Hz, 1H), 6.21 (s, 1H), 6.43 (d, J = 8 Hz, 1H), 6.63 (d, J = 9 Hz, 2H), 6.82 (d, J = 9 Hz, 2H), 7.27-7.40 (m, 5H)
(2) 5% Palladium-carbon (0.89 g) was added to a solution of N-[N-(tert-butoxycarbonyl)-L-valyl]-L-tyrosine benzyl ester (8.86 g, 19 mmol) in methanol (100 ml) and catalytic reduction was performed under a hydrogen atmosphere. Then the reaction mixture was filtered and the solvent was distilled off under reduced pressure to thereby give N-[N-(tert-butoxycarbonyl)-L-valyl]-L-tyrosine (7.16 g).
   ¹H-NMR (CDCl₃, 200 MHz) δ (ppm);
      0.84 (d, J = 7 Hz, 6H), 1.43 (s, 9H), 1.85-2.10 (m, 1H), 3.01 (m, 2H), 3.94 (t, J = 8 Hz, 1H), 4.73-4.86 (m, 1H), 5.44 (d, J = 8 Hz, 1H), 6.66 (d, J = 8 Hz, 2H), 6.73 (d, J = 8 Hz, 2H)
(3) To a solution of a mixture of N-[N-(tert-butoxycarbonyl)-L-valyl]-L-tyrosine (1.00 g, 2.6 mmol) and (S)-(+)-isoleucinol (0.34 g, 2.9 mmol) in methylene chloride (30 ml)/tetrahydrofuran (30 ml) were added 1-ethyl-3-(3-dimethylaminopropyl) carbodiimide hydrochloride (0.55 g, 2.9 mmol) and 1-hydroxybenzotriazole monohydrate (0.81 g, 5.3 mmol) and the obtained mixture was stirred at room temperature for 5 hours. After allowing to stand overnight, the reaction mixture was poured into water and extracted with ethyl acetate. Then the organic layer was washed with 10% hydrochloric acid, a saturated aqueous solution of sodium hydrogencarbonate and an aqueous solution of sodium chloride. After drying, the solvent was distilled off under reduced pressure and the obtained residue was purified by silica gel column chromatography [eluent: methanol/chloroform (1 : 20)] to thereby give N'-[N-(tert-butoxycarbonyl)-L-valyl]-N-[1-(hydroxymethyl)-2-methylbutyl]-L-tyrosinamide (0.61 g).
   ¹H-NMR (DMSO-d₆, 200 MHz) δ (ppm);
      0.66-0.73 (m, 12H), 0.90-1.08 (m, 1H), 1.23-1.62 (m, 2H), 1.36 (s, 9H), 1.73-1.90 (m, 1H), 2.67 (dd, J = 8.14 Hz, 1H), 2.84 (dd, J = 5.14 Hz, 1H), 3.30 (t, J = 5 Hz, 2H), 3.50-3.77 (m, 2H), 4.40 (t, J = 5 Hz, 1H), 4.41-4.53 (m, 1H), 6.60 (d, J = 8 Hz, 2H), 6.67 (d, J = 8 Hz, 1H), 7.00 (d, J = 8 Hz, 2H), 7.53 (d, J = 8 Hz, 1H), 7.79 (d, J = 8 Hz, 1H), 9.12 (s, 1H)
(4) A solution of N'-[N-(tert-butoxycarbonyl)-L-valyl]-N-[1-(hydroxymethyl)-2-methylbutyl]-L-tyrosinamide (380 mg, 0.79 mmol) in 4 N solution of hydrochloric acid/ethyl acetate (6 ml) was stirred at room temperature for 6 hours. Then the reaction mixture was diluted with ethyl acetate (30 ml) and the insoluble matters were collected by filtration and reprecipitated from methanol/ethyl acetate to thereby give the title compound (200 mg).
   ¹H-NMR (DMSO-d₆, 200 MHz) δ (ppm);
      0.73-1.10 (m, 13H), 1.30-1.64 (m, 2H), 2.00-2.18 (m, 1H), 2.70 (dd, J = 9.14 Hz, 1H), 2.87 (dd, J = 6.14 Hz, 1H), 3.26-3.34 (m, 2H), 3.52-3.65 (m, 2H), 4.40-4.58 (m, 2H), 6.65 (d, J = 9 Hz, 2H), 7.08 (d, J = 9 Hz, 2H), 7.78 (d, J = 8 Hz, 1H), 8.07 (bs, 3H), 8.58 (d, J = 8 Hz, 1H), 9.25 (s, 1H)
   MASS (m/e):
      380 (M⁺+1)

### EXAMPLE 43

### Production of N'-(L-valyl)-N-(2-hydroxy-1,1-dimethylethyl)-L-tyrosinamide hydrochloride

The title compound was obtained in the same manner as in Example 42 (3) and (4) but starting with N-[N-(tert-butoxycarbonyl)-L-valyl]-L-tyrosine and 2-amino-2-methyl-1-propanol.
¹H-NMR (DMSO-d₆, 300 MHz) δ (ppm);
   0.88-0.96 (m, 6H), 1.12 (s, 3H), 1.13 (s, 3H), 2.08-2.20 (m, 1H), 2.73 (dd, J = 9.14 Hz, 1H), 2.83 (dd, J = 6.14 Hz, 1H), 3.29 (s, 2H), 3.58-3.67 (m, 1H), 4.40-4.48 (m, 1H), 6.67 (d, J = 8 Hz, 2H), 7.07 (d, J = 8 Hz, 2H), 7.52 (s, 1H), 8.13 (bs, 3H), 8.60 (d, J = 7 Hz, 1H)
MASS (m/e):
   352 (M⁺+1)

### EXAMPLE 44

### Production of N'-(L-valyl)-N-[(S)-1-(hydroxymethyl)-3-methylbutyl]-L-tyrosinamide hydrochloride

The title compound was obtained in the same manner as in Example 42 (3) and (4) but starting with N-[N-(tert-butoxycarbonyl)-L-valyl]-L-tyrosine and (S)-(+)-leucinol.
¹H-NMR (DMSO-d₆, 200 MHz) δ (ppm);
   0.76-0.97 (m, 12H), 1.05-1.67 (m, 3H), 2.00-2.20 (m, 1H), 2.70 (dd, J = 9.14 Hz, 1H), 2.83 (dd, J = 6.14 Hz, 1H), 3.61 (d, J = 5 Hz, 1H), 3.72 (dd, J = 7.12 Hz, 1H), 3.87 (dd, J = 5.12 Hz, 1H), 3.94-4.08 (m, 1H), 4.38-4.52 (m, 1H), 6.66 (d, J = 9 Hz, 2H), 7.05 (d, J = 9 Hz, 2H), 8.03 (d, J = 9 Hz, 1H), 8.14 (bs, 3H), 8.65 (d, J = 9 Hz, 1H), 9.27 (s, 1H)
MASS (m/e):
   380 (M⁺+1)

### EXAMPLE 45

### Production of N'-(L-valyl)-N-(2-hydroxyethyl)-L-tyrosinamide hydrochloride

The title compound was obtained in the same manner as in Example 42 (3) and (4) but starting with N-[N-(tert-butoxycarbonyl)-L-valyl]-L-tyrosine and ethanolamine.
¹H-NMR (DMSO-d₆, 200 MHz) δ (ppm);
   0.86-0.95 (m, 6H), 2.00-2.20 (m, 1H), 2.73 (dd, J = 9.14 Hz, 1H), 2.87 (dd, J = 5.14 Hz, 1H), 3.09 (q, J = 6 Hz, 2H), 3.32 (t, J = 6 Hz, 2H), 3.62 (t, J = 6 Hz, 1H), 3.93 (t, J = 6 Hz, 1H), 4.37-4.50 (m, 1H), 6.67 (d, J = 9 Hz, 2H), 7.05 (d, J = 9 Hz, 2H), 8.04-8.20 (m, 4H), 8.58 (d, J = 7 Hz, 1H)
MASS (m/e):
   324 (M⁺+1)

### EXAMPLE 46

### Production of N'-(L-valyl)-N-[(S)-1-(hydroxymethyl)-2-phenylethyl]-L-tyrosinamide hydrochloride

The title compound was obtained in the same manner as in Example 42 (3) and (4) but starting with N-[N-(tert-butoxycarbonyl)-L-valyl]-L-tyrosine and (S)-(+)-phenylalaninol.
¹H-NMR (DMSO-d₆, 200 MHz) δ (ppm);
   0.83-0.90 (m, 6H), 2.00-2.16 (m, 1H), 2.66-2.86 (m, 4H), 3.53-3.67 (m, 1H), 3.79 (dd, J = 7.12 Hz, 1H), 3.92 (dd, J = 5.12 Hz, 1H), 4.07-4.20 (m, 1H), 4.38-4.50 (m, 1H), 6.65 (d, J = 8 Hz, 2H), 7.02 (d, J = 8 Hz, 2H), 7.15-7.35 (m, 5H), 8.14 (bs, 3H), 8.25 (d, J = 8 Hz, 1H), 8.63 (d, J = 8 Hz, 1H), 9.27 (s, 1H)
MASS (m/e):
   414 (M⁺+1)

### EXAMPLE 47

### Production of L-valyl-L-tyrosyl-L-isoleucine benzyl ester hydrochloride

The title compound was obtained in the same manner as in Example 42 (3) and (4) but starting with N-[N-(tert-butoxycarbonyl)-L-valyl]-L-tyrosine and L-isoleucine benzyl ester p-toluenesulfonate.
¹H-NMR (DMSO-d₆, 200 MHz) δ (ppm);
   0.74-0.93 (m, 12H), 1.07-1.44 (m, 2H), 1.73-1.90 (m, 1H), 2.00-2.20 (m, 1H), 2.67 (dd, J = 9.14 Hz, 1H), 2.84 (dd, J = 5.14 Hz, 1H), 3.59 (d, J = 5 Hz, 1H), 4.27 (t, J = 8 Hz, 1H), 4.56-4.70 (m, 1H), 5.12 (s, 2H), 6.67 (d, J = 9 Hz, 2H), 7.07 (d, J = 9 Hz, 2H), 7.30-7.40 (m, 5H), 8.08 (bs, 3H), 8.46 (d, J = 8 Hz, 1H), 8.58 (d, J = 8 Hz, 1H), 9.28 (s, 1H)

### EXAMPLE 48

### Production of N'-(L-valyl)-N-(2,4-dimethyl-3-pentyl)-L-tyrosinamide hydrochloride

The title compound was obtained in the same manner as in Example 42 (3) and (4) but starting with N-[N-(tert-butoxycarbonyl)-L-valyl]-L-tyrosine and 3-amino-2,4-dimethylpentane.
¹H-NMR (DMSO-d₆, 200 MHz) δ (ppm);
   0.63-0.95 (m, 18H), 1.60-1.84 (m, 2H), 2.02-2.20 (m, 1H), 3.26-3.46 (m, 1H), 3.55-3.67 (m, 1H), 4.56-4.70 (m, 1H), 6.67 (d, J = 9 Hz, 2H), 7.10 (d, J = 9 Hz, 2H), 7.65 (d, J = 8 Hz, 1H), 8.13 (bs, 3H), 8.65 (d, J = 8 Hz, 1H)

### EXAMPLE 49

### Production of N'-(L-valyl)-N-(2,2-diphenylethyl)-L-tyrosinamide hydrochloride

The title compound was obtained in the same manner as in Example 42 (3) and (4) but starting with N-[N-(tert-butoxycarbonyl)-L-valyl]-L-tyrosine and 2,2-diphenylethylamine.
¹H-NMR (DMSO-d₆, 200 MHz) δ (ppm);
   0.84-0.92 (m, 6H), 1.97-2.13 (m, 1H), 2.56 (d, J = 8 Hz, 2H), 3.55-3.82 (m, 3H), 4.14 (t, J = 8 Hz, 1H), 4.37 (q, J = 8 Hz, 1H), 6.63 (d, J = 9 Hz, 2H), 6.93 (d, J = 9 Hz, 2H), 7.12-7.32 (m, 10H), 8.10 (bs, 3H), 8.22 (t, J = 6 Hz, 1H), 8.52 (d, J = 8 Hz, 1H)

### EXAMPLE 50

### Production of N'-(L-norleucyl)-N-[(S)-1-(hydroxymethyl)-3-methylbutyl]-L-tyrosinamide hydrochloride

(1) To a solution of (S)-(+)-leucinol (0.23 g, 2.0 mmol) in N,N-dimethylformamide (20 ml) was added N-(tert-butoxycarbonyl)tyrosine-N-hydroxysuccinimide ester (0.76 g, 2.0 mmol) and the obtained mixture was stirred at room temperature for 3 hours. Then the reaction mixture was poured into water and extracted with ethyl acetate and dried. After distilling off solvent under reduced pressure, the obtained residue was purified by silica gel column chromatography [eluent: methanol/chloroform (1 : 20)] to thereby give N'-(tert-butoxycarbonyl)-N-[(S)-1-(hydroxymethyl)-3-methylbutyl]-L-tyrosinamide (0.54 g).
   ¹H-NMR (DMSO-d₆, 200 MHz) δ (ppm);
      0.78-0.90 (m, 6H), 1.23-1.37 (m, 2H), 1.32 (s, 9H), 1.50-1.70 (m, 1H), 2.61 (dd, J = 9.14 Hz, 1H), 2.79 (dd, J = 5.14 Hz, 1H), 3.07-3.35 (m, 2H), 3.70-3.84 (m, 1H), 3.95-4.10 (m, 1H), 4.58 (t, J = 5 Hz, 1H), 6.63 (d, J = 8 Hz, 2H), 6.76 (d, J = 8 Hz, 1H), 7.01 (d, J = 8 Hz, 2H), 7.41 (d, J = 8 Hz, 1H), 9.14 (s, 1H)
(2) A solution of N'-(tert-butoxycarbonyl)-N-[(S)-1-(hydroxymethyl)-3-methylbutyl]-L-tyrosinamide (0.43 g, 1.1 mmol) in a 4 N solution of hydrochloric acid/ethyl acetate (6 ml) was stirred for 1 hour. After distilling off the solvent under reduced pressure, the residue was dissolved in N,N-dimethylformamide (20 ml). Then triethylamine (0.17 g, 1.7 mmol) and N-(tert-butoxycarbonyl)-L-norleucine-N-hydroxysuccinimide ester (0.37 g, 1.1 mmol) were added thereto and the obtained mixture was stirred for 2 hours. The reaction mixture was poured into water and extracted with ethyl acetate. Then the organic layer was washed with diluted hydrochloric acid and dried. After distilling off solvent under reduced pressure, the obtained residue was purified by silica gel column chromatography [eluent: methanol/chloroform (1 : 20)] to thereby give N'-[N-(tert-butoxycarbonyl)-L-norleucyl]-N-[(S)-1-(hydroxymethyl)-3-methylbutyl]-L-tyrosinamide (0.48 g).
(3) A solution of N'-[N-(tert-butoxycarbonyl)-L-norleucyl]-N-[(S)-1-(hydroxymethyl)-3-methylbutyl]-L-tyrosinamide (0.48 g, 0.97 mmol) in a 4 N solution of hydrochloric acid/ethyl acetate (6 ml) was stirred for 2 hours. Then a saturated aqueous solution of sodium hydrogencarbonate (20 ml) was added to the reaction mixture followed by extraction with ethyl acetate. After drying and distilling off the solvent under reduced pressure, the title compound (0.13 g) was obtained.
   ¹H-NMR (DMSO-d₆, 200 MHz) δ (ppm);
      0.76-0.88 (m, 9H), 1.00-1.62 (m, 9H), 2.68 (dd, J = 8, 14 Hz, 1H), 2.83 (dd, J = 6.14 Hz, 1H), 3.03-3.46 (m, 5H), 3.65-3.80 (m, 1H), 4.34-4.47 (m, 1H), 6.61 (d, J = 8 Hz, 2H), 6.97 (d, J = 8 Hz, 2H), 7.58 (d, J = 8 Hz, 1H), 7.96 (d, J = 8 Hz, 1H), 9.14 (m, 1H)

### EXAMPLE 51

### Production of N'-(L-valyl)-N-[(1,1'-biphenyl)-4-yl-methyl]-L-tyrosinamide

(1) Under an argon gas stream, N-[N-(benzyloxycarbonyl)-L-valyl]-L-tyrosine (116 mg, 0.30 mmol) and 1-hydroxybenzotriazole monohydrate (50 mg, 0.33 mmol) were dissolved in tetrahydrofuran (3.0 ml) and N,N'-dicyclohexylcarbodiimide (68 mg, 0.33 mmol) was added thereto. The obtained mixture was stirred at room temperature for 1 hour and then 4-aminomethylbiphenyl (55 mg, 0.33 mmol) was added thereto. The mixture was stirred for 18 hours. After the reaction, the insoluble matters were filtered off and the solvent was distilled off under reduced pressure. The residue was extracted with chloroform, washed successively with 1 N hydrochloric acid, a saturated aqueous solution of sodium hydrogencarbonate, water and a saturated aqueous solution of sodium chloride and dried over anhydrous sodium sulfate. After distilling off the solvent under reduced pressure, N'-[N-(benzyloxycabronyl)-L-valyl]-N-[(1,1'-biphenyl)-4-yl-methyl]-L-tyrosinamide (76 mg) was obtained.
   ¹H-NMR (DMSO-d₆, 400 MHz) δ (ppm);
      0.76 (d, J = 6.8 Hz, 6H), 1.91 (m, 1H), 2.76 (dd, J = 8.2, 13.5 Hz, 1H), 2.88 (dd, J = 5.7, 13.5 Hz, 1H), 3.86 (dd, J = 7.0, 8.9 Hz, 1H), 4.24 (dd, J = 5.7, 15.3 Hz, 1H), 4.31 (dd, J = 6.0, 15.3 Hz, 1H), 4.53 (m, 1H), 4.97 (d, J = 12.5 Hz, 1H), 5.05 (d, J = 12.5 Hz, 1H), 6.64 (d, J = 8.3 Hz, 2H), 7.01 (d, J = 8.3 Hz, 2H), 7.16 (d, J = 8.5 Hz, 2H), 7.27 (d, J = 8.9 Hz, 1H), 7.28-7.40 (m, 6H), 7.42-7.49 (m, 2H), 7.55 (d, J = 8.1 Hz, 2H), 7.63 (d, J = 8.5 Hz, 1H), 7.63 (d, J = 7.3 Hz, 1H), 7.99 (d, J = 8.2 Hz, 1H), 8.42 (dd, J = 5.7, 6.0 Hz, 1H), 9.20 (s, 1H)
(2) Under a hydrogen gas stream, N'-[N-(benzyloxycabronyl)-L-valyl]-N-[(1,1'-biphenyl)-4-yl-methyl]-L-tyrosinamide (70 mg, 0.12 mmol) was dissolved in a mixture of methanol (2.0 ml) and tetrahydrofuran (3.0 ml). After adding 10% palladium-carbon (30 mg), the mixture was stirred at room temperature until the starting compounds disappeared. The solid matters were filtered off through celite and the solvent was distilled off under reduced pressure to thereby give the title compound (51 mg).
   ¹H-NMR (DMSO-d₆, 400 MHz) δ (ppm);
      0.89 (d, J = 6.9 Hz, 3H), 0.92 (d, J = 6.9 Hz, 3H), 2.09 (m, 1H), 2.78 (dd, J = 7.9, 13.6 Hz, 1H), 2.90 (dd, J = 6.8, 13.6 Hz, 1H), 3.61 (d, J = 5.4 Hz, 1H), 4.22-4.34 (m, 2H), 4.56 (m, 1H), 6.69 (d, J = 8.5 Hz, 2H), 7.06 (d, J = 8.5 Hz, 2H), 7.16 (d, J = 8.2 Hz, 2H), 7.36 (dd, J = 7.4, 7.4 Hz, 1H), 7.42-7.50 (m, 2H).7.55 (d, J = 8.2 Hz, 2H), 7.64 (d, J = 7.2 Hz, 1H), 7.64 (d, J = 8.5 Hz, 1H), 8.03 (br, 3H), 8.63 (t, J = 6.0 Hz, 1H), 8.64 (d, J = 8.3 Hz, 1H), 9.29 (s, 1H)
   MASS (m/e):
      445 (M⁺)

### EXAMPLE 52

### Production of N'-(L-valyl)-N-(4-heptyl)-L-tyrosinamide

The title compound was obtained in the same manner as in Example 51 (1) and (2) but starting with N-[N-(benzyloxcarbonyl)-L-valyl]-L-tyrosine and 4-aminoheptane.
¹H-NMR (DMSO-d₆, 400 MHz) δ (ppm);
   0.74 (t, J = 7.4 Hz, 3H), 0.79 (d, J = 6.9 Hz, 3H), 0.80 (t, J = 7.4 Hz, 3H), 0.87 (d, J = 6.8 Hz, 3H), 0.94-1.37 (m, 8H), 1.98 (m, 1H), 2.69 (dd, J = 7.7, 13.5 Hz, 1H), 2.80 (dd, J = 6.9, 13.5 Hz, 1H), 3.34 (d, J = 5.1 Hz, 1H), 3.64 (m, 1H), 4.47 (m, 1H), 6.63 (d, J = 8.5 Hz, 2H), 7.00 (d, J = 8.5 Hz, 2H), 7.65 (d, J = 8.8 Hz, 1H), 8.34 (d, J = 8.5 Hz, 1H), 9.40 (br, 1H)
MASS (m/e):
   377 (M⁺)

### EXAMPLE 53

### Production of N'-(L-valyl)-N-[(S)-1-phenylethyl]-L-tyrosinamide

The title compound was obtained in the same manner as in Example 51 (1) and (2) but starting with N-[N-(benzyloxycarbonyl)-L-valyl]-L-tyrosine and (S)-1-phenylethylamine.
¹H-NMR (DMSO-d₆, 400 MHz) δ (ppm);
   0.78 (d, J = 6.9 Hz, 3H), 0.97 (d, J = 6.9 Hz, 3H), 1.32 (d, J = 7.0 Hz, 3H), 2.00 (m, 1H), 2.69 (dd, J = 7.9, 13.8 Hz, 1H), 2.81 (dd, J = 6.4, 13.8 Hz, 1H), 2.81 (d, J = 6.4 Hz, 1H), 3.32 (m, 1H), 4.56 (m, 1H), 4.87 (m, 1H), 5.80 (br, 2H), 6.61 (d, J = 8.5 Hz, 2H), 6.96 (d, J = 8.5 Hz, 2H), 7.01-7.34 (m, 5H), 8.31 (d, J = 8.1 Hz, 1H), 8.47 (d, J = 8.0 Hz, 1H), 9.22 (s, 1H)
MASS (m/e):
   384 (M⁺+1)

### EXAMPLE 54

### Production of N'-(L-valyl)-N-[(R)-1-phenylethyl]-L-tyrosinamide

The title compound was obtained in the same manner as in Example 51 (1) and (2) but starting with N-[N-(benzyloxycarbonyl)-L-valyl]-L-tyrosine and (R)-1-phenylethylamine.
¹H-NMR (DMSO-d₆, 400 MHz) δ (ppm);
   0.64 (d, J = 6.8 Hz, 3H), 0.78 (d, J = 6.9 Hz, 3H), 1.23 (d, J = 7.0 Hz, 3H), 1.78-1.94 (m, 3H), 2.74 (dd, J = 8.0, 13.6 Hz, 1H), 2.81 (dd, J = 6.3, 13.6 Hz, 1H), 2.94 (d, J = 4.7 Hz, 1H), 4.52 (m, 1H), 4.84 (m, 1H), 6.64 (d, J = 8.5 Hz, 2H), 6.95 (d, J = 8.5 Hz, 2H), 7.10-7.32 (m, 5H), 7.99 (d, J = 8.4 Hz, 1H), 8.30 (d, J = 8.1 Hz, 1H), 9.16 (s, 1H)
MASS (m/e):
   383 (M⁺)

### EXAMPLE 55

### Production of (2S,3S)-2-[[N-(L-valyl)-L-tyrosyl]amino]-3-methylpentanal-O-benzyloxime hydrochloride

(1) Into a solution of pyridine/sulfur trioxide complex (1.5 g, 9.7 mmol) in dimethyl sulfoxide (3.0 ml) and dichloromethane (3.0 ml) was dropped under an argon gas stream a solution of (S)-N-(tert-butoxycarbonyl)-isoleucinol (0.42 g, 1.9 mmol) and triethylamine (0.98 g, 9.7 mmol) in dimethyl sulfoxide (3.0 ml) and dichloromethane (3.0 ml) under ice-cooling. After stirring for 30 minutes, the reaction mixture was poured into water and extracted with dichloromethane. The organic layer was washed successively with a saturated aqueous solution of citric acid, an aqueous solution of sodium hydrogencarbonate, water and an aqueous solution of sodium chloride and dried over anhydrous sodium sulfate. After distilling off the solvent under reduced pressure, (2S,3S)-2-(tert-butoxycarbonylamino)-3-methylpentanal (340 mg) was obtained.
   ¹H-NMR (CDCl₃, 200 MHz) δ (ppm);
      0.95 (t, J = 7.9 Hz, 3H), 0.96 (d, J = 6.9 Hz, 3H), 1.12-1.60 (m, 2H), 1.44 (s, 9H), 2.13 (m, 1H), 4.28 (m, 1H), 5.14 (m, 1H), 9.65 (s, 1H)
(2) To a solution of O-benzylhydroxylamine (167 mg, 0.78 mmol) in tetrahydrofuran (5.0 ml) was added an aqueous solution of sodium hydrogencarbonate until the pH value reached 6. To the obtained solution was added (2S,3S)-2-(tert-butoxycarbonylamino)-3-methylpentanal (128 mg, 0.80 mmol) at room temperature and the mixture was stirred for 8 hours. Then it was concentrated under reduced pressure and the organic matters were extracted with ethyl acetate, washed with water and an aqueous solution of sodium chloride and dried over anhydrous sodium sulfate. After concentration under reduced pressure, the residue was purified by column chromatography to thereby give (2S,3S)-2-(tert-butoxycarbonylamino)-3-methylpentanal-O-benzyloxime (163 mg).
   ¹H-NMR (CDCl₃, 200 MHz) δ (ppm);
      0.85 (d, J = 6.9 Hz, 3H), 0.92 (t, J = 7.4 Hz, 3H), 1.14 (m, 1H), 1.43 (m, 1H), 1.44 (s, 9H), 1.80 (m, 1H), 4.27 (m, 1H), 5.03 (m, 1H), 5.09 (s, 2H), 7.23-7.48 (m, 5H), 7.43 (d, J = 4.3 Hz, 1H)
(3) Under an argon gas stream, (2S,3S)-2-(tert-butoxycarbonylamino)-3-methylpentanal-O-benzyloxime (100 mg) was dissolved in ethyl acetate (1.5 ml) and a 4 N solution of hydrochloric acid/ethyl acetate was added thereto under ice-cooling. After stirring the mixture at room temperature for 2 hours, the solvent was distilled off under reduced pressure to thereby give (2S,3S)-2-amino-3-methylpentanal-O-benzyloxime hydrochloride (74 mg).
   ¹H-NMR (CDCl₃, 200 MHz) δ (ppm);
      0.92 (d, J = 6.8 Hz, 3H), 1.02 (d, J = 6.0 Hz, 3H), 1.37 (m, 1H), 1.50 (m, 1H), 2.10 (m, 1H), 3.97 (m, 1H), 5.13 (s, 2H), 7.20-7.40 (m, 5H), 7.47 (d, J = 4.3 Hz, 1H), 8.70 (br, 3H)
(4) (2S,3S)-2-Amino-3-methylpentanal-O-benzyloxime hydrochloride was neutralized with triethylamine and then allowed to react with N-[N-(tert-butoxycarbonyl)-L-valyl]-L-tyrosine in the same manner as in Example 51 (1) to thereby give (2S,3S)-2-[[[N-(tert-butoxycarbonyl)-L-valyl]-L-tyrosyl]amino]-3-methylpentanal-O-benzyloxime.
   ¹H-NMR (DMSO-d₆, 200 MHz) δ (ppm);
      0.65-0.93 (m, 12H), 0.93-1.98 (m, 4H), 1.37 (s, 9H), 2.57-2.90 (m, 2H), 3.73 (m, 1H), 4.18 (m, 1H), 4.46 (m, 1H), 5.01 (s, 2H), 6.60 (d, J = 8.4 Hz, 2H), 6.65 (m, 1H), 6.97 (d, J = 8.4 Hz, 2H), 7.25 (d, J = 7.1 Hz, 1H), 7.25-7.50 (m, 5H), 7.85 (m, 1H), 8.06 (m, 1H), 9.13 (s, 1H)
(5) From (2S,3S)-2-[[[N-(tert-butoxycarbonyl)-L-valyl]-L-tyrosyl]amino]-3-methylpentanal-O-benzyloxime, the title compound was obtained in the same manner as in Example 55 (3).
   ¹H-NMR (DMSO-d₆, 400 MHz) δ (ppm);
      0.76 (d, J = 6.9 Hz, 3H), 0.77 (t, J = 7.3 Hz, 3H), 0.88 (d, J = 6.9 Hz, 3H), 0.91 (d, J = 6.9 Hz, 3H), 1.04 (m, 1H), 1.36 (m, 1H), 1.60 (m, 1H), 2.09 (m, 1H), 2.70 (dd, J = 8.2, 13.8 Hz, 1H), 2.83 (dd, J = 6.1, 13.8 Hz, 1H), 3.60 (m, 1H), 4.18 (m, 1H), 4.48 (m, 1H), 5.02 (s, 2H), 6.66 (d, J = 8.5 Hz, 2H), 7.03 (d, J = 8.5 Hz, 2H), 7.25 (d, J = 7.2 Hz, 1H), 7.26-7.40 (m, 5H), 8.07 (br, 3H), 8.29 (d, J = 10.5 Hz, 1H), 8.62 (d, J = 8.0 Hz, 1H), 9.24 (s, 1H)
   MASS (m/e):
      482 (M⁺)

### EXAMPLE 56

### Production of (2S,3S)-2-[[N-(L-valyl)-L-tyrosyl]amino]-3-methylpentanal-O-undecyloxime hydrochloride

(1) (2S,3S)-2-(tert-Butoxycarbonylamino)-3-methylpentanal-O-undecyloxime was obtained from (2S,3S)-2-(tert-butoxycarbonylamino)-3-methylpentanal and O-undecylhydroxylamine in the same manner as in Example 55 (1).
   ¹H-NMR (CDCl₃, 200 MHz) δ (ppm);
      0.87 (d, J = 8.9 Hz, 3H), 0.88 (t, J = 6.6 Hz, 3H), 0.93 (t, J = 7.2 Hz, 3H), 1.17-1.90 (m, 21H), 1.43 (s, 9H), 4.02 (t, J = 6.7 Hz, 2H), 4.23 (m, 1H), 5.07 (m, 1H), 7.33 (d, J = 4.3 Hz, 1H)
(2) The title compound was obtained in the same manner as in Example 55 (3), (4) and (5) from (2S,3S)-2-(tert-butoxycarbonylamino)-3-methylpentanal-O-undecyloxime and N-[N-(tert-butoxycarbonyl)-L-valyl]-L-tyrosine.
   ¹H-NMR (DMSO-d₆, 400 MHz) δ (ppm);
      0.68-1.30 (m, 25H), 0.83 (t, J = 7.4 Hz, 3H), 0.88 (d, J = 6.9 Hz, 3H), 0.93 (d, J = 6.9 Hz, 3H), 1.37 (m, 1H), 1.62 (m, 1H), 2.11 (m, 1H), 2.73 (dd, J = 8.6, 13.8 Hz, 1H), 2.88 (dd, J = 5.5, 13.8 Hz, 1H), 3.54-3.70 (m, 3H), 3.99 (m, 1H), 4.43 (m, 1H), 6.68 (d, J = 8.5 Hz, 2H), 7.06 (d, J = 8.5 Hz, 2H), 7.08 (m, 1H), 7.94-8.22 (m, 4H), 8.52 (d, J = 8.2 Hz, 1H), 9.23 (s, 1H)
   MASS (m/e):
      547 (M⁺+1)

### EXAMPLE 57

### Production of (2S, 3S)-2-[[N-(L-valyl)-L-tyrosyl]amino]-3-methylpentanal-O-2-propyloxime hydrochloride

(1) (2S,3S)-2-(tert-Butoxycarbonylamino)-3-methylpentanal-O-2-propyloxime was obtained from (2S,3S)-2-(tert-butoxycarbonylamino)-3-methylpentanal and O-2-propylhydroxylamine in the same manner as in Example 55 (1).
   ¹H-NMR (CDCl₃, 200 MHz) δ (ppm);
      0.87 (d, J = 6.8 Hz, 3H), 0.93 (t, J = 7.9 Hz, 3H), 1.03-1.90 (m, 3H), 1.20 (d, J = 6.2 Hz, 6H), 1.44 (s, 9H), 4.25 (m, 1H), 4.33 (m, 1H), 5.06 (m, 1H), 7.31 (d, J = 4.3 Hz, 1H)
(2) The title compound was obtained in the same manner as in Example 55 (3), (4) and (5) from (2S,3S)-2-(tert-butoxycarbonylamino) -3-methylpentanal-O-2-propyloxime and N-[N-(tert-butoxycarbonyl)-L-valyl]-L-tyrosine.
   ¹H-NMR (DMSO-d₆, 400 MHz) δ (ppm);
      0.79 (d, J = 6.7 Hz, 3H), 0.80 (t, J = 7.4 Hz, 3H), 0.88 (d, J = 6.9 Hz, 3H), 0.92 (d, J = 6.9 Hz, 3H), 1.06 (m, 1H), 1.15 (d, J = 6.2 Hz, 3H), 1.16 (d, J = 6.2 Hz, 3H), 1.38 (m, 1H), 1.60 (m, 1H), 2.09 (m, 1H), 2.71 (dd, J = 8.2, 13.9 Hz, 1H), 2.85 (dd, J = 6.1, 13.9 Hz, 1H), 3.61 (m, 1H), 4.16-4.27 (m, 2H), 4.48 (m, 1H), 6.65 (d, J = 8.5 Hz, 2H), 7.02 (d, J = 8.5 Hz, 2H), 7.14 (d, J = 7.2 Hz, 1H), 8.09 (br, 3H), 8.28 (d, J = 8.7 Hz, 1H), 8.63 (d, J = 8.1 Hz, 1H), 9.24 (s, 1H)
   MASS (m/e):
      434 (M⁺)

### EXAMPLE 58

### Production of (3S,4S)-N'-(L-valyl)-N-[(E)-4-methyl-1-phenyl-1-hexen-3-yl]-L-tyrosinamide hydrochloride

(1) Into a suspension of benzyltriphenylphosphonium bromide (2.6 g, 6.0 mmol) in ether (30 ml) was dropped under an argon gas stream n-butyllithium (n-hexane solution, 1.7 M, 3.5 ml) and the obtained mixture was stirred for 3 hours. After adding a solution of (2S,3S)-2-(tert-butoxycarbonylamino)-3-methylpentanal (0.65 g, 3.0 mmol) in ether (6.0 ml), the reaction mixture was stirred for 19 hours. Then the reaction mixture was poured into ice/water, extracted with ethyl acetate and washed with water and an aqueous solution of sodium chloride. Then it was concentrated under reduced pressure and purified by column chromatography to thereby give (3S,4S)-(E)-(3-tert-butoxycarbonylamino)-4-methyl-1-phenyl-1-hexene (0.68 g).
   ¹H-NMR (CDCl₃, 200 MHz) δ (ppm);
      0.92 (d, J = 6.8 Hz, 3H), 0.94 (t, J = 7.9 Hz, 3H), 1.15 (m, 1H), 1.45 (s, 9H), 1.51 (m, 1H), 1.62 (m, 1H), 4.24 (m, 1H), 4.63 (m, 1H), 6.07 (dd, J = 6.7, 15.7 Hz, 1H), 6.50 (d, J = 15.7 Hz, 1H), 7.19-7.40 (m, 5H)
(2) From N-[N-(tert-butoxycarbonyl)-L-valyl]-L-tyrosine and (3S,4S)-(E)-(3-tert-butoxycarbonylamino)-4-methyl-1-phenyl-1-hexene, (3S,4S)-N'-[N-(tert-butoxycarbonyl)-L-valyl]-N-[(E)-4-methyl-1-phenyl-1-hexen-3-yl]-L-tyrosinamide in the same manner as in Example 55 (3) and Example 51 (1).
   ¹H-NMR (CDCl₃, CD₃OD, 400 MHz) δ (ppm);
      0.84 (d, J = 6.7 Hz, 3H), 0.85 (d, J = 6.7 Hz, 3H), 0.87 (t, J = 7.3 Hz, 3H), 0.92 (d, J = 6.8 Hz, 3H), 1.20-2.12 (m, 4H), 1.44 (s, 9H), 2.94 (d, J = 7.1 Hz, 2H), 3.91 (m, 1H), 4.40 (m, 1H), 4.53 (t, J = 7.1 Hz, 1H), 5.91 (dd, J = 7.0, 15.9 Hz, 1H), 6.28 (dd, J = 1.0, 15.9 Hz, 1H), 6.66 (d, J = 8.5 Hz, 2H), 7.01 (d, J = 8.5 Hz, 2H), 7.19-7.34 (m, 5H)
(3) The title compound was obtained from (3S,4S)-N'-[N-(tert-butoxycarbonyl)-L-valyl]-N-[(E)-4-methyl-1-phenyl-1-hexen-3-yl]-L-tyrosinamide in the same manner as in Example 55 (3).
   ¹H-NMR (CDCl₃, DMSO-d₆, 400 MHz) δ (ppm);
      0.89 (t, J = 7.3 Hz, 3H), 0.90 (d, J = 6.7 Hz, 3H), 0.95 (d, J = 6.6 Hz, 3H), 0.97 (d, J = 6.5 Hz, 3H), 1.17 (m, 1H), 1.51 (m, 1H), 1.66 (m, 1H), 2.31 (m, 1H), 2.95 (dd, J = 8.5, 13.7 Hz, 1H), 3.05 (dd, J = 5.8, 13.7 Hz, 1H), 3.68 (m, 1H), 4.42 (m, 1H), 4.64 (m, 1H), 6.09 (dd, J = 7.2, 15.9 Hz, 1H), 6.38 (d, J = 15.9 Hz, 1H), 6.70 (d, J = 8.2 Hz, 2H), 7.11 (d, J = 8.2 Hz, 2H), 7.17-7.38 (m, 5H), 7.61 (d, J = 8.6 Hz, 1H), 8.25 (bs, 3H), 8.80 (d, J = 8.3 Hz, 1H), 8.87 (bs, 1H)
   MASS (m/e):
      451 (M⁺)

### EXAMPLE 59

### Production of (3S,4S)-N'-(L-valyl)-N-(4-methyl-1-phenylhexan-3-yl)-L-tyrosinamide hydrochloride

(1) (3S,4S)-N'-[N-(tert-Butoxycarbonyl)-L-valyl]-N-[(E)-4-methyl-1-phenyl-1-hexen-3-yl)-L-tyrosinamide was hydrogenated in the same manner as in Example 51 (2) to thereby give (3S,4S)-N'-[N-(tert-butoxycarbonyl)-L-valyl]-N-(4-methyl-1-phenylhexan-3-yl)-L-tyrosinamide.
   ¹H-NMR (CDCl₃, CD₃OD, 200 MHz) δ (ppm);
      0.72 (d, J = 6.6 Hz, 3H), 0.77 (t, J = 6.9 Hz, 3H), 0.79 (d, J = 7.3 Hz, 3H), 0.87 (d, J = 6.8 Hz, 3H), 0.98 (m, 1H), 1.13-1.52 (m, 3H), 1.38 (s, 9H), 1.64 (m, 1H), 2.05 (m, 1H), 2.20-2.43 (m, 2H), 2.93 (d, J = 7.0 Hz, 2H), 3.74 (m, 1H), 3.85 (m, 1H), 4.53 (m, 1H), 5.13 (d, J = 7.6 Hz, 1H), 6.40 (d, J = 9.1 Hz, 1H), 6.67 (d, J = 8.3 Hz, 2H), 7.00 (d, J = 8.3 Hz, 2H), 7.00-7.27 (m, 5H)
(2) The title compound was obtained from (3S,4S)-N'-[N-(tert-butoxycarbonyl)-L-valyl]-N-(4-methyl-1-phenylhexan-3-yl)-L-tyrosinamide in the same manner as in Example 55 (3).
   ¹H-NMR (CDCl₃, DMSO-d₆, 400 MHz) δ (ppm);
      0.82 (d, J = 6.4 Hz, 3H), 0.83 (t, J = 6.6 Hz, 3H), 0.97 (d, J = 6.8 Hz, 3H), 0.99 (d, J = 6.7 Hz, 3H), 1.09 (m, 1H), 1.39 (m, 1H), 1.50 (m, 1H), 1.59 (m, 1H), 1.69 (m, 1H), 2.25-2.42 (m, 2H), 2.48 (m, 1H), 2.94 (dd, J = 8.2, 13.8 Hz, 1H), 3.04 (m, 1H), 3.70 (d, J = 5.3 Hz, 1H), 3.75 (m, 1H), 4.64 (m, 1H), 6.74 (d, J = 8.4 Hz, 2H), 7.09-7.29 (m, 8H), 8.20 (br, 3H), 8.75 (d, J = 8.5 Hz, 1H), 8.85 (s, 1H)
   MASS (m/e):
      453 (M⁺)

### EXAMPLE 60

### Production of (3S,4S)-N'-(L-norleucyl)-N-[(E)-4-methyl-1-(4-methylphenyl)-1-hexen-3-yl]-L-tyrosinamide hydrochloride

(1) Under an argon gas stream, N-(tert-butoxycarbonyl)-L-norleucine (1.7 g, 7.4 mmol) and 1-hydroxybenzotriazole monohydrate (1.2 g, 8.1 mmol) were dissolved in tetrahydrofuran (60 ml), and N,N'-dicyclohexylcarbodiimide (1.8 g, 8.1 mmol) was added thereto. After stirring at room temperature for 1 hour, a suspension of tyrosine methyl ester hydrochloride (1.7 g, 7.4 mmol) and triethylamine (1.5 g, 15 mmol) in tetrahydrofuran (10 ml) was added to the reaction mixture and the obtained mixture was stirred for 15 hours. After the reaction, the insoluble matters were filtered off and the solvent was distilled off under reduced pressure. The residue thus obtained was extracted with ethyl acetate and the organic layer was washed with 1 N hydrochloric acid, a saturated aqueous solution of sodium hydrogencarbonate, water and a saturated aqueous solution of sodium chloride and dried over anhydrous sodium sulfate. Then the solvent was distilled off under reduced pressure to thereby give N-[N-(tert-butoxycarbonyl)-L-norleucyl]-L-tyrosine methyl ester (2.8 g).
   ¹H-NMR (DMSO-d₆, 200 MHz) δ (ppm);
      0.84 (t, J = 6.5 Hz, 3H), 1.00-1.90 (m, 6H), 1.37 (s, 9H), 2.80 (dd, J = 8.2, 14.0 Hz, 1H), 2.90 (dd, J = 6.1, 14.0 Hz, 1H), 3.56 (s, 3H), 3.89 (m, 1H), 4.39 (m, 1H), 6.64 (d, J = 8.4 Hz, 2H), 6.76 (d, J = 8.4 Hz, 1H), 6.98 (d, J = 8.4 Hz, 2H), 8.06 (d, J = 7.6 Hz, 1H), 9.21 (s, 1H)
(2) N-[N-(tert-Butoxycarbonyl)-L-norleucyl]-L-tyrosine methyl ester (2.7 g, 6.9 mmol) was dissolved in methanol (69 ml). After adding a 2 N aqueous solution of sodium hydroxide (35 ml), the mixture was stirred at room temperature for 6 hours. After the reaction, 1 N hydrochloric acid was added and the solid matter thus formed was collected by filtration and dried to thereby give N-[N-(tert-butoxycarbonyl)-L-norleucyl]-L-tyrosine (1.6 g).
   ¹H-NMR (DMSO-d₆, 200 MHz) δ (ppm);
      0.83 (t, J = 6.3 Hz, 3H), 1.00-1.70 (m, 6H), 1.36 (s, 9H), 2.79 (dd, J = 6.4, 13.4 Hz, 1H), 2.93 (dd, J = 5.2, 13.4 Hz, 1H), 3.77 (m, 1H), 4.07 (m, 1H), 6.57 (d, J = 8.4 Hz, 2H), 6.91 (d, J = 8.4 Hz, 2H), 6.96 (d, J = 8.6 Hz, 1H), 7.49 (d, J = 6.9 Hz, 1H)
(3) (3S,4S)-(E)-3-Amino-4-methyl-1-(4-methylphenyl)-1-hexene hydrochloride was obtained from 4-methylbenzyltriphenylphosphonium chloride and (2S,3S)-2-(tert-butoxycarbonylamino)-3-methylpentanal in the same manner as in Example 58 (1) and Example 55 (3).
   ¹H-NMR (DMSO-d₆, 200 MHz) δ (ppm);
      0.89 (t, J = 7.4 Hz, 3H), 0.92 (d, J = 6.8 Hz, 3H), 1.15 (m, 1H), 1.42 (m, 1H), 1.85 (m, 1H), 2.29 (s, 3H), 3.75 (dd, J = 5.2, 8.2 Hz, 1H), 6.13 (dd, J = 8.2, 16.0 Hz, 1H), 6.68 (d, J = 16.0 Hz, 1H), 7.18 (d, J = 8.0 Hz, 2H), 7.34 (d, J = 8.0 Hz, 2H), 8.35 (br, 3H)
(4) The title compound was obtained from N-[N-(tert-butoxycarbonyl)-L-norleucyl]-L-tyrosine and (3S,4S)-(E)-3-amino-4-methyl-1-(4-methylphenyl)-1-hexene hydrochloride in the same manner as in Example 60 (1) and Example 55 (3).
   ¹H-NMR (DMSO-d₆, 400 MHz) δ (ppm);
      0.78-0.94 (m, 9H), 1.09 (m, 1H), 1.16-1.33 (m, 4H), 1.42 (m, 1H), 1.56 (m, 1H), 1.60-1.76 (m, 2H), 2.28 (s, 3H), 2.72 (dd, J = 8.1, 13.7 Hz, 1H), 2.87 (dd, J = 6.4, 13.7 Hz, 1H), 3.73 (t, J = 6.2 Hz, 1H), 4.31 (m, 1H), 4.59 (m, 1H), 6.06 (dd, J = 6.5, 15.9 Hz, 1H), 6.24 (d, J = 15.9 Hz, 1H), 6.63 (d, J = 8.5 Hz, 2H), 7.05 (d, J = 8.5 Hz, 2H), 7.13 (d, J = 8.1 Hz, 2H), 7.24 (d, J = 8.1 Hz, 2H), 8.04 (br, 3H), 8.12 (d, J = 8.8 Hz, 1H), 8.68 (d, J = 8.3 Hz, 1H), 9.21 (s, 1H)
   MASS (m/e):
      479 (M⁺)

### EXAMPLE 61

### Production of (3S,4S)-N'-(L-norleucyl)-N-[4-methyl-1-(4-methylphenyl)-1-hexan-3-yl]-L-tyrosinamide hydrochloride

The title compound was obtained by hydrogenating (3S,4S)-N'-(L-norleucyl)-N-[(E)-4-methyl-1-(4-methylphenyl)-1-hexen-3-yl]-L-tyrosinamide hydrochloride in the same manner as in Example 51 (2).
¹H-NMR (DMSO-d₆, 400 MHz) δ (ppm);
   0.75 (d, J = 7.8 Hz, 3H), 0.76 (t, J = 7.6 Hz, 3H), 0.84 (t, J = 6.6 Hz, 3H), 1.02 (m, 1H), 1.13-1.75 (m, 10H), 2.10-2.37 (m, 2H), 2.24 (s, 3H), 2.73 (dd, J = 7.8, 13.6 Hz, 1H), 2.90 (dd, J = 7.1, 13.6 Hz, 1H), 3.55 (m, 1H), 3.73 (t, J = 6.1 Hz, 1H), 4.58 (m, 1H), 6.66 (d, J = 8.4 Hz, 2H), 6.96 (d, J = 7.9 Hz, 2H), 7.05 (d, J = 7.9 Hz, 2H), 7.08 (d, J = 8.4 Hz, 2H), 7.90 (d, J = 9.0 Hz, 1H), 8.08 (br, 3H), 8.72 (d, J = 8.5 Hz, 1H), 9.12 (s, 1H)
MASS (m/e):
   481 (M⁺)

### TEST EXAMPLE 1

The capability of binding to angiotensin IV receptor was measured by using Hartley guinea pig hippocampus in accordance with the method reported by Miller-Wing *et al.*, *J. Pharmacol. Exp. Therp*., *266*:1718 (1993).

The Hartley guinea pig hippocampus was homogenized in a 50 mM Tris hydrochloride buffer solution (pH 7.4) containing 1 mM of ethylenediamine-N,N,N',N'-tetraacetic acid (EDTA) and 100 µm of phenylmethanesufonyl fluoride (PMSF). After centrifuging at 900 × g, the supernatant thus obtained was further centrifuged at 48,000 × g. The precipitate was washed once with the same buffer solution and then the precipitate was suspended in a 50 mM Tris hydrochloride buffer solution (pH 7.4) containing 150 mM of sodium chloride, 1 mM of EDTA, 100 µm of PMSF and 0.1% of bovine serum albumin (BSA) in such a manner as to give a protein concentration of 50 µg/ml to thereby give a crude membrane specimen.

This membrane specimen was allowed to react with 0.2 nM ¹²⁵I-angiotensin IV and a test compound dissolved in dimethyl sulfoxide at 37°C for 90 minutes. Then ¹²⁵I-angiotensin IV binding to the membrane was filtered under suction onto GF/B filer paper with the use of a cell harvester for laboratory use. The radioactivity on the filter paper was measured with a gamma-counter. The nonspecific bond was measured in the presence of 1 µM of angiotensin IV and the specific bond was calculated by subtracting the nonspecific bond from the total bond. The capability (IC₅₀) of the test compound of binding to angiotensin IV receptor was determined as the concentration of the test compound capable of inhibiting the binding at an extent of 50% from the inhibition curve obtained by reacting 0.2 nM ¹²⁵I-angiotensin IV with the test compound at various concentrations. Tables 1 and 2 show the results.

**TABLE 1**

| Test Compound (Ex. No.) | IC₅₀ (nM) | Test Compound (Ex. No.) | IC₅₀ (nM) |
|---|---|---|---|
| 1 | 38.30 | 22 | 97.70 |
| 2 | 10.00 | 23 | 42.29 |
| 3 | 0.93 | 24 | 0.49 |
| 4 | 1.23 | 25 | 0.28 |
| 5 | 3.76 | 26 | 1.23 |
| 6 | 2.60 | 27 | 1.96 |
| 7 | 5.99 | 28 | 0.64 |
| 8 | 1.79 | 29 | 1.23 |
| 9 | 5.99 | 30 | 97.70 |
| 10 | 29.15 | 31 | 26.58 |
| 11 | 29.15 | 32 | 11.50 |
| 12 | 12.62 | 33 | 12.62 |
| 13 | 12.62 | 34 | 911.16 |
| 14 | 12.62 | 35 | 7.92 |
| 15 | 26.56 | 36 | 1.35 |
| 16 | 4.13 | 37 | 4.53 |
| 17 | 31.99 | 38 | 327.5 |
| 18 | 0.40 | 39 | 4.53 |
| 19 | 0.28 | 40 | 327.5 |
| 20 | 129.15 | 41 | 271.9 |
| 21 | 73.91 | | |

**TABLE 2**

| Test Compound (Ex. No.) | IC₅₀ (nM) | Test Compound (Ex. No.) | IC₅₀ (nM) |
|---|---|---|---|
| 42 | 29.15 | 52 | 138.49 |
| 43 | 359.38 | 53 | 155.57 |
| 44 | 24.20 | 54 | 67.34 |
| 45 | 830.22 | 55 | 17.53 |
| 46 | 29.15 | 56 | 11.54 |
| 47 | 38.54 | 57 | 15.08 |
| 48 | 205.65 | 58 | 10.48 |
| 49 | 81.11 | 59 | 26.53 |
| 50 | 9.55 | 60 | 0.77 |
| 51 | 242.00 | 61 | 24.20 |

### INDUSTRIAL APPLICABILITY

Because of agonistically acting on angiotensin IV receptor, the novel peptide derivatives of the present invention are useful as remedies for various diseases in which angiotensin IV participates and exert various physiological actions upon, for example, acceleration of renal blood flow, cerebral vasodilation, inhibition of cell proliferation and hypermnesia.

## Claims

1. A peptide derivative represented by the following formula (1); 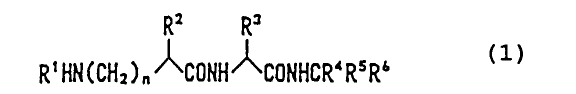 wherein
R¹ represents a hydrogen atom or a lower alkyl group;
R² represents a hydrogen atom or a substituted or unsubstituted lower alkyl, cycloalkyl, cycloalkyl-substituted alkyl, aralkyl or aryl group, or R¹ and R² may together form a substituted or unsubstituted alkylene group having 2 to 4 carbon atoms;
R³ represents a substituted or unsubstituted lower alkyl or aralkyl group;
R⁴ represents a hydrogen atom or a substituted or unsubstituted lower alkyl or aralkyl group;
R⁵ represents a hydrogen atom or a substituted or unsubstituted lower alkyl, alkenyl or aralkyl group;
R⁶ represents a hydrogen atom or a substituted or unsubstituted alkyl, alkenyl, cycloalkyl, aralkyl, aryl, N-alkoxyiminomethyl, N-aralkyloxyiminomethyl, aralkyloxycarbonyl or CONR⁷R⁸ group, in which R⁷ represents a hydrogen atom or a substituted or unsubstituted lower alkyl group; and R⁸ represents a substituted or unsubstituted alkyl, cycloalkyl, aralkyl or aryl group; and
n is an integer of from 0 to 3, or
a pharmaceutically acceptable salt thereof.

2. The peptide derivative or the pharmaceutically acceptable salt thereof according to claim 1, wherein R¹ is a hydrogen atom.

3. The peptide derivative or the pharmaceutically acceptable salt thereof according to claim 2, wherein n is 0.

4. The peptide derivative or the pharmaceutically acceptable salt thereof according to claim 3, wherein R³ is a 4-hydroxybenzyl group.

5. The peptide derivative or the pharmaceutically acceptable salt thereof according to claim 4, wherein R² is a substituted or unsubstituted lower alkyl or aralkyl group.

6. An angiotensin IV receptor agonist comprising as an active ingredient the peptide derivative or the pharmaceutically acceptable salt thereof according to claim 1.

7. The angiotensin IV receptor agonist comprising as an active ingredient a peptide derivative or the pharmaceutically acceptable salt thereof according to claim 2.

8. The angiotensin IV receptor agonist comprising as an active ingredient a peptide derivative or the pharmaceutically acceptable salt thereof according to claim 3.

9. The angiotensin IV receptor agonist comprising as an active ingredient the peptide derivative or the pharmaceutically acceptable salt thereof according to claim 4.

10. The angiotensin IV receptor agonist comprising as an active ingredient the peptide derivative or the pharmaceutically acceptable salt thereof according to claim 5.
